(19) Europäisches Patentamt
European Patent Office
Office européen des brevets

(11) EP 1 498 485 A1

(12) # EUROPEAN PATENT APPLICATION
published in accordance with Art. 158(3) EPC

(43) Date of publication:
**19.01.2005 Bulletin 2005/03**

(21) Application number: **03720897.2**

(22) Date of filing: **09.04.2003**

(51) Int Cl.[7]: **C12N 15/09**, C12N 5/10, C12P 21/08, A01H 5/00, A01K 67/027, C07K 16/00, A61K 39/395, A61P 35/00, A61P 37/00, A61P 9/00, A61P 31/12, A61P 31/04

(86) International application number:
**PCT/JP2003/004507**

(87) International publication number:
**WO 2003/085107 (16.10.2003 Gazette 2003/42)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.04.2002 JP 2002106953**

(83) **Declaration under Rule 28(4) EPC (expert solution)**

(71) Applicant: **KYOWA HAKKO KOGYO CO., LTD.**
**Chiyoda-ku, Tokyo 100-8185 (JP)**

(72) Inventors:
- **OHNUKI, Naoko**
**c/o Tokyo Research Laboratories**
**Machida-shi, Tokyo 194-8533 (JP)**
- **SATOH, Mitsuo**
**c/o Tokyo Research Laboratories**
**Machida-shi, Tokyo 194-8533 (JP)**
- **MORI, Katsuhiro**
**c/o Tokyo Research Laboratories**
**Machida-shi, Tokyo 194-8533 (JP)**
- **YAMANO, Kazuya**
**c/o Tokyo Research Laboratories**
**Machida-shi, Tokyo 194-8533 (JP)**

(74) Representative: **VOSSIUS & PARTNER**
**Siebertstrasse 4**
**81675 München (DE)**

(54) **CELLS WITH MODIFIED GENOME**

(57) A cell in which genome is modified so as to have a more decreased or deleted activity of an enzyme relating to modification of a sugar chain in which 1-position of fucose is bound to 6-position of *N*-acetylglucosamine in the reducing end through $\alpha$-bond in a complex *N*-glycoside-linked sugar chain than its parent cell, and a process for producing an antibody composition using the cell.

EP 1 498 485 A1

Printed by Jouve, 75001 PARIS (FR)

SEQUENCE LISTING

<110> KYOWA HAKKO KOGYO CO., LTD.

<120> Cells of which genome is modified

<130> K2606 EP

<150> JP 2002-106953
<151> 2002-04-09

<160> 31

<170> PatentIn Ver. 2.1

<210> 1
<211> 2008
<212> DNA
<213> Cricetulus griseus

<400> 1
aacagaaact tattttcctg tgtggctaac tagaaccaga gtacaatgtt tccaattctt 60
tgagctccga gaagacagaa gggagttgaa actctgaaaa tgcgggcatg gactggttcc 120
tggcgttgga ttatgctcat tctttttgcc tgggggacct tattgtttta tataggtggt 180
catttggttc gagataatga ccaccctgac cattctagca gagaactctc caagattctt 240
gcaaagctgg agcgcttaaa acaacaaaat gaagacttga ggagaatggc tgagtctctc 300
cgaataccag aaggccctat tgatcagggg acagctacag gaagagtccg tgttttagaa 360
gaacagcttg ttaaggccaa agaacagatt gaaaattaca agaaacaagc taggaatgat 420
ctgggaaagg atcatgaaat cttaaggagg aggattgaaa atggagctaa agagctctgg 480
ttttttctac aaagtgaatt gaagaaatta aagaaattag aaggaaacga actccaaaga 540
catgcagatg aaattctttt ggatttagga catcatgaaa ggtctatcat gacagatcta 600
tactacctca gtcaaacaga tggagcaggt gagtggcggg aaaaagaagc caaagatctg 660
acagagctgg tccagcggag aataacatat ctgcagaatc ccaaggactg cagcaaagcc 720
agaaagctgg tatgtaatat caacaaaggc tgtggctatg gatgtcaact ccatcatgtg 780
gtttactgct tcatgattgc ttatggcacc cagcgaacac tcatcttgga atctcagaat 840
tggcgctatg ctactggagg atgggagact gtgtttagac ctgtaagtga gacatgcaca 900
gacaggtctg gcctctccac tggacactgg tcaggtgaag tgaaggacaa aaatgttcaa 960

gtggtcgagc tccccattgt agacagcctc catcctcgtc ctccttactt acccttggct 1020
gtaccagaag accttgcaga tcgactcctg agagtccatg gtgatcctgc agtgtggtgg 1080
gtatcccagt ttgtcaaata cttgatccgt ccacaacctt ggctggaaag ggaaatagaa 1140
gaaaccacca agaagcttgg cttcaaacat ccagttattg gagtccatgt cagacgcact 1200
gacaaagtgg gaacagaagc agccttccat cccattgagg aatacatggt acacgttgaa 1260
gaacattttc agcttctcga acgcagaatg aaagtggata aaaaaagagt gtatctggcc 1320
actgatgacc cttctttgtt aaaggaggca aagacaaagt actccaatta tgaatttatt 1380
agtgataact ctatttcttg gtcagctgga ctacacaacc gatacacaga aaattcactt 1440
cggggcgtga tcctggatat acactttctc tcccaggctg acttccttgt gtgtactttt 1500
tcatcccagg tctgtagggt tgcttatgaa atcatgcaaa cactgcatcc tgatgcctct 1560
gcaaacttcc attctttaga tgacatctac tattttggag gccaaaatgc ccacaaccag 1620
attgcagttt atcctcacca acctcgaact aaagaggaaa tccccatgga acctggagat 1680
atcattggtg tggctggaaa ccattggaat ggttactcta aaggtgtcaa cagaaaacta 1740
ggaaaaacag gcctgtaccc ttcctacaaa gtccgagaga agatagaaac agtcaaatac 1800
cctacatatc ctgaagctga aaaatagaga tggagtgtaa gagattaaca acagaattta 1860
gttcagacca tctcagccaa gcagaagacc cagactaaca tatggttcat tgacagacat 1920
gctccgcacc aagagcaagt gggaaccctc agatgctgca ctggtggaac gcctctttgt 1980
gaagggctgc tgtgccctca agcccatg 2008

<210> 2
<211> 1728
<212> DNA
<213> Mus musculus

<400> 2
atgcgggcat ggactggttc ctggcgttgg attatgctca ttctttttgc ctgggggacc 60
ttgttatttt atataggtgg tcatttggtt cgagataatg accaccctga tcactccagc 120
agagaactct ccaagattct tgcaaagctt gaacgcttaa aacagcaaaa tgaagacttg 180
aggcgaatgg ctgagtctct ccgaatacca gaaggcccca ttgaccaggg gacagctaca 240
ggaagagtcc gtgttttaga agaacagctt gttaaggcca aagaacagat tgaaaattac 300
aagaaacaag ctagaaatgg tctggggaag gatcatgaaa tcttaagaag gaggattgaa 360
aatggagcta aagagctctg gtttttctta caaagcgaac tgaagaaatt aaagcattta 420
gaaggaaatg aactccaaag acatgcagat gaaattcttt tggatttagg acaccatgaa 480
aggtctatca tgacagatct atactacctc agtcaaacag atggagcagg ggattggcgt 540
gaaaaagagg ccaaagatct gacagagctg gtccagcgga gaataacata tctccagaat 600
cctaaggact gcagcaaagc caggaagctg gtgtgtaaca tcaataaagg ctgtggctat 660

ggttgtcaac tccatcacgt ggtctactgt ttcatgattg cttatggcac ccagcgaaca 720
ctcatcttgg aatctcagaa ttggcgctat gctactggtg gatgggagac tgtgtttaga 780
cctgtaagtg agacatgtac agacagatct ggcctctcca ctggacactg gtcaggtgaa 840
gtaaatgaca aaaacattca agtggtcgag ctccccattg tagacagcct ccatcctcgg 900
cctccttact taccactggc tgttccagaa gaccttgcag accgactcct aagagtccat 960
ggtgaccctg cagtgtggtg ggtgtcccag tttgtcaaat acttgattcg tccacaacct 1020
tggctggaaa aggaaataga agaagccacc aagaagcttg gcttcaaaca tccagttatt 1080
ggagtccatg tcagacgcac agacaaagtg ggaacagaag cagccttcca ccccatcgag 1140
gagtacatgg tacacgttga agaacatttt cagcttctcg cacgcagaat gcaagtggat 1200
aaaaaaagag tatatctggc tactgatgat cctactttgt taaaggaggc aaagacaaag 1260
tactccaatt atgaatttat tagtgataac tctatttctt ggtcagctgg actacacaat 1320
cggtacacag aaaattcact tcggggtgtg atcctggata tacactttct ctcacaggct 1380
gactttctag tgtgtacttt ttcatcccag gtctgtcggg ttgcttatga aatcatgcaa 1440
accctgcatc ctgatgcctc tgcgaacttc cattctttgg atgacatcta ctattttgga 1500
ggccaaaatg cccacaatca gattgctgtt tatcctcaca aacctcgaac tgaagaggaa 1560
attccaatgg aacctggaga tatcattggt gtggctggaa accattggga tggttattct 1620
aaaggtatca acagaaaact tggaaaaaca ggcttatatc cctcctacaa agtccgagag 1680
aagatagaaa cagtcaagta tcccacatat cctgaagctg aaaaatag 1728

<210> 3
<211> 9196
<212> DNA
<213> Cricetulus griseus

<400> 3
tctagaccag gctggtctcg aactcacaga gaaccacctg cctctgccac ctgagtgctg 60
ggattaaagg tgtgcaccac caccgcccgg cgtaaaatca tatttttgaa tattgtgata 120
atttacatta taattgtaag taaaaatttt cagcctattt tgttatacat ttttgcgtaa 180
attattcttt tttgaaagtt ttgttgtcca taatagtcta gggaaacata aagttataat 240
ttttgtctat gtatttgcat atatatctat ttaatctcct aatgtccagg aaataaatag 300
ggtatgtaat agcttcaaca tgtggtatga tagaattttt cagtgctata taagttgtta 360
cagcaaagtg ttattaattc atatgtccat atttcaattt tttatgaatt attaaattga 420
atccttaagc tgccagaact agaattttat tttaatcagg aagccccaaa tctgttcatt 480
ctttctatat atgtggaaag gtaggcctca ctaactgatt cttcacctgt tttagaacat 540
ggtccaagaa tggagttatg taaggggaat tacaagtgtg agaaaactcc tagaaaacaa 600
gatgagtctt gtgaccttag tttctttaaa aacacaaaat tcttggaatg tgttttcatg 660

ttcctcccag gtggatagga gtgagtttat ttcagattat ttattacaac tggctgttgt 720
tacttgtttc tatgtcttta tagaaaaaca tatttttttt gccacatgca gcttgtcctt 780
atgattttat acttgtgtga ctcttaactc tcagagtata aattgtctga tgctatgaat 840
aaagttggct attgtatgag acttcagccc acttcaatta ttggcttcat tctctcagat 900
cccaccacct ccagagtggt aaacaacttg aaccattaaa cagactttag tctttatttg 960
aatgatagat ggggatatca gatttatagg cacagggttt tgagaaaggg agaaggtaaa 1020
cagtagagtt taacaacaac aaaaagtata ctttgtaaac gtaaaactat ttattaaagt 1080
agtagacaag acattaaata ttccttggga ttagtgcttt ttgaattttg ctttcaaata 1140
atagtcagtg agtatacccc tcccccattc tatattttag cagaaatcag aataaatggt 1200
gtttctggta cattcttttg tagagaattt attttctttg ggtttttgtg catttaaagt 1260
caataaaaat taaggttcag taatagaaaa aaaactctga tttttggaat ccccttttctt 1320
cagcttttct atttaatctc ttaatgataa tttaatttgt ggccatgtgg tcaaagtata 1380
tagccttgta tatgtaaatg ttttaaccaa cctgccttta cagtaactat ataattttat 1440
tctataatat atgacttttc ttccatagct ttagagttgc ccagtcactt taagttacat 1500
tttcatatat gttctttgtg ggaggagata attttatttc taagagaatc ctaagcatac 1560
tgattgagaa atggcaaaca aaacacataa ttaaagctga taaagaacga acatttggag 1620
tttaaaatac atagccaccc taagggttta actgttgtta gccttctttt ggaattttta 1680
ttagttcata tagaaaaatg gattttatcg tgacatttcc atatatgtat ataatatatt 1740
tacatcatat ccacctgtaa ttattagtgt ttttaaatat atttgaaaaa ataatggtct 1800
ggtttgatcc atttgaacct tttgatgttt ggtgtggttg ccaattggtt gatggttatg 1860
ataacctttg cttctctaag gttcaagtca gtttgagaat atgtcctcta aaaatgacag 1920
gttgcaagtt aagtagtgag atgacagcga gatggagtga tgagaatttg tagaaatgaa 1980
ttcacttata ctgagaactt gttttgcttt tagataatga acatattagc ctgaagtaca 2040
tagccgaatt gattaattat tcaaagatat aatcttttaa tccctataaa agaggtatta 2100
cacaacaatt caagaaagat agaattagac ttccagtatt ggagtgaacc atttgttatc 2160
aggtagaacc ctaacgtgtg tggttgactt aaagtgttta cttttttacct gatactgggt 2220
agctaattgt ctttcagcct cctggccaaa gataccatga aagtcaactt acgttgtatt 2280
ctatatctca aacaactcag ggtgtttctt actctttcca cagcatgtag agcccaggaa 2340
gcacaggaca agaaagctgc ctccttgtat caccaggaag atctttttgt aagagtcatc 2400
acagtatacc agagagacta attttgtctg aagcatcatg tgttgaaaca acagaaactt 2460
attttcctgt gtggctaact agaaccagag tacaatgttt ccaattcttt gagctccgag 2520
aagacagaag ggagttgaaa ctctgaaaat gcgggcatgg actggttcct ggcgttggat 2580
tatgctcatt ctttttgcct gggggacctt attgttttat ataggtggtc atttggttcg 2640
agataatgac caccctgacc attctagcag agaactctcc aagattcttg caaagctgga 2700
gcgcttaaaa caacaaaatg aagacttgag gagaatggct gagtctctcc ggtaggtttg 2760
aaatactcaa ggatttgatg aaatactgtg cttgaccttt aggtataggg tctcagtctg 2820
ctgttgaaaa atataatttc tacaaaccgt ctttgtaaaa ttttaagtat tgtagcagac 2880

tttttaaaag tcagtgatac atctatatag tcaatatagg tttacatagt tgcaatctta 2940
ttttgcatat gaatcagtat atagaagcag tggcatttat atgcttatgt tgcatttaca 3000
attatgttta gacgaacaca aactttatgt gatttggatt agtgctcatt aaattttttt 3060
attctatgga ctacaacaga gacataaatt ttgaaaggct tagttactct taaattctta 3120
tgatgaaaag caaaaattca ttgttaaata gaacagtgca tccggaatgt gggtaattat 3180
tgccatattt ctagtctact aaaaattgtg gcataactgt tcaaagtcat cagttgtttg 3240
gaaagccaaa gtctgattta aatggaaaac ataaacaatg atatctattt ctagatacct 3300
ttaacttgca gttactgagt ttacaagttg tctgacaact ttggattctc ttacttcata 3360
tctaagaatg atcatgtgta cagtgcttac tgtcacttta aaaaactgca gggctagaca 3420
tgcagatatg aagactttga cattagatgt ggtaattggc actaccagca agtggtatta 3480
agatacagct gaatatatta ctttttgagg aacataattc atgaatggaa agtggagcat 3540
tagagaggat gccttctggc tctcccacac cactgtttgc atccattgca tttcacactg 3600
cttttagaac tcagatgttt catatggtat attgtgtaac tcaccatcag ttttatcttt 3660
aaatgtctat ggatgataat gttgtatgtt aacactttta caaaaacaaa tgaagccata 3720
tcctcggtgt gagttgtgat ggtggtaatt gtcacaatag gattattcag caaggaacta 3780
agtcagggac aagaagtggg cgatactttg ttggattaaa tcattttact ggaagttcat 3840
cagggagggt tatgaaagtt gtggtctttg aactgaaatt atatgtgatt cattattctt 3900
gatttaggcc ttgctaatag taactatcat ttattgggaa tttgtcatat gtgccaattt 3960
gtcatgggcc agacagcgtg ttttactgaa tttctagata tctttatgag attctagtac 4020
tgttttcagc cattttacag atgaagaatc ttaaaaaatg ttaaataatt tagtttgccc 4080
aagattatac gttaacaaat ggtagaacct tctttgaatt ctggcagtat ggctacacag 4140
tccgaactct tatcttccta agctgaaaac agaaaaagca atgacccaga aaattttatt 4200
taaaagtctc aggagagact tcccatcctg agaagatctc ttttcccttt tataatttag 4260
gctcctgaat aatcactgaa ttttctccat gttccatcta tagtactgtt atttctgttt 4320
tcctttttttc ttaccacaaa gtatcttgtt tttgctgtat gaaagaaaat gtgttattgt 4380
aatgtgaaat tctctgtccc tgcagggtcc cacatccgcc tcaatcccaa ataaacacac 4440
agaggctgta ttaattatga aactgttggt cagttggcta gggcttctta ttggctagct 4500
ctgtcttaat tattaaacca taactactat tgtaagtatt tccatgtggt cttatcttac 4560
caaggaaagg gtccagggac ctcttactcc tctggcgtgt tggcagtgaa gaggagagag 4620
cgatttccta tttgtctctg cttattttct gattctgctc agctatgtca cttcctgcct 4680
ggccaatcag ccaatcagtg ttttattcat tagccaataa aagaaacatt tacacagaag 4740
gacttccccc atcatgttat ttgtatgagt tcttcagaaa atcatagtat cttttaatac 4800
taatttttat aaaaaattaa ttgtattgaa aattatgtgt atatgtgtct gtgtgtcgat 4860
ttgtgctcat aagtagcatg gagtgcagaa gagggaatca gatctttttt taagggacaa 4920
agagtttatt cagattacat tttaaggtga taatgtatga ttgcaaggtt atcaacatgg 4980
cagaaatgtg aagaagctgg tcacattaca tccagagtca agagtagaga gcaatgaatt 5040
gatgcatgca ttcctgtgct cagctcactt ttcctggagc tgagctgatt gtaagccatc 5100

tgatgtcttt gctgggaact aactcaaagg caagttcaaa acctgttctt aagtataagc 5160
catctctcca gtccctcata tggtctctta agacactttc tttatattct tgtacataga 5220
aattgaattc ctaacaactg cattcaaatt acaaaatagt ttttaaaagc tgatataata 5280
aatgtaaata caatctagaa catttttata aataagcata ttaactcagt aaaaataaat 5340
gcatggttat tttccttcat tagggaagta tgtctcccca ggctgttctc tagattctac 5400
tagtaatgct gtttgtacac catccacagg ggttttattt taaagctaag acatgaatga 5460
tggacatgct tgttagcatt tagacttttt tccttactat aattgagcta gtatttttgt 5520
gctcagtttg atatctgtta attcagataa atgtaatagt aggtaatttc tttgtgataa 5580
aggcatataa attgaagttg gaaaacaaaa gcctgaaatg acagttttta agattcagaa 5640
caataatttt caaaagcagt tacccaactt tccaaataca atctgcagtt ttcttgatat 5700
gtgataaatt tagacaaaga aatagcacat tttaaaatag ctatttactc ttgatttttt 5760
tttcaaattt aggctagttc actagttgtg tgtaaggtta tggctgcaaa catctttgac 5820
tcttggttag ggaatccagg atgatttacg tgtttggcca aaatcttgtt ccattctggg 5880
tttcttctct atctaggtag ctagcacaag ttaaaggtgt ggtagtattg gaaggctctc 5940
aggtatatat ttctatattc tgtatttttt tcctctgtca tatatttgct ttctgtttta 6000
ttgatttcta ctgttagttt gatacttact ttcttacact ttctttggga tttattttgc 6060
tgttctaaga tttcttagca agttcatatc actgatttta acagttgctt cttttgtaat 6120
atagactgaa tgccccttat ttgaaatgct tgggatcaga aactcagatt tgaacttttc 6180
ttttttaata tttccatcaa gtttaccagc tgaatgtcct gatccaagaa tatgaaatct 6240
gaaatgcttt gaaatctgaa actttttagag tgataaagct tccctttaaa ttaatttgtg 6300
ttctatattt tttgacaatg tcaacctttc attgttatcc aatgagtgaa catattttca 6360
attttttttgt ttgatctgtt atattttgat ctgaccatat ttataaaatt ttatttaatt 6420
tgaatgttgt gctgttactt atctttatta ttatttttgc ttattttcta gccaaatgaa 6480
attatattct gtattatttt agtttgaatt ttactttgtg gcttagtaac tgccttttgt 6540
tggtgaatgc ttaagaaaaa cgtgtggtct actgatattg gttctaatct tatatagcat 6600
gttgtttgtt aggtagttga ttatgctggt cagattgtct tgagtttatg caaatgtaaa 6660
atatttagat gcttgttttg ttgtctaaga acaaagtatg cttgctgtct cctatcggtt 6720
ctggttttc cattcatctc ttcaagctgt tttgtgtgtt gaatactaac tccgtactat 6780
cttgttttct gtgaattaac cccttttcaa aggtttcttt tctttttttt tttaagggac 6840
aacaagttta ttcagattac attttaagct gataatgtat gattgcaagg ttatcaacat 6900
ggcagaaatg tgaagaagct aggcacatta catccacatg gagtcaagag cagagagcag 6960
tgaattaatg catgcattcc tgtggtcagc tcacttttcc tattcttaga tagtctagga 7020
tcataaacct ggggaatagt gctaccacaa tgggcatatc cacttacttc agttcatgca 7080
atcaaccaag gcacatccac aggaaaaact gatttagaca acctctcatt gagactcttc 7140
ccagatgatt agactgtgtc aagttgacaa ttaaaactat cacacctgaa gccatcacta 7200
gtaaatataa tgaaaatgtt gattatcacc ataattcatc tgtatccctt tgttattgta 7260
gattttgtga agttcctatt caagtccctg ttccttcctt aaaaacctgt tttttagtta 7320

aataggtttt ttagtgttcc tgtctgtaaa tactttttta aagttagata ttattttcaa 7380
gtatgttctc ccagtctttg gcttgtattt tcatcccttc aatacatata tttttgtaat 7440
ttattttttt tatttaaatt agaaacaaag ctgcttttac atgtcagtct cagttccctc 7500
tccctcccct cctcccctgc tccccaccta agccccaatt ccaactcctt tcttctcccc 7560
aggaagggtg aggccctcca tgggggaaat cttcaatgtc tgtcatatca tttggagcag 7620
ggcctagacc ctccccagtg tgtctaggct gagagagtat ccctctatgt ggagagggct 7680
cccaaagttc atttgtgtac taggggtaaa tactgatcca ctatcagtgg ccccatagat 7740
tgtccggacc tccaaactga cttcctcctt cagggagtct ggaacagttc tatgctggtt 7800
tcccagatat cagtctgggg tccatgagca accccttgtt caggtcagtt gtttctgtag 7860
gtttccccag cccggtcttg accccttttgc tcatcacttc tccctctctg caactggatt 7920
ccagagttca gctcagtgtt tagctgtggg tgtctgcatc tgcttccatc agctactgga 7980
tgagggctct aggatggcat ataaggtagt catcagtctc attatcagag aagggctttt 8040
aaggtagcct cttgattatt gcttagattg ttagttgggg tcaaccttgt aggtctctgg 8100
acagtgacag aattctcttt aaacctataa tggctccctc tgtggtggta tccctttttct 8160
tgctctcatc cgttcctccc ctgactagat cttcctgctc cctcatgtcc tcctctcccc 8220
tccccttctc cccttctctt tcttctaact ccctctcccc tccacccacg atccccatta 8280
gcttatgaga tcttgtcctt attttagcaa aaccttttttg gctataaaat taattaattt 8340
aatatgctta tatcaggttt attttggcta gtatttgtat gtgtttggtt agtgttttta 8400
accttaattg acatgtatcc ttatatttag acacagattt aaatatttga agtttttttt 8460
tttttttttt ttaaagattt atttattttt tatgtcttct gcctgcatgc cagaagaggg 8520
caccagatct cattcaaggt ggttgtgagc caccatgtgg ttgctgggaa ttgaactcag 8580
gacctctgga agaacagtca gtgctcttaa ccgctgagcc atctctccag cccctgaagt 8640
gtttctttta aagaggatag cagtgcatca tttttccctt tgaccaatga ctcctacctt 8700
actgaattgt tttagccatt tatatgtaat gctgttacca ggtttacatt ttcttttatc 8760
ttgctaaatt tcttccctgt ttgtctcatc tcttattttt gtctgttgga ttatataggc 8820
ttttattttt ctgtttttac agtaagttat atcaaattaa aattatttta tggaatgggt 8880
gtgttgacta catgtatgtc tgtgcaccat gtgctgacct ggtcttggcc agaagaaggt 8940
gtcatattct ctgaaactgg tattgtggat gttacgaact gccatagggt gctaggaatc 9000
aaaccccagc tcctctggaa aagcagccac tgctctgagc cactgagtcc tctcttcaag 9060
caggtgatgc caacttttaa tggttaccag tggataagag tgcttgtatc tctagcaccc 9120
atgaaaattt atgcattgct atatgggctt gtcacttcag cattgtgtga cagagacagg 9180
aggatcccaa gagctc 9196

<210> 4
<211> 575
<212> PRT

<213> Cricetulus griseus

<400> 4

Met Arg Ala Trp Thr Gly Ser Trp Arg Trp Ile Met Leu Ile Leu Phe
1 5 10 15
Ala Trp Gly Thr Leu Leu Phe Tyr Ile Gly Gly His Leu Val Arg Asp
20 25 30
Asn Asp His Pro Asp His Ser Ser Arg Glu Leu Ser Lys Ile Leu Ala
35 40 45
Lys Leu Glu Arg Leu Lys Gln Gln Asn Glu Asp Leu Arg Arg Met Ala
50 55 60
Glu Ser Leu Arg Ile Pro Glu Gly Pro Ile Asp Gln Gly Thr Ala Thr
65 70 75 80
Gly Arg Val Arg Val Leu Glu Glu Gln Leu Val Lys Ala Lys Glu Gln
85 90 95
Ile Glu Asn Tyr Lys Lys Gln Ala Arg Asn Asp Leu Gly Lys Asp His
100 105 110
Glu Ile Leu Arg Arg Arg Ile Glu Asn Gly Ala Lys Glu Leu Trp Phe
115 120 125
Phe Leu Gln Ser Glu Leu Lys Lys Leu Lys Lys Leu Glu Gly Asn Glu
130 135 140
Leu Gln Arg His Ala Asp Glu Ile Leu Leu Asp Leu Gly His His Glu
145 150 155 160
Arg Ser Ile Met Thr Asp Leu Tyr Tyr Leu Ser Gln Thr Asp Gly Ala
165 170 175
Gly Glu Trp Arg Glu Lys Glu Ala Lys Asp Leu Thr Glu Leu Val Gln
180 185 190
Arg Arg Ile Thr Tyr Leu Gln Asn Pro Lys Asp Cys Ser Lys Ala Arg
195 200 205
Lys Leu Val Cys Asn Ile Asn Lys Gly Cys Gly Tyr Gly Cys Gln Leu
210 215 220
His His Val Val Tyr Cys Phe Met Ile Ala Tyr Gly Thr Gln Arg Thr
225 230 235 240
Leu Ile Leu Glu Ser Gln Asn Trp Arg Tyr Ala Thr Gly Gly Trp Glu
245 250 255
Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285
Val Glu Leu Pro Ile Val Asp Ser Leu His Pro Arg Pro Pro Tyr Leu
290 295 300
Pro Leu Ala Val Pro Glu Asp Leu Ala Asp Arg Leu Leu Arg Val His
305 310 315 320
Gly Asp Pro Ala Val Trp Trp Val Ser Gln Phe Val Lys Tyr Leu Ile
325 330 335
Arg Pro Gln Pro Trp Leu Glu Arg Glu Ile Glu Glu Thr Thr Lys Lys
340 345 350
Leu Gly Phe Lys His Pro Val Ile Gly Val His Val Arg Arg Thr Asp
355 360 365
Lys Val Gly Thr Glu Ala Ala Phe His Pro Ile Glu Glu Tyr Met Val
370 375 380
His Val Glu Glu His Phe Gln Leu Leu Glu Arg Arg Met Lys Val Asp
385 390 395 400
Lys Lys Arg Val Tyr Leu Ala Thr Asp Asp Pro Ser Leu Leu Lys Glu
405 410 415
Ala Lys Thr Lys Tyr Ser Asn Tyr Glu Phe Ile Ser Asp Asn Ser Ile
420 425 430
Ser Trp Ser Ala Gly Leu His Asn Arg Tyr Thr Glu Asn Ser Leu Arg
435 440 445
Gly Val Ile Leu Asp Ile His Phe Leu Ser Gln Ala Asp Phe Leu Val
450 455 460
Cys Thr Phe Ser Ser Gln Val Cys Arg Val Ala Tyr Glu Ile Met Gln
465 470 475 480
Thr Leu His Pro Asp Ala Ser Ala Asn Phe His Ser Leu Asp Asp Ile
485 490 495
Tyr Tyr Phe Gly Gly Gln Asn Ala His Asn Gln Ile Ala Val Tyr Pro
500 505 510
His Gln Pro Arg Thr Lys Glu Glu Ile Pro Met Glu Pro Gly Asp Ile
515 520 525
Ile Gly Val Ala Gly Asn His Trp Asn Gly Tyr Ser Lys Gly Val Asn
530 535 540
Arg Lys Leu Gly Lys Thr Gly Leu Tyr Pro Ser Tyr Lys Val Arg Glu
545 550 555 560
Lys Ile Glu Thr Val Lys Tyr Pro Thr Tyr Pro Glu Ala Glu Lys

565 570 575

<210> 5
<211> 575
<212> PRT
<213> Mus musculus

<400> 5
Met Arg Ala Trp Thr Gly Ser Trp Arg Trp Ile Met Leu Ile Leu Phe
1 5 10 15
Ala Trp Gly Thr Leu Leu Phe Tyr Ile Gly Gly His Leu Val Arg Asp
20 25 30
Asn Asp His Pro Asp His Ser Ser Arg Glu Leu Ser Lys Ile Leu Ala
35 40 45
Lys Leu Glu Arg Leu Lys Gln Gln Asn Glu Asp Leu Arg Arg Met Ala
50 55 60
Glu Ser Leu Arg Ile Pro Glu Gly Pro Ile Asp Gln Gly Thr Ala Thr
65 70 75 80
Gly Arg Val Arg Val Leu Glu Glu Gln Leu Val Lys Ala Lys Glu Gln
85 90 95
Ile Glu Asn Tyr Lys Lys Gln Ala Arg Asn Gly Leu Gly Lys Asp His
100 105 110
Glu Ile Leu Arg Arg Arg Ile Glu Asn Gly Ala Lys Glu Leu Trp Phe
115 120 125
Phe Leu Gln Ser Glu Leu Lys Lys Leu Lys His Leu Glu Gly Asn Glu
130 135 140
Leu Gln Arg His Ala Asp Glu Ile Leu Leu Asp Leu Gly His His Glu
145 150 155 160
Arg Ser Ile Met Thr Asp Leu Tyr Tyr Leu Ser Gln Thr Asp Gly Ala
165 170 175
Gly Asp Trp Arg Glu Lys Glu Ala Lys Asp Leu Thr Glu Leu Val Gln
180 185 190
Arg Arg Ile Thr Tyr Leu Gln Asn Pro Lys Asp Cys Ser Lys Ala Arg
195 200 205
Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240
Leu Ile Leu Glu Ser Gln Asn Trp Arg Tyr Ala Thr Gly Gly Trp Glu
245 250 255
Thr Val Phe Arg Pro Val Ser Glu Thr Cys Thr Asp Arg Ser Gly Leu
260 265 270
Ser Thr Gly His Trp Ser Gly Glu Val Asn Asp Lys Asn Ile Gln Val
275 280 285
Val Glu Leu Pro Ile Val Asp Ser Leu His Pro Arg Pro Pro Tyr Leu
290 295 300
Pro Leu Ala Val Pro Glu Asp Leu Ala Asp Arg Leu Leu Arg Val His
305 310 315 320
Gly Asp Pro Ala Val Trp Trp Val Ser Gln Phe Val Lys Tyr Leu Ile
325 330 335
Arg Pro Gln Pro Trp Leu Glu Lys Glu Ile Glu Glu Ala Thr Lys Lys
340 345 350
Leu Gly Phe Lys His Pro Val Ile Gly Val His Val Arg Arg Thr Asp
355 360 365
Lys Val Gly Thr Glu Ala Ala Phe His Pro Ile Glu Glu Tyr Met Val
370 375 380
His Val Glu Glu His Phe Gln Leu Leu Ala Arg Arg Met Gln Val Asp
385 390 395 400
Lys Lys Arg Val Tyr Leu Ala Thr Asp Asp Pro Thr Leu Leu Lys Glu
405 410 415
Ala Lys Thr Lys Tyr Ser Asn Tyr Glu Phe Ile Ser Asp Asn Ser Ile
420 425 430
Ser Trp Ser Ala Gly Leu His Asn Arg Tyr Thr Glu Asn Ser Leu Arg
435 440 445
Gly Val Ile Leu Asp Ile His Phe Leu Ser Gln Ala Asp Phe Leu Val
450 455 460
Cys Thr Phe Ser Ser Gln Val Cys Arg Val Ala Tyr Glu Ile Met Gln
465 470 475 480
Thr Leu His Pro Asp Ala Ser Ala Asn Phe His Ser Leu Asp Asp Ile
485 490 495
Tyr Tyr Phe Gly Gly Gln Asn Ala His Asn Gln Ile Ala Val Tyr Pro
500 505 510
His Lys Pro Arg Thr Glu Glu Glu Ile Pro Met Glu Pro Gly Asp Ile

515 520 525

Ile Gly Val Ala Gly Asn His Trp Asp Gly Tyr Ser Lys Gly Ile Asn

530 535 540

Arg Lys Leu Gly Lys Thr Gly Leu Tyr Pro Ser Tyr Lys Val Arg Glu

545 550 555 560

Lys Ile Glu Thr Val Lys Tyr Pro Thr Tyr Pro Glu Ala Glu Lys

565 570 575

<210> 6
<211> 18
<212> PRT
<213> Homo sapiens

<400> 6
Asp Glu Ser Ile Tyr Ser Asn Tyr Tyr Leu Tyr Glu Ser Ile Pro Lys
1 5 10 15
Pro Cys

<210> 7
<211> 24
<212> DNA
<213> Artificial Sequence

<220>
<223> Description of Artificial Sequence: Synthetic DNA

<400> 7
gtctgaagca ttatgtgttg aagc 24

<210> 8
<211> 23
<212> DNA
<213> Artificial Sequence
<220>

<223> Description of Artificial Sequence: Synthetic DNA

<400> 8
gtgagtacat tcattgtact gtg 23

<210> 9
<211> 18
<212> DNA
<213> Artificial Sequence

<220>
<223> Description of Artificial Sequence : Synthetic DNA

<400> 9
ggtaggcctc actaactg 18

<210> 10
<211> 25
<212> DNA
<213> Artificial Sequence

<220>
<223> Description of Artificial Sequence : Synthetic DNA

<400> 10
catagaaaca agtaacaaca gccag 25

<210> 11
<211> 21
<212> DNA
<213> Artificial Sequence

<220>
<223> Description of Artificial Sequence: Synthetic DNA

<400> 11
gtgagtccat ggctgtcact g 21

<210> 12
<211> 20
<212> DNA
<213> Artificial Sequence

<220>
<223> Description of Artificial Sequence: Synthetic DNA

<400> 12
cctgacttgg ctattctcag 20

<210> 13
<211> 28
<212> DNA
<213> Artificial Sequence

<220>
<223> Description of Artificial Sequence: Synthetic DNA

<400> 13
gagacttcag cccacttcaa ttattggc 28

<210> 14
<211> 25
<212> DNA
<213> Artificial Sequence

<220>
<223> Description of Artificial Sequence: Synthetic DNA

<400> 14
gaggccactt gtgtagcgcc aagtg 25

<210> 15
<211> 25
<212> DNA
<213> Artificial Sequence

<220>
<223> Description of Artificial Sequence: Synthetic DNA

<400> 15
cttgtgtgac tcttaactct cagag 25

<210> 16
<211> 23
<212> DNA
<213> Artificial Sequence

<220>
<223> Description of Artificial Sequence: Synthetic DNA

<400> 16
ccctcgagat aacttcgtat agc 23

<210> 17
<211> 384
<212> DNA
<213> Mus musculus

<400> 17
atg gat ttt cag gtg cag att atc agc ttc ctg cta atc agt gct tca 48
Met Asp Phe Gln Val Gln Ile Ile Ser Phe Leu Leu Ile Ser Ala Ser
1 5 10 15

gtc ata atg tcc aga gga caa att gtt ctc tcc cag tct cca gca atc 96
Val Ile Met Ser Arg Gly Gln Ile Val Leu Ser Gln Ser Pro Ala Ile
20 25 30
ctg tct gca tct cca ggg gag aag gtc aca atg act tgc agg gcc agc 144
Leu Ser Ala Ser Pro Gly Glu Lys Val Thr Met Thr Cys Arg Ala Ser
35 40 45
tca agt gta agt tac atc cac tgg ttc cag cag aag cca gga tcc tcc 192
Ser Ser Val Ser Tyr Ile His Trp Phe Gln Gln Lys Pro Gly Ser Ser
50 55 60
ccc aaa ccc tgg att tat gcc aca tcc aac ctg gct tct gga gtc cct 240
Pro Lys Pro Trp Ile Tyr Ala Thr Ser Asn Leu Ala Ser Gly Val Pro
65 70 75 80
gtt cgc ttc agt ggc agt ggg tct ggg act tct tac tct ctc acc atc 288
Val Arg Phe Ser Gly Ser Gly Ser Gly Thr Ser Tyr Ser Leu Thr Ile
85 90 95
agc aga gtg gag gct gaa gat gct gcc act tat tac tgc cag cag tgg 336
Ser Arg Val Glu Ala Glu Asp Ala Ala Thr Tyr Tyr Cys Gln Gln Trp
100 105 110
act agt aac cca ccc acg ttc gga ggg ggg acc aag ctg gaa atc aaa 384
Thr Ser Asn Pro Pro Thr Phe Gly Gly Gly Thr Lys Leu Glu Ile Lys
115 120 125

<210> 18
<211> 420
<212> DNA
<213> Mus musculus

<400> 18
atg ggt tgg agc ctc atc ttg ctc ttc ctt gtc gct gtt gct acg cgt 48
Met Gly Trp Ser Leu Ile Leu Leu Phe Leu Val Ala Val Ala Thr Arg
1 5 10 15
gtc ctg tcc cag gta caa ctg cag cag cct ggg gct gag ctg gtg aag 96
Val Leu Ser Gln Val Gln Leu Gln Gln Pro Gly Ala Glu Leu Val Lys
20 25 30
cct ggg gcc tca gtg aag atg tcc tgc aag gct tct ggc tac aca ttt 144
Pro Gly Ala Ser Val Lys Met Ser Cys Lys Ala Ser Gly Tyr Thr Phe

35 40 45
acc agt tac aat atg cac tgg gta aaa cag aca cct ggt cgg ggc ctg 192
Thr Ser Tyr Asn Met His Trp Val Lys Gln Thr Pro Gly Arg Gly Leu
50 55 60
gaa tgg att gga gct att tat ccc gga aat ggt gat act tcc tac aat 240
Glu Trp Ile Gly Ala Ile Tyr Pro Gly Asn Gly Asp Thr Ser Tyr Asn
65 70 75 80
cag aag ttc aaa ggc aag gcc aca ttg act gca gac aaa tcc tcc agc 288
Gln Lys Phe Lys Gly Lys Ala Thr Leu Thr Ala Asp Lys Ser Ser Ser
85 90 95
aca gcc tac atg cag ctc agc agc ctg aca tct gag gac tct gcg gtc 336
Thr Ala Tyr Met Gln Leu Ser Ser Leu Thr Ser Glu Asp Ser Ala Val
100 105 110
tat tac tgt gca aga tcg act tac tac ggc ggt gac tgg tac ttc aat 384
Tyr Tyr Cys Ala Arg Ser Thr Tyr Tyr Gly Gly Asp Trp Tyr Phe Asn
115 120 125
gtc tgg ggc gca ggg acc acg gtc acc gtc tct gca 420
Val Trp Gly Ala Gly Thr Thr Val Thr Val Ser Ala
130 135 140

<210> 19
<211> 91
<212> DNA
<213> Artificial Sequence

<220>
<223> Description of Artificial Sequence: Synthetic DNA

<400> 19
caggaaacag ctatgacgaa ttcgcctcct caaaatggat tttcaggtgc agattatcag 60
cttcctgcta atcagtgctt cagtcataat g 91

<210> 20
<211> 91
<212> DNA

<213> Artificial Sequence

<220>
<223> Description of Artificial Sequence: Synthetic DNA

<400> 20
gtgaccttct cccctggaga tgcagacagg attgctggag actgggagag aacaatttgt 60
cctctggaca ttatgactga agcactgatt a 91

<210> 21
<211> 90
<212> DNA
<213> Artificial Sequence

<220>
<223> Description of Artificial Sequence: Synthetic DNA

<400> 21
ctccagggga gaaggtcaca atgacttgca gggccagctc aagtgtaagt tacatccact 60
ggttccagca gaagccagga tcctccccca 90

<210> 22
<211> 89
<212> DNA
<213> Artificial Sequence

<220>
<223> Description of Artificial Sequence: Synthetic DNA

<400> 22
ccagacccac tgccactgaa gcgaacaggg actccagaag ccaggttgga tgtggcataa 60
atccagggtt tgggggagga tcctggctt 89

<210> 23

<211> 91
<212> DNA
<213> Artificial Sequence

<220>
<223> Description of Artificial Sequence: Synthetic DNA

<400> 23
tcagtggcag tgggtctggg acttcttact ctctcaccat cagcagagtg gaggctgaag 60
atgctgccac ttattactgc cagcagtgga c 91

<210> 24
<211> 90
<212> DNA
<213> Artificial Sequence

<220>
<223> Description of Artificial Sequence: Synthetic DNA

<400> 24
gttttcccag tcacgaccgt acgtttgatt tccagcttgg tccccctcc gaacgtgggt 60
gggttactag tccactgctg gcagtaataa 90

<210> 25
<211> 99
<212> DNA
<213> Artificial Sequence

<220>
<223> Description of Artificial Sequence: Synthetic DNA

<400> 25
caggaaacag ctatgacgcg gccgcgaccc ctcaccatgg gttggagcct catcttgctc 60
ttccttgtcg ctgttgctac gcgtgtcctg tcccaggta 99

<210> 26
<211> 98
<212> DNA
<213> Artificial Sequence

<220>
<223> Description of Artificial Sequence: Synthetic DNA

<400> 26
atgtgtagcc agaagccttg caggacatct tcactgaggc cccagccttc accagctcag 60
ccccaggctg ctgcagttgt acctgggaca ggacacgc 98

<210> 27
<211> 97
<212> DNA
<213> Artificial Sequence

<220>
<223> Description of Artificial Sequence: Synthetic DNA

<400> 27
caaggcttct ggctacacat ttaccagtta caatatgcac tgggtaaaac agacacctgg 60
tcggggcctg gaatggattg gagctattta tcccgga 97

<210> 28
<211> 99
<212> DNA
<213> Artificial Sequence

<220>
<223> Description of Artificial Sequence: Synthetic DNA

<400> 28
gtaggctgtg ctggaggatt tgtctgcagt caatgtggcc ttgcctttga acttctgatt 60

gtaggaagta tcaccatttc cgggataaat agctccaat 99

<210> 29
<211> 99
<212> DNA
<213> Artificial Sequence

<220>
<223> Description of Artificial Sequence: Synthetic DNA

<400> 29
aatcctccag cacagcctac atgcagctca gcagcctgac atctgaggac tctgcggtct 60
attactgtgc aagatcgact tactacggcg gtgactggt 99

<210> 30
<211> 98
<212> DNA
<213> Artificial Sequence

<220>
<223> Description of Artificial Sequence: Synthetic DNA

<400> 30
gttttcccag tcacgacggg cccttggtgg aggctgcaga gacggtgacc gtggtccctg 60
cgccccagac attgaagtac cagtcaccgc cgtagtaa 98

<210> 31
<211> 25
<212> DNA
<213> Artificial Sequence

<220>
<223> Description of Artificial Sequence: Synthetic DNA

<400> 31
gagctggtga agcctggggc ctcag 25

**Description**

TECHNICAL FIELD

The present invention relates to a cell in which genome is modified so as to have more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell and a process for producing an antibody molecule using the cell.

BACKGROUND ART

In the Fc region of an antibody of an IgG type, two *N*-glycoside-linked sugar chain binding sites are present. In serum IgG, to the sugar chain binding site, generally, binds a complex type sugar chain having plural branches and in which addition of sialic acid or bisecting *N*-acetylglucosamine is low. It is known that there is variety regarding the addition of galactose to the non-reducing end of the complex type sugar chain and the addition of fucose to the *N*-acetylglucosamine in the reducing end [*Biochemistry,* 36, 130 (1997)].

It has been considered that such a structure of a sugar chain is determined by sugar chain genes, namely a gene for a glycosyltransferase which synthesizes a sugar chain and a gene for a glycolytic enzyme which hydrolyzes the sugar chain.

Synthesis of an *N*-glycoside-linked sugar chain is described below.

Glycoproteins are modified with a sugar chain in the endoplasmic reticulum (hereinafter referred to as "ER") lumen. During the biosynthesis step of the *N*-glycoside-linked sugar chain, a relatively large sugar chain is transferred to the polypeptide chain which is elongating in the ER lumen. In the transformation, the sugar chain is firstly added in succession to phosphate groups of a long chain lipid carrier comprising about 20 α-isoprene units, which is called dolichol phosphate (hereinafter sometiems referred to as "P-Dol"). That is, *N*-acetylglucosamine is transferred to dolichol phosphate to thereby form GlcNAc-P-P-Dol and then one more GlcNAc is transferred to form GlcNAc-GlcNAc-P-P-Dol. Next, five mannoses (hereinafter mannose is sometimes referred to as "Man") are transferred to thereby form $(Man)_5$-$(GlcNAc)_2$-P-P-Dol and then four Man's and three glucoses (hereinafter glucose is sometimes referred to as "Glc") are transferred. Thus, a sugar chain precursor, $(Glc)_3$-$(Man)_9$-$(GlcNAc)_2$-P-P-Dol, called core oligosaccharide is formed. The sugar chain precursor comprising 14 sugars is transferred as a mass to a polypeptide having an asparagine-X-serine or asparagine-X-threonine sequence in the ER lumen. In the reaction, dolichol pyrophosphate (P-P-Dol) bound to the core oligosaccharide is released but again becomes dolichol phosphate by hydrolysis with pyrophosphatase and is recycled. Trimming of the sugar chain immediately starts after the sugar chain binds to the polypeptide. That is, three Glc's and one or two Man's are eliminated on the ER, and it is known that α-1,2-glucosidase I, α-1,3-glucosidase II and α-1,2-mannosidase relates to the elimination. The glycoprotein which was subjected to trimming on the ER is transferred to the Golgi body and are variously modified. In the *cis* part of the Golgi body, *N*-acetylglucosamine phosphotransferase which relates to addition of mannose phosphate, *N*-acetylglucosamine 1-phosphodiester α-*N*-acetylglucosaminidase and α-mannosidase I are present and reduce the Man residues to 5. In the medium part of the Golgi body, *N*-acetylglucosamine transferase I (GnTI) which relates to addition of the first outside GlcNAc of the complex type *N*-glycoside-linked sugar chain, α-mannosidase II which relates to elimination of two Man's, *N*-acetylglucosamine transferase II (GnTII) which relates to addition of the second GlcNAc from the outside and α1,6-fucosyltransferase which relates to addition of fucose to the reducing end *N*-acetylglucosamine are present. In the *trans* part of the Golgi body, galactose transferase which relates to addition of galactose and sialyltransferase which relates to addition of sialic acid such as *N*-acetylneuraminic acid or the like are present. It is known that *N*-glycoside-linked sugar chain is formed by activities of these various enzymes.

Regarding a sugar chain in an antibody, it is reported that addition-modification of fucose to *N*-acetylglucosamine in the reducing end in the *N*-glycoside-linked sugar chain of an antibody changes the antibody-dependent cell-mediated cytotoxic activity (hereinafter referred to as "ADCC activity") of the antibody greatly (WO00/61739). This report indicates that the structure of the sugar chain plays an important role in the effector functions of human antibodies of IgG1 subclass.

In general, most of the humanized antibodies of which application to medicaments is in consideration are prepared by using genetic recombination techniques and produced by using animal cells, such as Chinese hamster ovary tissue-derived CHO cell, as the host cell. But as described above, since the sugar chain structure plays a remarkably important role in the effector function of antibodies and differences are observed in the sugar chain structure of glycoproteins expressed by host cells, development of a host cell which can be used for the production of an antibody having higher effector function is desired.

Also, an attempt has been made to modify the sugar chain structure of a produced glycoprotein by introducing an enzyme gene relating to the modification of sugar chains, and as its examples, it has been reported that 1) it is

possible to produce a protein in which sialic acid is added in a large number to the non-reducing end of a sugar chain by introducing rat β-galactoside-α-2,6-sialyltransferase into CHO cell [*J. Biol. Chem.,* 261, 13848 (1989)], 2) it is possible to express an H antigen in which fucose (hereinafter also referred to as "Fuc") is added to the non-reducing end of a sugar chain (Fucα1-2Galβ1-) by introducing human β-galactoside-2-α-fucosyltransferase into mouse L cell [*Science,* 252, 1668 (1991)], and 3) it is possible to produce an antibody having a high addition ratio of the bisecting *N*-acetylglucosamine of *N*-glycoside binding sugar chains by producing an antibody using a β-1,4-*N*-acetylglucosamine transferase III (GnTIII)-introduced CHO cell [*Glycobiology.,* 5, 813 (1995): WO 99/54342]. When the antibody was expressed by using a GnTIII-introduced CHO cell, it showed 16 times higher ADCC activity than the antibody expressed in the parent cell. However, since it has been reported that over-expression of GnTIII or β-1,4-*N*-acetylglucosamine transferase V (GnTV) shows toxicity upon CHO cell, it is not suitable for the production of antibody medicaments.

It has also been reported on a production example of a glycoprotein in which a produced sugar chain structure was changed by using, as a host cell, a mutant in which the activity of an enzyme gene relating to the modification of sugar chains was changed, and as its example, it has been reported that an antibody having a high mannose type sugar chain structure using a mutant clone of CHO cell in which the activity of 4-*N*-acetylglucosamine transferase I (GnTI) was deleted was produced [*J. Immunol.,* 160, 3393 (1998)]. In addition, expression of an antibody having a sugar chain structure in which sialic acid is not added to the non-reducing end side in the sugar chain and an expression example of an antibody having no addition of galactose, by using a CMP-sialic acid transporter- or UDP-galactose transporter-deficient clone, have been reported, but no antibody having improved effector functions suitable for the application to medicaments has been found [*J. Immunol.,* 160, 3393 (1998)]. Since the mutant clones have been obtained as clones resulting from the introduction of random mutation by mutagen treatment, they are not suitable as clones used in the production of pharmaceutical preparations.

Thus, in order to modify a sugar chain structure of a produced glycoprotein, attempts have been made to control the activity of an enzyme relating to the modification of sugar chains in host cells. But in fact, since the sugar chain modification mechanism is diversified and complicated and it cannot be said that physiological roles of sugar chains has been sufficiently revealed, it is the present situation that trial and error are repeated. Particularly, it has been revealed gradually that effector functions of antibodies have great influences by sugar chain structures, but a host cell capable of producing antibody molecules modified with a most suitable sugar chain structure has not been obtained yet.

## DISCLOSURE OF THE INVENTION

The present invention relates to the following (1) to (43).

(1) A cell in which genome is modified so as to have a more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell.

(2) The cell according to (1), wherein a genomic gene encoding an enzyme relating to modification of a sugar chain in which 1-position of fucose is bound to 6-position of *N*-acetylglucosamine in the reducing end through α-bond in a complex *N*-glycoside-linked sugar chain is knocked out.

(3) The cell according to (1) or (2), wherein all of alleles on a genome encoding an enzyme relating to modification of a sugar chain in which 1-position of fucose is bound to 6-position of *N*-acetylglucosamine in the reducing end through α-bond in a complex *N*-glycoside-linked sugar chain are knocked out.

(4) The cell according to any one of (1) to (3), wherein the enzyme relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

(5) The cell according to (4), wherein the α1,6-fucosyltransferase is a protein encoded by a DNA selected from the group consisting of the following (a) to (d):

(a) a DNA comprising the nucleotide sequence represented by SEQ ID NO:1;
(b) a DNA comprising the nucleotide sequence represented by SEQ ID NO:2;
(c) a DNA which hybridizes with a DNA comprising the nucleotide sequence represented by SEQ ID NO:1 under stringent conditions and having an α1,6-fucosyltransferase activity;
(d) a DNA which hybridizes with a DNA comprising the nucleotide sequence represented by SEQ ID NO:2 under stringent conditions and having an α1,6-fucosyltransferase activity.

(6) The cell according to (4), wherein the α1,6-fucosyltransferase is a protein selected from the group consisting of the following (a), (b), (c), (d), (e) and (f):

(a) a protein comprising the amino acid sequence represented by SEQ ID NO:4;
(b) a protein comprising the amino acid sequence represented by SEQ ID NO:5;
(c) a protein which comprises an amino acid sequence in which at least one amino acid is deleted, substituted, inserted and/or added in the amino acid sequence represented by SEQ ID NO:4 and has an α1,6-fucosyltransferase activity;
(d) a protein which comprises an amino acid sequence in which at least one amino acid is deleted, substituted, inserted and/or added in the amino acid sequence represented by SEQ ID NO:5 and has an α1,6-fucosyltransferase activity;
(e) a protein which comprises an amino acid sequence having a homology of 80% or more with the amino acid sequence represented by SEQ ID NO:4 and has an α1,6-fucosyltransferase activity;
(f) a protein which comprises an amino acid sequence having a homology of 80% or more with the amino acid sequence represented by SEQ ID NO:5 and has an α1,6-fucosyltransferase activity.

(7) The cell according to any one of (1) to (6), which is resistant to a lectin which recognizes a sugar chain in which 1-position of fucose is bound to 6-position of *N*-acetylglucosamine in the reducing end through α-bond in a complex *N*-glycoside-linked sugar chain.
(8) The cell according to (7), which is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

(9) The cell according to any one of (1) to (8), which is selected from the group consisting of the following (a) to (j):

(a) a CHO cell derived from a Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-Ag14 cell;
(e) a BHK cell derived from a Syrian hamster kidney tissue;
(f) a hybridoma cell which produces an antibody;
(g) a human leukemic cell line Namalwa cell;
(h) an embryonic stem cell;
(i) a fertilized egg cell;
(j) a plant cell.

(10) The cell according to any one of (1) to (9), which contains a gene encoding an antibody molecule.
(11) The cell according to (10), wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

(12) The cell according to (10) or (11), wherein the antibody molecule belongs to an IgG class.
(13) The cell according to any one of (1) to (12), which produces an antibody composition having a higher antibody-dependent cell-mediated cytotoxic activity than an antibody composition produced by its parent cell.
(14) The cell according to (13), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.
(15) The cell according to (14), wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.
(16) The cell according to any one of (13) to (15), wherein the ratio of a sugar chain in which fucose is not bound to *N*-acetylglucosamine in the reducing end through α-bond is 20% or more of total complex *N*-glycoside-linked

sugar chains bound to the Fc region in the antibody composition.

(17) The cell according to any one of (13) to (16), wherein the antibody composition having a higher antibody-dependent cell-mediated cytotoxic activity is an antibody composition which has no sugar chain in which fucose is bound to *N*-acetylglucosamine in the reducing end in the sugar chain.

(18) A process for producing an antibody composition, which comprises using the cell according to any one of (10) to (17).

(19) A process for producing an antibody composition, which comprises culturing the cell according to any one of (10) to (18) in a medium to form and accumulate an antibody composition in the culture; and recovering the antibody composition from the culture.

(20) The process according to (18) or (19), wherein the antibody composition is an antibody composition having a higher antibody-dependent cell-mediated cytotoxic activity than an antibody composition produced by its parent cell.

(21) The process according to (20), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

(22) The process according to (21), wherein the sugar chain in which fucose is not bound is a sugar chain in which 1-position of the fucose is not bound to 6-position of *N*-acetylglucosamine in the reducing end through $\alpha$-bond in a complex *N*-glycoside-linked sugar chain.

(23) The cell according to any one of (20) to (22), wherein the antibody composition having a higher antibody-dependent cell-mediated cytotoxic activity is a an antibody composition in which a ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.

(24) The cell according to any one of (20) to (23), wherein the antibody composition having a higher antibody-dependent cell-mediated cytotoxic activity is an antibody composition which has no sugar chain in which fucose is bound to N-acetylglucosamine in the reducing end in the sugar chain.

(25) A transgenic non-human animal or plant or the progenies thereof, which is produced by using the cell according to any one of (1) to (9).

(26) The transgenic non-human animal or plant or the progenies thereof according to (24), wherein the transgenic non-human animal is an animal selected from the group consisting of cattle, sheep, goat, pig, horse, mouse, rat, fowl, monkey and rabbit.

(27) The transgenic non-human animal or plant or the progenies thereof according to (25) or (26), which is introduced with a gene encoding an antibody molecule.

(28) The transgenic non-human animal or plant or the progenies thereof according to (27), wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

(29) The transgenic non-human animal or plant or the progenies thereof according to (27) or (28), wherein the antibody molecule belongs to an IgG class.

(30) A process for producing an antibody composition, which comprises rearing the transgenic non-human animal or plant according to any one of (27) to (29); isolating a tissue or body fluid comprising an antibody molecule introduced from the reared animal or plant; and recovering an antibody composition comprising a desired antibody molecule from the isolated tissue or body fluid.

(31) A process for producing an antibody composition, which comprises isolating an antibody-producing cell from the transgenic non-human animal or plant or the progenies thereof according to any one of (26) to (29); culturing the isolated antibody-producing cell in a medium to form and accumulate an antibody composition in the culture; and recovering the antibody composition from the culture.

(32) The process according to (30) or (31), wherein the antibody composition is an antibody composition having a higher antibody-dependent cell-mediated cytotoxic activity than an antibody composition produced by a transgenic non-human animal or plant or the progenies thereof in which genome is not modified.

(33) The process according to (32), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by a transgenic non-human animal or plant

or the progenies thereof in which genome is not modified.

(34) The process according to (33), wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

(35) The process according to any one of (32) to (34), wherein the antibody composition having a higher antibody-dependent cell-mediated cytotoxic activity is an antibody composition in which a ratio of a sugar chain in which fucose is not bound to *N*-acetylglucosamine in the reducing end in the sugar chain is 20% or more of total complex *N*-glycoside-linked sugar chains bound to the Fc region in the antibody composition.

(36) The cell according to any one of (32) to (35), wherein the antibody composition having a higher antibody-dependent cell-mediated cytotoxic activity is an antibody composition which has no sugar chain in which fucose is bound to *N*-acetylglucosamine in the reducing end in the sugar chain.

(37) An antibody composition comprising an antibody molecule having an *N*-glycoside-linked sugar chain in the Fc region, which has a sugar chain in which fucose is not bound to *N*-acetylglucosamine in the reducing end in the sugar chain among total complex *N*-glycoside-linked sugar chains bound to the Fc region in the antibody composition.

(38) The antibody composition according to (37), wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

(39) An antibody composition produced by the process of any one of (18) to (24).

(40) An antibody composition produced by the process of any one of (18) to (24).

(41) A medicament comprising as an active ingredient the antibody composition according to any one of (37) to (40).

(42) The medicament according to (41), which is a diagnostic agent, an preventing agent or a treating agent for tumor-accompanied diseases, allergy-accompanied diseases, inflammatory-accompanied diseases, autoimmune diseases, cardiovascular diseases, viral infection-accompanied diseases or bacterial infection-accompanied diseases.

(43) Use of the antibody composition according to any one of (37) to (40) in the manufacture of the medicament according to (41) or (42).

A method of genome modification in a cell in which genome is modified so as to have more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell (hereinafter referred to as "cell of the present invention") is not particularly limited, so long as the genome of the cell is modified so as to have more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain (hereinafter referred to as "α1,6-fucose modifying enzyme") than its parent cell.

The parent cell is a cell before a method for decreasing or deleting activity of the α1,6-fucose modifying enzyme is applied to the genome. The parent cell is not particularly limited, and includes the following cells.

The parent cell of NS0 cell includes NS0 cells described in literatures such as *BIO/TECHNOLOGY,* 10, 169 (1992) and *Biotechnol. Bioeng.,* 73 261 (2001), NS0 cell line (RCB 0213) registered at RIKEN Cell Bank, The Institute of Physical and Chemical Research, sub-cell lines obtained by naturalizing these cell lines to media in which they can grow, and the like.

The parent cell of SP2/0-Ag14 cell includes SP2/0-Ag14 cells described in literatures such as *J. Immunol.,* 126, 317 (1981), *Nature,* 276, 269 (1978) and *Human Antibodies and Hybridomas,* 3, 129 (1992), SP2/0-Ag14 cell (ATCC CRL-1581) registered at ATCC, sub-cell lines obtained by naturalizing these cell lines to media in which they can grow (ATCC CRL-1581.1), and the like.

The parent cell of CHO cell derived from Chinese hamster ovary tissue includes CHO cells described in literatures such as *Journal of Experimental Medicine (Jikken Igaku),* 108, 945 (1958), *Proc. Natl. Acad. Sci. USA,* 60, 1275 (1968), *Genetics,* 55, 513 (1968), *Chromosoma,* 41, 129 (1973), *Methods in Cell Science,* 18, 115 (1996), *Radiation Research,* 148, 260 (1997), *Proc. Natl. Acad. Sci. USA,* 77, 4216 (1980), *Proc. Natl. Acad. Sci. USA,* 60, 1275 (1968), *Cell,* 6, 121 (1975) and *Molecular Cell Genetics,* Appendix I, II (p. 883-900), cell line CHO-K1 (ATCC CCL-61), cell line DUXB11 (ATCC CRL-9096) and cell line Pro-5 (ATCC CRL-1781) registered at ATCC, commercially available cell line CHO-S (Cat # 11619 of Life Technologies), sub-cell lines obtained by naturalizing these cell lines to media in which they can grow, and the like.

The parent cell of a rat myeloma cell line YB2/3HL.P2.G11.16Ag.20 cell includes cell lines established from Y3/Ag1.2.3 cell (ATCC CRL-1631), YB2/3HL.P2.G11.16Ag.20 cell described in literatures such as *J. Cell. Biol.,* 93, 576 (1982) and *Methods Enzymol.,* 73B, 1 (1981), YB2/3HL.P2.G11.16Ag.20 cell (ATCC CRL-1662) registered at ATCC, sub-lines obtained by naturalizing these cell lines to media in which they can grow, and the like.

The α1,6-fucose modifying enzyme includes any enzyme, so long as it is an enzyme relating to the reaction

of binding of 1-position of fucose to 6-position of *N*-acetylglucosamine in the reducing end through α-bond in the complex *N*-glycoside-linked sugar chain. The enzyme relating to the reaction of binding of 1-position of fucose to 6-position of *N*-acetylglucosamine in the reducing end through α-bond in the complex *N*-glycoside-linked sugar chain includes an enzyme which has influence on the reaction of binding of 1-position of fucose to 6-position of *N*-acetylglucosamine in the reducing end through α-bond in the complex *N*-glycoside-linked sugar chain.

The 1,6-fucose modifying enzyme includes α1,6-fucosyltransferase, α-L-fucosidase and the like.

Also, the enzyme having influence on the reaction of binding of 1-position of fucose to 6-position of *N*-acetylglucosamine in the reducing end through α-bond in the complex *N*-glycoside-linked sugar chain includes an enzyme which has influence on the activity the enzyme relating to the reaction of binding of 1-position of fucose to 6-position of *N*-acetylglucosamine in the reducing end through α-bond in the complex *N*-glycoside-linked sugar chain and an enzyme which has influence on the structure of substances as the substrate of the enzyme.

In the present invention, the α1,6-fucose modifying enzyme includes a protein encoded by a DNA of the following (a), (b), (c) or (d) and a protein of the following (e), (f), (g), (h), (i) or (j):

(a) a DNA comprising the nucleotide sequence represented by SEQ ID NO:1;
(b) a DNA comprising the nucleotide sequence represented by SEQ ID NO:2;
(c) a DNA which hybridizes with the DNA comprising the nucleotide sequence represented by SEQ ID NO:1 under stringent conditions and encodes a protein having α1,6-fucosyltransferase activity;
(d) a DNA which hybridizes with the DNA comprising the nucleotide sequence represented by SEQ ID NO:2 under stringent conditions and encodes a protein having α1,6-fucosyltransferase activity;
(e) a protein comprising the amino acid sequence represented by SEQ ID NO:4,
(f) a protein comprising the amino acid sequence represented by SEQ ID NO:5,
(g) a protein which comprises an amino acid sequence in which at least one amino acid is deleted, substituted, inserted and/or added in the amino acid sequence represented by SEQ ID NO:4 and has α1,6-fucosyltransferase activity,
(h) a protein which comprises an amino acid sequence in which at least one amino acid is deleted, substituted, inserted and/or added in the amino acid sequence represented by SEQ ID NO:5 and has α1,6-fucosyltransferase activity,
(i) a protein which comprises an amino acid sequence having a homology of 80% or more with the amino acid sequence represented by SEQ ID NO:4 and has α1,6-fucosyltransferase activity,
(j) a protein which comprises an amino acid sequence having a homology of 80% or more with the amino acid sequence represented by SEQ ID NO:5 and has α1,6-fucosyltransferase activity, and the like.

In the present invention, a DNA which hybridizes under stringent conditions is a DNA obtained, e.g., by a method such as colony hybridization, plaque hybridization or Southern blot hybridization using a DNA such as the DNA having the nucleotide sequence represented by SEQ ID NO:1 or 2 or a partial fragment thereof as the probe, and specifically includes a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1 to 2 × SSC solution (composition of the 1 × SSC solution comprising 150 mM sodium chloride and 15 mM sodium citrate). The hybridization can be carried out in accordance with the methods described, e.g., in *Molecular Cloning, A Laboratory Manual,* Second Edition, Cold Spring Harbor Laboratory Press (1989) (hereinafter referred to as *"Molecular Cloning, Second Edition"*), *Current Protocols in Molecular Biology,* John Wiley & Sons, 1987-1997 (hereinafter referred to as *"Current Protocols in Molecular Biology"*); *DNA Cloning 1: Core Techniques, A Practical Approach,* Second Edition, Oxford University (1995); and the like. The hybridizable DNA includes a DNA having at least 60% or more, preferably 70% or more, more preferably 80% or more, still more preferably 90% or more, far more preferably 95% or more, and most preferably 98% or more, of homology with the nucleotide sequence represented by SEQ ID NO:1 or 2.

In the present invention, the protein which comprises an amino acid sequence in which at least one amino acid is deleted, substituted, inserted and/or added in the amino acid sequence represented by SEQ ID NO:4 or 5 and has α1,6-fucosyltransferase activity can be obtained, e.g., by introducing a site-directed mutation into a DNA encoding a protein having the amino acid sequence represented by SEQ ID NO:4 or 5, respectively, using the site-directed mutagenesis described, e.g., in *Molecular Cloning, Second Edition; Current Protocols in Molecular Biology; Nucleic Acids Research,* 10, 6487 (1982); *Proc. Natl. Acad. Sci. USA,* 79, 6409 (1982); *Gene,* 34, 315 (1985); *Nucleic Acids Research,* 13, 4431 (1985); *Proc. Natl. Acad. Sci. USA*, 82, 488 (1985); and the like. The number of amino acids to be deleted, substituted, inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

Also, in the present invention, the protein which comprises an amino acid sequence having a homology of

80% or more with the amino acid sequence represented by SEQ ID NO:4 or 5 and has α1,6-fucosyltransferase activity is a protein having at least 80% or more, preferably 85% or more, more preferably 90% or more, still more preferably 95% or more, far more preferably 97% or more, and most preferably 99% or more, of homology with the amino acid sequence represented by SEQ ID NO:4 or 5, when calculated by using an analyzing soft such as BLAST [*J. Mol. Biol.,* 215, 403 (1990)], FASTA [*Methods in Enzymology,* 183, 63 (1990)] or the like.

In the present invention, modification of genome so as to have more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell means that mutation is introduced into an expression-controlling region of the enzyme so as to decrease the expression of the enzyme, or that mutation is introduced into an amino acid sequence of the gene so as to decrease the function of the enzyme. Introduction of the mutation means that modification such as deletion, substitution, insertion and/or addition is carried out in the nucleotide sequence of the genome.

A cell in which genomic gene is knocked out means that the expression or function of the genomic gene is completely inhibited in the cell. The cell in which genomic gene is knocked out includes a cell in which a target gene is completely or partly deleted from the genome. As a method for obtaining such a cell, any technique can be used, so long as the genome of interest can be modified. However, genetic engineering techniques are preferred. Examples include:

(a) a gene disruption technique which comprises targeting a gene encoding the 1,6-fucose modifying enzyme,
(b) a technique for introducing a dominant negative mutant of a gene encoding the 1,6-fucose modifying enzyme,
(c) a technique for introducing mutation into a gene encoding the 1,6-fucose modifying enzyme,
(d) a technique for suprressing transcription and/or translation of a gene encoding the α1,6-fucose modifying enzyme, and the like.

Furthermore, the cell of the present invention can be obtained by using a method for selecting a clone resistant to a lectin which recognizes a sugar chain in which 1-position of fucose is bound to 6-position of *N*-acetylglucosamine in the reducing end through α-bond in the complex *N*-glycoside-linked sugar chain.

The growth of lectin-resistant cell is not inhibited in the presence of a lectin at an effective concentration during cell culturing. The effective concentration is a concentration in which the parent cell cannot normally grow or higher than the concentration, and is a concentration which is preferably similar to, more preferably 2 to 5 times, still more preferably at least 10 times, and most preferably at least 20 times, higher than the concentration in which the parent cell cannot normally grow.

In the present invention, the effective concentration of a lectin in which does not inhibit the growth can be decided depending on the cell line, and is generally 10 μg/ml to 10.0 mg/ml, preferably 0.5 to 2.0 mg/ml.

As the lectin which recognizes a sugar chain structure in which 1-position of fucose is bound to 6-position of *N*-acetylglucosamine in the reducing end through α-bond in the *N*-glycoside-linked sugar chain, any lectin can be used, so long as it can recognize the sugar chain structure. Examples include a *Lens culinaris* lectin LCA (lentil agglutinin derived from *Lens culinaris),* a pea lectin PSA (pea lectin derived from *Pisum sativum),* a broad bean lectin VFA (agglutinin derived from *Vicia faba*), an *Aleuria aurantia* lectin AAL (lectin derived from *Aleuria aurantia*) and the like.

The cell of the present invention may be any cell, so long as it can express an antibody molecule. Examples include a yeast, an animal cell, an insect cell, a plant cell and the like, and specific examples include those described in the item 3 below. The animal cell includes a CHO cell derived from a Chinese hamster ovary tissue, a rat myeloma cell line YB2/3HL.P2.G11.16Ag.20 cell, a mouse myeloma cell line NS0 cell, a mouse myeloma SP2/0-Ag14 cell, a BHK cell derived from a syrian hamster kidney tissue, an antibody producing-hybridoma cell, a human leukemia cell line Namalwa cell, an embryonic stem cell, a fertilized egg cell and the like. Preferable examples include the above myeloma cell and hybridoma cell used for producing an antibody composition, a host cell for producing a humanized antibody and a human antibody, an embryonic stem cell and fertilized egg cell for preparing a non-human transgenic animal which produces a human antibody, a plant cell for preparing a transgenic plant which produces a humanized antibody and a human antibody, and the like.

The cell of the present invention can produce an antibody composition having higher ADCC activity than that of an antibody composition produced by a parent cell.

Furthermore, the cell of the present invention can produce an antibody composition wherein among the total complex *N*-glycoside-linked sugar chains bound to the Fc region in the composition, the ratio of a sugar chain in which fucose is not bound to *N*-acetylglucosamine in the reducing end in the sugar chain is higher than that of an antibody composition produced by a parent cell.

The present invention relates to a process for producing an antibody composition, which is characterized by using the cell of the present invention.

The antibody composition is a composition which comprises an antibody molecule having a complex *N*-glycoside-linked sugar chain in the Fc region.

The antibody is a tetramer in which two molecules of each of two polypeptide chains, a heavy chain (hereinafter referred to as "H chain") and a light chain (hereinafter referred to as "L chain"), are respectively associated. Each of about a quarter of the *N*-terminal side of the H chain and about a quarter of the *N*-terminal side of the L chain (more than 100 amino acids for each) is called V region which is rich in diversity and directly relates to the binding to an antigen. The greater part of the moiety other than the V region is called C region. Based on homology with the C region, antibody molecules are classified into classes IgG, IgM, IgA, IgD and IgE.

Also, the IgG class is further classified into subclasses IgG1 to IgG4 based on homology with the C region.

The H chain is classified into four immunoglobulin domains VH, CH1, CH2 and CH3 from its *N*-terminal side, and a highly flexible peptide region called hinge region is present between CH1 and CH2 to divide CH1 and CH2. A structural unit comprising CH2 and CH3 after the hinge region is called Fc region to which a complex *N*-glycoside-linked sugar chain is bound and is also a region to which an Fc receptor, a complement and the like are bound (*Immunology Illustrated,* the Original, 5th edition, published on February 10, 2000, by Nankodo; *Handbook of Antibody Technology* (*Kotai Kogaku Nyumon),* 1st edition on January 25, 1994, by Chijin Shokan).

Sugar chains of glycoproteins such as an antibody are roughly classified into two types, namely a sugar chain which binds to asparagine (N-glycoside-linked sugar chain) and a sugar chain which binds to other amino acid such as serine, threonine (*O* glycoside-linked sugar chain), based on the binding form to the protein moiety. The *N*-glycoside-linked sugar chains have a basic common core structure shown by the following structural formula (I) [*Biochemical Experimentation Method 23 - Method for Studying Glycoprotein Sugar Chain* (Gakujutsu Shuppan Center), edited by Reiko Takahashi (1989)]:

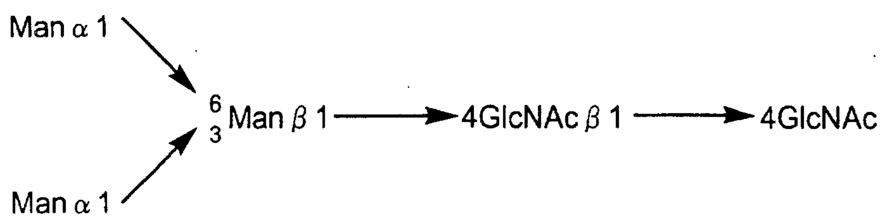

(I)

In formula (I), the sugar chain terminus which binds to asparagine is called a reducing end, and the opposite side is called a non-reducing end.

The *N*-glycoside-linked sugar chain may be any *N*-glycoside-linked sugar chain, so long as it comprises the core structure of formula (I). Examples include a high mannose type in which mannose alone binds to the non-reducing end of the core structure; a complex type in which the non-reducing end side of the core structure has at least one parallel branches of galactose-*N*-acetylglucosamine (hereinafter referred to as "Gal-GlcNAc") and the non-reducing end side of Gal-GlcNAc has a structure of sialic acid, bisecting *N*-acetylglucosamine or the like; a hybrid type in which the non-reducing end side of the core structure comprises branches of both of the high mannose type and complex type; and the like.

Since the Fc region in the antibody molecule has positions to which *N*-glycoside-linked sugar chains are separately bound, two sugar chains are bound per one antibody molecule. Since the N-glycoside-linked sugar chain which binds to an antibody molecule includes any sugar chain comprising the core structure represented by formula (I), a number of combinations of sugar chains may be possible for the two *N*-glycoside-linked sugar chains which bind to the antibody.

Accordingly, in the present invention, the antibody composition of the present invention which is prepared by using the cell of the present invention comprises an antibody having the same sugar chain structure or an antibody having different sugar chain structures, so long as the effect of the present invention is obtained from the composition. The antibody composition of the present invention is preferably an antibody composition in which, among the total complex *N*-glycoside-linked sugar chains bound to the Fc region in the antibody composition, the ratio of a sugar chain in which fucose is not bound to *N*-acetylglucosamine in the reducing end in the sugar chain is higher than that of an antibody composition produced by a parent cell in which genome is not modified.

Furthermore, in the present invention, the antibody composition which is prepared by using a non-human animal or plant or the progenies thereof in which genome is modified so as to have more decreased or deleted activity of the α1,6-fucose modifying enzyme may comprise an antibody having the same sugar chain structure or an antibody having different sugar chain structures, so long as the effect of the present invention is obtained from the composition.

The antibody composition of the present invention is preferably an antibody composition in which, among the total complex *N*-glycoside-linked sugar chains bound to the Fc region in the antibody composition, the ratio of a sugar

chain in which fucose is not bound to *N*-acetylglucosamine in the reducing end in the sugar chain is higher than that of an antibody composition prepared by using a non-human animal or plant or the progenies thereof (hereinafter referred to as "parent individual") in which genome is not modified.

The transgenic non-human animal or plant or the progenies thereof in which genome is modified so as to have a more decreased or deleted activity of the α1,6-fucose modifying enzyme can be prepared by using an embryonic stem cell, a fertilized egg or a plant cell.

In the present invention, the ratio of a sugar chain in which fucose is not bound to *N*-acetylglucosamine in the reducing end in the sugar chain among the total complex *N*-glycoside-linked sugar chains bound to the Fc region contained in the antibody composition is a ratio of the number of a sugar chain in which fucose is not bound to *N*-acetylglucosamine in the reducing end in the sugar chain to the total number of the complex *N*-glycoside-linked sugar chains bound to the Fc region contained in the antibody composition.

The sugar chain in which fucose is not bound to *N*-acetylglucosamine in the reducing end in the complex *N*-glycoside-linked sugar chain is a sugar chain in which fucose is not bound to *N*-acetylglucosamine in the reducing end through α-bond in the complex *N*-glycoside-linked sugar chain. Specifically, it is a complex *N*-glycoside-linked sugar chain in which 1-position of fucose is not bound to 6-position of *N*-acetylglucosamine through α-bond.

The ratio of a sugar chain in which fucose is not bound to *N*-acetylglucosamine in the reducing end in the sugar chain among the total complex *N*-glycoside-linked sugar chains bound to the Fc region contained in the antibody composition of the present invention is preferably 20% or more, more preferably 30% or more, still more preferably 40% or more, most preferably 50% or more, and far most preferably 100%.

The antibody composition having higher ADCC activity than the antibody composition produced by the parent cell or parent individual includes those in which, among total complex *N*-glycoside-linked sugar chains bound to the Fc region in the antibody composition, the ratio of a sugar chain in which fucose is not bound to *N*-acetylglucosamine in the reducing end in the sugar chain is higher than the ratio in an antibody composition produced by the parent cell or parent individual. Examples include an antibody composition in which the activity is at least 2 times, preferably at least 3 times, more preferably at least 5 times, and still more preferably 10 times or higher. An antibody composition in which all of complex *N*-glycoside-linked sugar chains bound to the Fc region in the antibody composition are sugar chains in which fucose is not bound to *N*-acetylglucosamine in the reducing end in the sugar chain is most preferred.

The antibody composition having a ratio of a sugar chain in which fucose is not bound to *N*-acetylglucosamine in the reducing end in the sugar chain of 100% or the antibody composition in which all of complex *N*-glycoside-linked sugar chains bound to the Fc region in the antibody composition are sugar chains in which 1-position of the fucose is not bound to 6-position of *N*-acetylglucosamine in the reducing end contains those in which fucose in such a degree that it cannot be detected by the sugar chain analysis described in the following item 5.

In the antibody composition obtained in the present invention, when, among total complex *N*-glycoside-linked sugar chains bound to the Fc region, the ratio of a sugar chain in which fucose is not bound to *N*-acetylglucosamine in the reducing end in the sugar chain is higher than that in an antibody composition produced by the parent cell or parent individual, the antibody composition obtained in the present invention has higher ADCC activity than the antibody composition comprising an antibody molecule produced by the parent cell or parent individual.

The ADCC activity is a cytotoxic activity in which an antibody bound to a cell surface antigen existed on a tumor cell in the living body activate an effector cell through an Fc receptor existing on the antibody Fc region and effector cell surface and thereby obstruct the tumor cell and the like [*Monoclonal Antibodies: Principles and Applications,* Wiley-Liss, Inc., Chapter 2.1 (1955)]. The effector cell includes a killer cell, a natural killer cell, an activated macrophage and the like.

The ratio of a sugar chain in which fucose is not bound to *N*-acetylglucosamine in the reducing end in the sugar chain contained in the composition which comprises an antibody molecule having complex *N*-glycoside-linked sugar chains in the Fc region can be determined by releasing the sugar chain from the antibody molecule by using a known method such as hydrazinolysis, enzyme digestion or the like [*Biochemical Experimentation Methods 23 - Method for Studying Glycoprotein Sugar Chain* (Japan Scientific Societies Press), edited by Reiko Takahashi (1989)], carrying out fluorescence labeling or radioisotope labeling of the released sugar chain and then separating the labeled sugar chain by chromatography. Also, the released sugar chain can also be determined by analyzing it with the HPAED-PAD method [*J. Liq. Chromatogr.,* 6, 1577 (1983)].

Also, the antibody of the present invention is preferably an antibody which recognizes a tumor-related antigen, an antibody which recognizes an allergy- or inflammation-related antigen, an antibody which recognizes cardiovascular disease-related antigen or an antibody which recognizes a viral or bacterial infection-related antigen are exemplified below, and preferably belongs to IgG class.

The antibody which recognizes a tumor-related antigen includes anti-GD2 antibody [*Anticancer Res.,* 13, 331-336 (1993)], anti-GD3 antibody [*Cancer Immunol. Immunother.,* 36, 260-266 (1993)], anti-GM2 antibody [*Cancer Res.,* 54, 1511-1516 (1994)], anti-HER2 antibody [*Proc. Natl. Acad. Sci. USA,* 89, 4285-4289 (1992)], anti-CD52 antibody [*Proc. Natl. Acad. Sci. USA,* 89, 4285-4289 (1992)], anti-MAGE antibody [*British J. Cancer,* 83, 493-497 (2000)],

anti-HM1.24 antibody [*Molecular Immunol.,* 36, 387-395 (1999)], anti-parathyroid hormone-related protein (PTHrP) antibody [*Cancer,* 88, 2909-2911 (2000)], anti-basic fibroblast growth factor antibody and anti-FGF8 antibody [*Proc. Natl. Acad. Sci. USA,* 86, 9911-9915 (1989)], anti-basic fibroblast growth factor receptor antibody and anti-FGF8 receptor antibody [*J. Biol. Chem.,* 265, 16455-16463 (1990)], anti-insulin-like growth factor antibody [*J. Neurosci. Res.,* 40, 647-659 (1995)], anti-insulin-like growth factor receptor antibody [*J. Neurosci. Res.,* 40, 647-659 (1995)], anti-PMSA antibody [*J. Urology,* 160, 2396-2401 (1998)], anti-vascular endothelial cell growth factor antibody [*Cancer Res.,* 57, 4593-4599 (1997)], anti-vascular endothelial cell growth factor receptor antibody [*Oncogene,* 19, 2138-2146 (2000)], anti-CA125 antibody, anti-17-1A antibody, anti-integrin $\alpha v \beta 3$ antibody, anti-CD33 antibody, anti-CD22 antibody, anti-HLA antibody, anti-HLA-DR antibody, anti-CD20 antibody, anti-CD19 antibody, anti-EGF receptor antibody [*Immunology Today,* 21(8), 403-410 (2000)], anti-CD10 antibody [*American Journal of Clinical Pathology,* 113, 374-382 (2000)] and the like.

The antibody which recognizes an allergy- or inflammation-related antigen includes anti-interleukin 6 antibody [*Immunol. Rev.,* 127, 5-24 (1992)], anti-interleukin 6 receptor antibody [*Molecular Immunol.,* 31, 371-381 (1994)], anti-interleukin 5 antibody [*Immunol. Rev.,* 127, 5-24 (1992)], anti-interleukin 5 receptor antibody and anti-interleukin 4 antibody [*Cytokine,* 3, 562-567 (1991)], anti-interleukin 4 antibody [*J. Immunol. Meth.,* 217, 41-50 (1991)], anti-tumor necrosis factor antibody [*Hybridoma,* 13, 183-190 (1994)], anti-tumor necrosis factor receptor antibody [*Molecular Pharmacol.,* 58, 237-245 (2000)], anti-CCR4 antibody [*Nature,* 400, 776-780 (1999)], anti-chemokine antibody [*J. Immuno. Meth.,* 174, 249-257 (1994)], anti-chemokine receptor antibody [*J. Exp. Med.,* 186, 1373-1381 (1997)], anti-IgE antibody, anti-CD23 antibody, anti-CD11a antibody [*Immunology Today,* 21(8), 403-410 (2000)], anti-CRTH2 antibody [*J Immunol.,* 162, 1278-1286 (1999)], anti-CCR8 antibody (WO99/25734), anti-CCR3 antibody (US6207155) and the like.

The antibody which recognizes a cardiovascular disease-related antigen includes anti-GpIIb/IIIa antibody [*J. Immunol.,* 152, 2968-2976 (1994)], anti-platelet-derived growth factor antibody [*Science,* 253, 1129-1132 (1991)], anti-platelet-derived growth factor receptor antibody [*J. Biol. Chem.,* 272, 17400-17404 (1997)], anti-blood coagulation factor antibody [*Circulation,* 101, 1158-1164 (2000)] and the like.

The antibody which recognizes an antigen relating to autoimmune diseases includes an anti-auto-DNA antibody [*Immunol. Letters,* 72, 61-68 (2000)] and the like.

The antibody which recognizes a viral or bacterial infection-related antigen includes anti-gp120 antibody [*Structure,* 8, 385-395 (2000)], anti-CD4 antibody [*J. Rheumatology,* 25, 2065-2076 (1998)], anti-CCR4 antibody, anti-Vero toxin antibody [*J. Clin. Microbiol.,* 37, 396-399 (1999)], antibody against autoimmune diseases (psoriasis, rheumarthritis, Crohn's diseases, colitis ulcerosa, systemic erythematodes, disseminated sclerosis, *etc.*), anti-CD11a antibody, anti-ICAM3 antibody, anti-CD80 antibody, anti-CD2 antibody, anti-CD3 antibody, anti-CD4 antibody, anti-integrin $\alpha 4 \beta 7$ antibody, anti-GD40L antibody, anti-IL-2 antibody [*Immunology Today,* 21(8), 403-410 (2000)], and the like.

The antibody molecule may be any antibody molecule, so long as it comprises the Fc region of an antibody. Examples include an antibody, an antibody fragment, a fusion protein comprising an Fc region, and the like.

The antibody is a protein which is produced in the living body by immune reaction as a result of exogenous antigen stimulation and has an activity to specifically bind to the antigen. Examples include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from mouse or the like and can produce a monoclonal antibody having the desired antigen specificity.

The humanized antibody includes a human chimeric antibody, a human CDR-grafted antibody and the like.

A human chimeric antibody is an antibody which comprises an H chain V region (hereinafter referred to as "HV" or "VH") and an L chain V region (hereinafter referred to as "LV" or "VL"), both of a non-human animal antibody, a human antibody H chain C region (hereinafter also referred to as "CH") and a human antibody L chain C region (hereinafter also referred to as "CL"). The non-human animal may be any animal such as mouse, rat, hamster, rabbit or the like, so long as a hybridoma can be prepared therefrom.

The human chimeric antibody can be produced by obtaining cDNA's encoding VH and VL from a monoclonal antibody-producing hybridoma, inserting them into an expression vector for host cell having genes encoding human antibody CH and human antibody CL to thereby construct a vector for expression of human chimeric antibody, and then introducing the vector into a host cell to express the antibody.

The CH of human chimeric antibody may be any CH, so long as it belongs to human immunoglobulin (hereinafter referred to as "hIg") can be used. Those belonging to the hIgG class are preferred and any one of the subclasses belonging to the hIgG class, such as hIgG 1, hIgG2, hIgG3 and hIgG4, can be used. Also, as the CL of human chimeric antibody, any CL can be used, so long as it belongs to the hIg class, and those belonging to the $\kappa$ class or $\lambda$ class can also be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNA's encoding V regions in which CDRs of VH and VL of a non-human animal antibody are grafted into CDRs of VH and VL of a human antibody, inserting them into an expression vector for host cell having genes encoding human antibody CH and human antibody CL to thereby construct a human CDR-grafted antibody expression vector, and then introducing the expression vector into a host cell to express the human CDR-grafted antibody.

The CH of human CDR-grafted antibody may be any CH, so long as it belongs to the hIg. Those of the hIgG class are preferred and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ class can also be used.

A human antibody is originally an antibody naturally existing in the human body, but it also includes antibodies obtained from a human antibody phage library, a human antibody-producing transgenic non-human animal and a human antibody-producing transgenic plant, which are prepared based on the recent advance in genetic engineering, cell engineering and developmental engineering techniques.

Regarding the antibody existing in the human body, a lymphocyte capable of producing the antibody can be cultured by isolating a human peripheral blood lymphocyte, immortalizing it by its infection with EB virus or the like and then cloning it, and the antibody can be purified from the culture.

The human antibody phage library is a library in which antibody fragments such as Fab, single chain antibody and the like are expressed on the phage surface by inserting a gene encoding an antibody prepared from a human B cell into a phage gene. A phage expressing an antibody fragment having the desired antigen binding activity can be recovered from the library based on its activity to bind to an antigen-immobilized substrate. The antibody fragment can be converted further into a human antibody molecule comprising two full H chains and two full L chains by genetic engineering techniques.

A human antibody-producing transgenic non-human animal is a non-human animal in which a human antibody gene is introduced into cells. Specifically, a human antibody-producing transgenic animal can be prepared by introducing a human antibody gene into embryonic stem cell of a mouse, transplanting the embryonic stem cell into an early stage embryo of other mouse and then developing it. By introducing a human chimeric antibody gene into a fertilized egg and developing it, the transgenic non-human animal can be also prepared. Regarding the preparation method of a human antibody from the human antibody-producing transgenic non-human animal, the human antibody can be produced and accumulated in a culture by obtaining a human antibody-producing hybridoma by a hybridoma preparation method usually carried out in non-human mammals and then culturing it.

The transgenic non-human animal includes cattle, sheep, goat, pig, horse, mouse, rat, fowl, monkey, rabbit and the like.

Also, in the present invention, it is preferred that the antibody is an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises a part of Fc region of an antibody. The Fc region is a region at the C-terminal of H chain of an antibody, and includes a natural type and a mutant type. The part of Fc region of the IgG class is from Cys at position 226 to the C-terminal or from Pro at position 230 to the C-terminal according to the numbering of EU Index of *Kabat et al.* [*Sequences of Proteins of Immunological Interest,* 5$^{th}$ Ed., Public Health Service, National Institutes of Health, Bethesda, MD. (1991)]. Examples include an antibody, an antibody fragment, a fusion protein comprising an Fc region, and the like. The antibody fragment includes an H chain monomer, an H chain dimer and the like.

A fusion protein comprising a part of Fc region is a protein in which an antibody comprising the Fc region of an antibody or the antibody fragment is fused with a protein such as an enzyme or a cytokine (hereinafter referred to as "Fc fusion protein").

The present invention is explained below in detail.

1. Preparation of cell of the present invention

The cell of the present invention can be prepared by the following techniques.

(1) Gene disruption technique which comprises targeting gene encoding enzyme

The cell of the present invention can be prepared by using a gene disruption technique by targeting a gene

encoding 1,6-fucose modifying enzyme. The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.

The gene disruption method may be any method, so long as it can disrupt the gene of the target enzyme is included. Examples include a homologous recombination method, an RNA-DNA oligonucleotide (RDO) method, a method using retrovirus, a method using transposon, and the like. The methods are specifically described below.

(a) Preparation of antibody-producing cell of the present invention by homologous recombination

The cell of the present invention can be produced by modifying a target gene on chromosome through a homologous recombination technique for targeting a gene encoding the α1,6-fucose modifying enzyme.

The target gene on the chromosome can be modified by using a method described in *Manipulating the Mouse Embryo, A Laboratory Manual,* Second Edition, Cold Spring Harbor Laboratory Press (1994) (hereinafter referred to as "*Manipulating the Mouse Embryo, A Laboratory Manual"*); *Gene Targeting, A Practical Approach,* IRL Press at Oxford University Press (1993); *Biomanual Series 8, Gene Targeting, Preparation of Mutant Mice using ES Cells,* Yodosha (1995) (hereinafter referred to as *"Preparation of Mutant Mice using ES Cells"*); or the like, for example, as follows.

A cDNA encoding the α1,6-fucose modifying enzyme is prepared.

Based on the obtained cDNA, a genomic DNA encoding the α1,6-fucose modifying enzyme is prepared.

Based on the nucleotide sequence of the genomic DNA, a target vector is prepared for homologous recombination of a target gene to be modified (e.g., structural gene of the α1,6-fucose modifying enzyme, or a promoter gene).

The host cell of the present invention can be produced by introducing the prepared target vector into a host cell and selecting a cell in which homologous recombination occurred between the target gene and target vector.

As the host cell, any cell such as yeast, an animal cell, an insect cell or a plant cell can be used, so long as it has the target gene encoding the α1,6-fucose modifying enzyme. Examples include cells described in the following item 3.

The method for obtaining a cDNA or a genomic DNA encoding the α1,6-fucosyltransferase includes the method described below.

<u>Preparation method of cDNA:</u>

A total RNA or mRNA is prepared from various host cells.

A cDNA library is prepared from the prepared total RNA or mRNA.

Degenerative primers are produced based on the α1,6-fucose modifying enzyme, e.g., human amino acid sequence, and a gene fragment encoding the α1,6-fucose modifying enzyme is obtained by PCR using the prepared cDNA library as the template.

A cDNA encoding the α1,6-fucose modifying enzyme can be obtained by screening the cDNA library by using the obtained gene fragment as a probe.

As the mRNA of various cells, a commercially available product (e.g., manufactured by Clontech) may be used or may be prepared from various host cells as follows. The method for preparing a total RNA from various host cells includes the guanidine thiocyanate-cesium trifluoroacetate method [*Methods in Enzymology,* <u>154,</u> 3 (1987)], the acidic guanidine thiocyanate phenol chloroform (AGPC) method [*Analytical Biochemistry,* <u>162,</u> 156 (1987); *Experimental Medicine (Jikken Igaku),* <u>9,</u> 1937 (1991)] and the like.

Furthermore, the method for preparing mRNA as poly(A)$^+$ RNA from a total RNA includes the oligo(dT)-immobilized cellulose column method (*Molecular Cloning, Second Edition*) and the like.

In addition, mRNA can be prepared by using a kit such as Fast Track mRNA Isolation Kit (manufactured by Invitrogen), Quick Prep mRNA Purification Kit (manufactured by Pharmacia) or the like.

A cDNA library is prepared from the prepared mRNA of a human or non-human animal tissue or cell. The method for preparing cDNA libraries includes the methods described in *Molecular Cloning, Second Edition; Current Protocols in Molecular Biology; A Laboratory Manual,* Second Edition (1989); and the like, or methods using commercially available kits such as Superscript Plasmid System for cDNA Synthesis and Plasmid Cloning (manufactured by Life Technologies), ZAP-cDNA Synthesis Kit (manufactured by STRATAGENE) and the like.

As the cloning vector for the preparation of the cDNA library, any vector such as a phage vector, a plasmid vector or the like can be used, so long as it is autonomously replicable in *Escherichia coli* K12. Examples include ZAP Express [manufactured by STRATAGENE, *Strategies,* <u>5</u>, 58 (1992)], pBluescript II SK(+) [*Nucleic Acids Research,* <u>17,</u> 9494 (1989)], Lambda ZAP II (manufactured by STRATAGENE), λgt10 and λgt11 [DNA Cloning, A Practical Approach, <u>1</u>, 49 (1985)], λTriplEx (manufactured by Clontech), λExCell (manufactured by Pharmacia), pcD2 [*Mol. Cell. Biol.,* <u>3</u>, 280 (1983)], pUC18 [*Gene,* <u>33</u>, 103 (1985)] and the like.

Any microorganism can be used as the host microorganism for preparing the cDNA library, and *Escherichia*

*coli* is preferably used. Examples include *Escherichia coli* XLI-Blue MRF' [manufactured by STRATAGENE, *Strategies,* 5, 81 (1992)], *Escherichia coli* C600 [*Genetics,* 39, 440 (1954)], *Escherichia coli* Y1088 [*Science,* 222, 778 (1983)], *Escherichia coli* Y1090 [*Science,* 222, 778 (1983)], *Escherichia coli* NM522 [*J. Mol. Biol.,* *166,* 1 (1983)], *Escherichia coli* K802 [*J. Mol. Biol.,* 16, 118 (1966)], *Escherichia coli* JM105 [*Gene,* 38, 275 (1985)] and the like.

The cDNA library can be used as such in the subsequent analysis, and in order to obtain a full length cDNA as efficient as possible by decreasing the ratio of an infull length cDNA, a cDNA library prepared by using the oligo cap method developed by Sugano *et al*. [*Gene,* 138, 171 (1994); *Gene,* 200, 149 (1997); *Protein, Nucleic Acid, Protein*, 41, 603 (1996); *Experimental Medicine (Jikken Igaku),* 11, 2491 (1993); *cDNA Cloning* (Yodo-sha) (1996); *Methods for Preparing Gene Libraries* (Yodo-sha) (1994)] can be used in the following analysis.

Based on the amino acid sequence of the α1,6-fucose modifying enzyme, degenerative primers specific for the 5'-terminal and 3'-terminal nucleotide sequences of a nucleotide sequence presumed to encode the amino acid sequence are prepared, and DNA is amplified by PCR [*PCR Protocols,* Academic Press (1990)] using the prepared cDNA library as the template to obtain a gene fragment encoding the α1,6-fucose modifying enzyme.

It can be confirmed that the obtained gene fragment is a DNA encoding the α1,6-fucose modifying enzyme by a method generally used for analyzing a nucleotide, such as the dideoxy method of *Sanger et al.* [*Proc. Natl. Acad. Sci. USA,* 74, 5463 (1977)], a nucleotide sequence analyzer such as ABIPRISM 377 DNA Sequencer (manufactured by PE Biosystems) or the like.

A DNA encoding the α1,6-fucose modifying enzyme can be obtained by carrying out colony hybridization or plaque hybridization (*Molecular Cloning, Second Edition*) for the cDNA or cDNA library synthesized from the mRNA contained in the human or non-human animal tissue or cell, using the gene fragment as a DNA probe.

Also, a DNA encoding the α1,6-fucose modifying enzyme can also be obtained by carrying out screening by PCR using the cDNA or cDNA library synthesized from the mRNA contained in a human or non-human animal tissue or cell as the template and using the primers used for obtaining the gene fragment encoding the α1,6-fucose modifying enzyme.

The nucleotide sequence of the obtained DNA encoding the α1,6-fucose modifying enzyme is analyzed from its terminus and determined by a method generally used for analyzing a nucleotide, such as the dideoxy method of Sanger *et al.* [*Proc. Natl. Acad. Sci. USA,* 74, 5463 (1977)], a nucleotide sequence analyzer such as ABIPRISM 377 DNA Sequencer (manufactured by PE Biosystems) or the like.

A gene encoding the α1,6-fucose modifying enzyme can also be determined from genes in data bases by searching nucleotide sequence data bases such as GenBank, EMBL, DDBJ and the like by using a homology retrieving program such as BLAST based on the determined cDNA nucleotide sequence.

The nucleotide sequence of the gene encoding the α1,6-fucose modifying enzyme includes the nucleotide sequence represented by SEQ ID NO:1 or 2.

The cDNA encoding the α1,6-fucose modifying enzyme can also be obtained by chemically synthesizing it with a DNA synthesizer such as DNA Synthesizer model 392 manufactured by Perkin Elmer or the like by using the phosphoamidite method, based on the determined DNA nucleotide sequence.

As an example of the method for preparing a genomic DNA encoding the α1,6-fucose modifying enzyme, the method described below is exemplified.

Preparation method of genomic DNA:

The method for preparing genomic DNA includes known methods described in *Molecular Cloning, Second Edition; Current Protocols in Molecular Biology;* and the like. In addition, a genomic DNA encoding the α1,6-fucose modifying enzyme can also be isolated by using a kit such as Genome DNA Library Screening System (manufactured by Genome Systems), Universal Genome Walker™ Kits (manufactured by CLONTECH) or the like.

The nucleotide sequence of the genomic DNA encoding the α1,6-fucose modifying enzyme includes the nucleotide sequence represented by SEQ ID NO:3.

The target vector used in the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The target vector can be used as both a replacement type and an insertion type.

For introducing the target vector into various host cells, the methods for introducing recombinant vectors suitable for various host cells described in the following item 3 can be used.

The method for efficiently selecting a homologous recombinant includes a method such as the positive selection, promoter selection, negative selection or polyA selection described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The method for selecting the homologous recombinant of interest from the selected clones includes the Southern hybridization method for genomic DNA (*Molecular Cloning, Second Edition*),

PCR [*PCR Protocols,* Academic Press (1990)], and the like.

A homologous recombinant can be obtained based on the change of the activity of an enzyme relating to the modification of a sugar chain wherein 1-position of fucose is bound to 6-position of *N*-acetylglucosamine in the reducing end through α-bond in the complex *N*-glycoside-linked sugar chain. The following method is exemplified as a method for selecting a transformant as described below.

Method for selecting transformant:

The method for selecting a cell in which the activity of the α1,6-fucose modifying enzyme is decreased or deleted includes biochemical methods or genetic engineering techniques described in *New Biochemical Experimentation Series 3-Saccharides I, Glycoprotein* (Tokyo Kagaku Dojin), edited by Japanese Biochemical society (1988); *Cell Engineering, Supplement, Experimental Protocol Series, Glycobiology Experimental Protocol, Glycoprotein, Glycolipid and Proteoglycan* (Shujun-sha), edited by Naoyuki Taniguchi, Akemi Suzuki, Kiyoshi Furukawa and Kazuyuki Sugawara (1996); *Molecular Cloning, Second Edition; Current Protocols in Molecular Biology;* and the like. The biochemical method includes a method in which the enzyme activity is evaluated by using an enzyme-specific substrate and the like. The genetic engineering technique includes the Northern analysis, RT-PCR and the like which measures the amount of mRNA of a gene encoding the enzyme.

The method for confirming that the lectin-resistant cell includes a method for confirming expression of GDP-fucose synthase, α1,6-fucose modifying enzyme or GDP-fucose transport enzyme, a method for culturing cells in a medium to which lectin is directly added. Specifically, the expression amount of mRNA of α1,6-fucosyltransferase which is one of α1,6-fucose modifying enzymes in the cells. Cells in which the activity of the α1,6-fucose modifying enzyme is decreased are resistant to lectin.

Furthermore, the method for selecting a cell based on morphological change caused by decrease of the activity of the α1,6-fucose modifying agent includes a method for selecting a transformant based on the sugar chain structure of a produced antibody molecule, a method for selecting a transformant using the sugar chain structure of a glycoprotein on a cell membrane, and the like. The method for selecting a transformant using the sugar chain structure of an antibody-producing molecule includes method described in the following item 5. The method for selecting a transformant using the sugar chain structure of a glycoprotein on a cell membrane includes a method selecting a clone resistant to a lectin which recognizes a sugar chain structure wherein 1-position of fucose is bound to 6-position of *N*-acetylglucosamine in the reducing end through α-bond in the complex *N*-glycoside-linked sugar chain. Examples include a method using a lectin described in *Somatic Cell Mol. Genet.,* 12, 51 (1986).

As the lectin, any lectin can be used, so long as it is a lectin which recognizes a sugar chain structure in which 1-position of fucose is bound to 6-position of *N*-acetylglucosamine in the reducing end through α-bond in the *N*-glycoside-linked sugar chain. Examples include a *Lens culinaris* lectin LCA (lentil agglutinin derived from *Lens culinaris),* a pea lectin PSA (pea lectin derived from *Pisum sativum),* a broad bean lectin VFA (agglutinin derived from *Vicia faba),* an *Aleuria aurantia* lectin AAL (lectin derived from *Aleuria aurantia*) and the like.

Specifically, the host cell of the present invention can be selected by culturing cells for 1 day to 2 weeks, preferably 3 days to 1 week, in a medium comprising the lectin at a concentration of several ten μg/ml to 10 mg/ml, preferably 0.5 to 2.0 mg/ml, subculturing surviving cells or picking up a colony and transferring it into a culture vessel, and subsequently continuing the culturing in the lectin-containing medium.

(b) Preparation of cell of the present invention by RDO method

The cell of the present invention can be prepared by an RDO method by targeting a gene encoding the α1,6-fucose modifying enzyme, for example, as follows.

A cDNA or a genomic DNA encoding the α1,6-fucose modifying enzyme is prepared.

The nucleotide sequence of the prepared cDNA or genomic DNA is determined.

Based on the determined DNA sequence, an RDO construct of an appropriate length comprising a part of a translation region, a part of an untranslated region or a part of intron of the target gene, is designed and synthesized.

The cell of the present invention can be obtained by introducing the synthesized RDO into a host cell and then selecting a transformant in which a mutation occurred in the target enzyme, namely the α1,6-fucose modifying enzyme.

As the host cell, any cell such as yeast, an animal cell, an insect cell or a plant cell can be used, so long as it has a gene encoding the α1,6-fucose modifying enzyme. Examples include the host cells described in the following item 3.

The method for introducing RDO into various host cells includes the methods for introducing recombinant vectors suitable for various host cells, described in the following item 3.

The method for preparing cDNA encoding the α1,6-fucose modifying enzyme includes the methods for "Preparation method of cDNA" described in the item 1(1)(a) and the like.

The method for preparing a genomic DNA encoding the α1,6-fucose modifying enzyme includes the methods for "Preparation method of genomic DNA" described in the item 1(1)(a) and the like.

The nucleotide sequence of the DNA can be determined by digesting it with appropriate restriction enzymes, cloning the DNA fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad. Sci. USA,* 74 , 5463 (1977)] or the like, and then analyzing the clones by using an automatic nucleotide sequence analyzer such as A.L.F. DNA Sequencer (manufactured by Pharmacia) or the like.

The RDO can be prepared by a usual method or using a DNA synthesizer.

The method for selecting a cell in which a mutation occurred, by introducing the RDO into the host cell, in the target enzyme, the gene encoding the α1,6-fucosyltransferase, includes the methods for directly detecting mutations in chromosomal genes described in *Molecular Cloning, Second Edition, Current Protocols in Molecular Biology* and the like.

Furthermore, "Method for selecting transformant" described in the item 1(1)(a) based on the change of the activity of the α1,6-fucose modifying enzyme can also be used.

The construct of the RDO can be designed in accordance with the methods described in *Science,* 273, 1386 (1996); *Nature Medicine,* 4, 285 (1998); *Hepatology,* 25, 1462 (1997); *Gene Therapy,* 5, 1960 (1999); *J. Mol. Med.,* 75, 829 (1997); *Proc. Natl. Acad. Sci. USA,* 96 8774 (1999); *Proc. Natl. Acad. Sci. USA,* 96, 8768 (1999); *Nuc. Acids. Res.,* 27, 1323 (1999); *Invest. Dematol.,* 111, 1172 (1998); *Nature Biotech.,* 16, 1343 (1998); *Nature Biotech.,* 18, 43 (2000); *Nature Biotech.,* 18, 555 (2000); and the like.

(c) Preparation of cell of the present invention by method using transposon

The cell of the present invention can be prepared by inducing mutation using a transposon system described in *Nature Genet.,* 25, 35 (2000) or the like, and then by selecting a mutant based on the activity of the α1,6-fucose modifying enzyme, or the sugar chain structure of a produced antibody molecule or of a glycoprotein on the cell membrane.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a host cell.

As the cell, any cell such as yeast, an animal cell, an insect cell or a plant cell can be used, so long as it has a gene encoding the target α1,6-fucose modifying enzyme. Examples include the host cells described in the following item 3.

For introducing a gene encoding an antibody molecule into the cell of the present invention as a host cell, the method for introducing recombinant vectors suitable for various host cells described in the following item 3 can be used.

The method for selecting a mutant based on the activity of the α1,6-modifying enzyme includes "Method for selecting transformant" described in the item 1(1)(a) based on change of the activity of the α1,6-fucose modifying enzyme.

(d) Preparation of cell of the present invention by antisense method or ribozyme method

The cell of the present invention can be prepared by the antisense method or the ribozyme method described in *Cell Technology,* 12, 239 (1993); *BIO/TECHNOLOGY,* 17, 1097 (1999); *Hum. Mol. Genet.,* 5, 1083 (1995); *Cell Technology,* 13, 255 (1994); *Proc. Natl. Acad. Sci. USA,* 96 1886 (1999); or the like, e.g., in the following manner by targeting the gene encoding the α1,6-fucose modifying enzyme.

A cDNA or a genomic DNA of the target gene is prepared.

The nucleotide sequence of the prepared cDNA or genomic DNA is determined.

Based on the determined DNA sequence, an antisense gene or ribozyme construct of an appropriate length comprising a part of a translation region, a part of an untranslated region or a part of an intron of the target gene is designed.

In order to express the antisense gene or ribozyme in a cell, a recombinant vector is prepared by inserting a fragment or total length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the

protein encoded by the target gene. The cell of the present invention can also be obtained by selecting a transformant based on the sugar chain structure of a glycoprotein on the cell membrane or the sugar chain structure of the produced antibody molecule.

As the host cell used for the production of the cell of the present invention, any cell such as yeast, an animal cell, an insect cell or a plant cell can be used, so long as it has the target gene. Examples include host cells described in the following item 3.

As the expression vector, a vector which is autonomously replicable in the host cell or can be integrated into the chromosome and comprises a promoter at such a position that the designed antisense gene or ribozyme can be transferred is used. Examples include expression vectors described in the following item 3.

As the method for introducing a gene into various host cells, the methods for introducing recombinant vectors suitable for various host cells described in the following item 3 can be used.

The method for obtaining the cDNA or genomic DNA of the target gene includes the methods described in "Preparation method of cDNA" and "Preparation method of genomic DNA" in the item 1(1)(a).

The method for selecting based on the activity of the protein encoded by the target gene includes the methods described in "Method for selecting transformant" in the item 1(1)(a).

Furthermore, the method for selecting a cell based on morphological change caused by decrease of the activity of a protein encoded by the target gene includes a method for selecting a transformant based on the sugar chain structure of a produced antibody molecule, a method for selecting a transformant based on the sugar chain structure of a glycoprotein on a cell membrane, and the like. The method for selecting a transformant based on the sugar chain structure of an antibody-producing molecule includes the method described in the following item 5. The method for selecting a transformant based on the sugar chain structure of a glycoprotein on a cell membrane includes the above method selecting a strain resistant to a lectin which recognizes a sugar chain structure wherein 1-position of fucose is bound to 6-position of *N*-acetylglucosamine in the reducing end through α-bond in the complex *N*-glycoside-linked sugar chain. Examples include a method using a lectin described in *Somatic Cell Mol. Genet.,* 12, 51 (1986).

Furthermore, the cell of the present invention can also be obtained without using an expression vector, by directly introducing an antisense oligonucleotide or ribozyme into a host cell, which is designed based on the nucleotide sequence of the target gene.

The antisense oligonucleotide or ribozyme can be prepared by the usual method or using a DNA synthesizer. Specifically, it can be prepared based on the sequence information of an oligonucleotide having a corresponding sequence of continued 5 to 150 bases, preferably 5 to 60 bases, and more preferably 10 to 40 bases, among nucleotide sequences of a cDNA and a genomic DNA of the target gene by synthesizing an oligonucleotide which corresponds to a sequence complementary to the oligonucleotide (antisense oligonucleotide) or a ribozyme comprising the oligonucleotide sequence.

The oligonucleotide includes oligo RNA and derivatives of the oligonucleotide (hereinafter referred to as "oligonucleotide derivatives").

The oligonucleotide derivatives includes oligonucleotide derivatives in which a phosphodiester bond in the oligonucleotide is converted into a phosphorothioate bond, an oligonucleotide derivative in which a phosphodiester bond in the oligonucleotide is converted into an N3'-P5' phosphoamidate bond, an oligonucleotide derivative in which ribose and a phosphodiester bond in the oligonucleotide are converted into a peptide-nucleic acid bond, an oligonucleotide derivative in which uracil in the oligonucleotide is substituted with C-5 propynyluracil, an oligonucleotide derivative in which uracil in the oligonucleotide is substituted with C-5 thiazoleuracil, an oligonucleotide derivative in which cytosine in the oligonucleotide is substituted with C-5 propynylcytosine, an oligonucleotide derivative in which cytosine in the oligonucleotide is substituted with phenoxazine-modified cytosine, an oligonucleotide derivative in which ribose in the oligonucleotide is substituted with 2'-*O*-propylribose and an oligonucleotide derivative in which ribose in the oligonucleotide is substituted with 2'-methoxyethoxyribose [*Cell Technology (Saibo Kogaku),* 16, 1463 (1997)].

(e) Preparation of cell of the present invention by RNAi method

The cell of the present invention can be prepared by the RNAi (RNA interference) method by targeting a gene encoding the protein of the present invention, for example, as follows. The RNAi method means a method in which double stranded RNA is introduced into a cell and mRNA present in the cell homologous to the sequence of the RNA is decomposed and destroyed to thereby inhibit gene expression.

A cDNA of the target gene is prepared.

The nucleotide sequence of the prepared cDNA is determined.

Based on the determined DNA sequence, an RNAi gene construct of an appropriate length comprising a part of the translation region or untranslated region of the target gene, is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the protein encoded by the target gene or the sugar chain structure of the produced antibody molecule or of a glycoprotein on the cell membrane.

As the host cell, any cell such as yeast, an animal cell, an insect cell or a plant cell can be used, so long as it has a gene encoding the target of the produced antibody molecule. Examples include the host cells described in the following item 3.

As the expression vector, a vector which is autonomously replicable in the host cell or can be integrated into the chromosome and comprises a promoter at such a position that the designed RNAi gene can be transferred is used. Examples include the expression vectors described in the following item 3.

As the method for introducing a gene into various host cells, the methods for introducing recombinant vectors suitable for various host cells described in the following item 3 can be used.

The method for selecting a transformant based on the activity of the protein encoded by the target gene or the method for selecting a transformant based on the sugar chain structure of a glycoprotein on the cell membrane includes the method described in the item 1(1)(a). The method for selecting a transformant based on the sugar chain structure of a produced antibody molecule includes the method described in the following item 5.

The method for preparing cDNA of the protein encoded by the target gene includes the method described in "Preparation method of cDNA" in the item 1(1)(a) and the like.

Furthermore, the cell of the present invention can also be obtained without using an expression vector, by directly introducing an RNAi gene designed based on the nucleotide sequence of the target gene.

The RNAi gene can be prepared by the usual method or using a DNA synthesizer.

The RNAi gene construct can be designed in accordance with the methods described in *Nature,* 391, 806 (1998); *Proc. Natl. Acad. Sci. USA,* 95, 15502 (1998); *Nature,* 395, 854 (1998); *Proc. Natl. Acad. Sci. USA,* 96, 5049 (1999); *Cell,* 95, 1017 (1998); *Proc. Natl. Acad. Sci. USA,* 96, 1451 (1999); *Proc. Natl. Acad. Sci. USA,* 95, 13959 (1998); *Nature Cell Biol.,* 2, 70 (2000); and the like.

The RNA used in the RNAi method of the present invention includes RNA corresponding to DNA encoding an enzyme protein relating to the modification of a sugar chain wherein 1-position of fucose is bound to 6-position of *N*-acetylglucosamine in the reducing end through α-bond in the complex *N*-glycoside-linked sugar chain, and is preferably RNA corresponding to DNA encoding α1,6-fucosyltransferase as the enzyme protein relating to the modification of a sugar chain wherein 1-position of fucose is bound to 6-position of *N*-acetylglucosamine in the reducing end through α-bond in the complex *N*-glycoside-linked sugar chain.

As the RNA used in the RNAi method of the present invention, any RNA can be used, so long as it is a double stranded RNA consisting of RNA and its complementary RNA and capable of decreasing the amount of mRNA of α1,6-fucosyltransferase. Regarding the length of the RNA, the RNA is a continuous RNA of preferably 1 to 30, more preferably 5 to 29, still more preferably 10 to 29, and most preferably 15 to 29.

(2) Preparation of cell of the present invention by method for introducing dominant negative mutant

The cell of the present invention can be prepared by targeting the α1,6-fucose modifying enzyme by using a technique for introducing a dominant negative mutant of the protein.

As the dominant negative mutant, a protein relating to transport of an intracellular sugar nucleotide, GDP-fucose, to the Golgi body, is exemplified.

It is known that a transporter of an intracellular sugar nucleotide functions in the form of a dimer on the membrane of endoplasmic reticulum or the Golgi body [*J. Biol. Chem.,* 275, 17718 (2000)]. Also, it is reported that, when a mutant of a transporter of an intracellular sugar nucleotide is compulsorily expressed intracellularly, a heterodimer is formed with a wild type transporter, and the formed heterodimer has an activity to inhibit a wild type homodimer [*J. Biol. Chem.,* 275, 17718 (2000)]. Accordingly, a mutant of a transporter of an intracellular sugar nucleotide is prepared and introduced into a cell so that it can function as a dominant negative mutant. The mutant can be prepared by using site-directed mutagenesis method described in *Molecular Cloning, Second Edition, Current Protocols in Molecular Biology* and the like.

The cell of the present invention can be prepared by using the prepared dominant negative mutant gene of the α1,6-fucose modifying enzyme prepared in the above according to the method described in *Molecular Cloning, Second Edition, Current Protocols in Molecular Biology, Manipulating the Mouse Embryo* or the like, for example, as follows.

A dominant negative mutant gene of the α1,6-fucose modifying enzyme is prepared.

Based on the prepared full length DNA of the dominant negative mutant gene, a DNA fragment of an appropriate length containing a moiety encoding the protein is prepared, if necessary.

A recombinant vector is prepared by inserting the DNA fragment or full length DNA into downstream of the promoter of an appropriate expression vector.

A transformant is obtained by introducing the recombinant vector into a host cell suitable for the expression vector.

The host cell of the present invention can be prepared by selecting a transformant based on the activity of the α1,6-fucose modifying enzyme or the sugar chain structure of a produced antibody molecule or of a glycoprotein on the cell membrane.

As the host cell, any cell such as yeast, an animal cell, an insect cell or a plant cell can be used, so long as it has the gene encoding the target protein. Examples include the host cells described in the following item 3.

As the expression vector, a vector which is autonomously replicable in the host cell or can be integrated into the chromosome and comprises a promoter at a position where transcription of the DNA encoding the dominant negative mutant of interest can be effected is used. Examples include the expression vectors described in the following item 3.

For introducing the gene into various host cells, the method for introducing recombinant vectors suitable for various host cells described in the following item 3 can be used.

The method for selecting a mutant based on the activity of the target protein or the method for selecting a mutant based on the sugar chain structure of a glycoprotein on the cell membrane includes the method described in the above item 1(1). The method for selecting a mutant based on the sugar chain structure of a produced antibody molecule includes the methods described in the following item 4.

(3) Method for introducing mutation into enzyme

The cell of the present invention can be prepared by introducing a mutation into a gene encoding the α1,6-fucose modifying enzyme, and then by selecting a clone of interest in which the mutation occurred in the enzyme.

The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.

The method includes 1) a method in which a desired clone is selected from mutants obtained by a mutation-inducing treatment of a parent cell line with a mutagen or spontaneously generated mutants based on the activity of the α1,6-fucose modifying enzyme, 2) a method in which a desired clone is selected from mutants obtained by a mutation-inducing treatment of a parent cell line with a mutagen or spontaneously generated mutants based on the sugar chain structure of a produced antibody molecule and 3) a method in which a desired clone is selected from mutants obtained by a mutation-inducing treatment of a parent cell line with a mutagen or spontaneously generated mutants based on the sugar chain structure of a glycoprotein on the cell membrane.

As the mutation-inducing treatment, any treatment can be used, so long as it can induce a point mutation, a deletion or frame shift mutation in the DNA of the parent cell line. Examples include treatment with ethyl nitrosourea, nitrosoguanidine, benzopyrene or an acridine pigment and treatment with radiation. Also, various alkylating agents and carcinogens can be used as mutagens. The method for allowing a mutagen to act upon cells includes the methods described in *Tissue Culture Techniques,* 3rd edition (Asakura Shoten), edited by Japanese Tissue Culture Association (1996), *Nature Genet.,* 24, 314 (2000) and the like.

The spontaneously generated mutant includes mutants which are spontaneously formed by continuing subculture under general cell culture conditions without applying special mutation-inducing treatment.

The method for selecting a clone of interest based on the activity of the α1,6-fucose modifying enzyme, the method for selecting a sugar chain of interest based on the sugar chain structure of a prepared antibody molecule and the method for selecting a clone of interest based on the sugar chain structure of a glycoprotein on the cell membrane include "Method for selecting transformant" described in the item 1(1)(a) based on change of the activity of the α1,6-fucose modifying enzyme.

(4) Method for inhibiting transcription or translation of GDP-fucose transport protein

The host cell of the present invention can be prepared by targeting a gene encoding the α1,6-fucose modifying enzyme and inhibiting transcription or translation of the target gene using the antisense RNA/DNA technique [*Bioscience and Industry,* 50, 322 (1992); *Chemistry (Kagaku),* 46, 681 (1991); *Biotechnology,* 9, 358 (1992); *Trends in Biotechnology,* 10, 87 (1992), *Trends in Biotechnology,* 10, 152 (1992), *Cell Technology,* 16, 1463 (1997)], triple-herix technique [*Trends in Biotechnology,* 10, 132 (1992)] or the like.

2. Preparation of transgenic non-human animal or plant or the progenies thereof of the present invention

The transgenic non-human animal or plant or the progenies thereof of the present invention is a transgenic non-human animal or plant or the progenies thereof in which a genomic gene is modified in such a manner that the activity of an enzyme relating to the modification of a sugar chain of an antibody molecule can be controlled, and it can be prepared from an embryonic stem cell, fertilized egg cell or plant cell according to the method described in the item 1, by targeting a gene encoding the α1,6-fucose modifying enzyme.

The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.

A specific method is described below.

In a transgenic non-human animal, the embryonic stem cell of the present invention in which the activity of the α1,6-fucose modifying enzyme is controlled can be prepared by applying the method described in the item 1 to an embryonic stem cell of the intended non-human animal such as cattle, sheep, goat, pig, horse, mouse, rat, fowl, monkey, rabbit or the like.

Specifically, a mutant clone is prepared in which a gene encoding the α1,6-fucose modifying enzyme is inactivated or substituted with any sequence, by a known homologous recombination technique [e.g., *Nature,* 326, 6110, 295 (1987); *Cell,* 51, 3, 503 (1987); or the like]. Using the prepared mutant clone, a chimeric individual comprising an embryonic stem cell clone and a normal cell can be prepared by an injection chimera method into blastocyst of fertilized egg of an animal or by an aggregation chimera method. The chimeric individual is crossed with a normal individual, so that a transgenic non-human animal in which the activity of the α1,6-fucose modifying enzyme is decreased in the whole body cells can be obtained.

The target vector for the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995) or the like. The target vector can be used as any of a replacement type, an insertion type and a gene trap type.

As the method for introducing the target vector into the embryonic stem cell, any method can be used, so long as it can introduce DNA into an animal cell. Examples include electroporation [*Cytotechnology,* 3, 133 (1990)], the calcium phosphate method (Japanese Published Unexamined Patent Application No. 227075/90), the lipofection method [*Proc. Natl. Acad. Sci. USA,* 84, 7413 (1987)], the injection method [*Manipulating the Mouse Embryo, A Laboratory Manual*], a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method [*Manipulating Mouse Embryo,* Second Edition] and the like.

The method for efficiently selecting a homologous recombinant includes a method such as the positive selection, promoter selection, negative selection or polyA selection described in *Gene Targeting, A Practical Approach,* IRL Press at Oxford University Press (1993), or the like. Specifically, in the case of the target vector containing hprt gene, it is introduced into the hprt gene-defected embryonic stem cell, the embryonic stem cell is cultured in a medium containing aminopterin, hypoxanthine and thymidine, and positive selection which selects the homologous recombinant of the hprt gene can be carried out by selecting a homogenous recombinant containing an aminopterin-resistant clone. In the case of the target vector containing a neomycin-resistant gene, the vector-introduced embryonic stem cell is cultured in a medium containing G418, and positive selection can be carried out by selecting a homogenous recombinant containing a neomycin-resistant gene. In the case of the target vector containing DT gene, the vector-introduced embryonic stem cell is cultured, and negative a DT gene-free homogenous recombinant can be carried out by selecting the grown clone (since the DT gene is expressed while integrated in the chromosome, the recombinants introduced into a chromosome at random other than the homogenous recombination cannot grow due to the toxicity of DT). The method for selecting the homogenous recombinant of interest among the selected clones include the Southern hybridization for genomic DNA (*Molecular Cloning,* Second Edition), PCR [*PCR Protocols,* Academic Press (1990)] and the like.

When the embryonic stem cell is introduced into a fertilized egg by using an aggregation chimera method, in general, a fertilized egg at the development stage before 8-cell stage is preferably used. When the embryonic stem cell is introduced into a fertilized egg by using an injection chimera method, in general, it is preferred that a fertilized egg at the development stage from 8-cell stage to blastocyst stage is preferably used.

When the fertilized egg is transplanted into a female mouse, it is preferred that a fertilized egg obtained from a pseudopregnant female mouse in which fertility is induced by mating with a male non-human mammal which is subjected to vasoligation is artificially transplanted or implanted. Although the psuedopregnant female mouse can beobtained by natural mating, the pseudopregnant female mouse in which fertility is induced can be obtained by mating with a male mouse after administration of a luteinizing hormone-releasing hormone (hereinafter referred to as "LHRH")

or its analogue thereof. The analogue of LHRH includes [3,5-Dil-Tyr5]-LHRH, [Gln8]-LHRH, [D-Ala6]-LHRH, des-Gly10-[D-His(Bzl)6]-LHRH ethylamide and the like.

Also, a fertilized egg cell of the present invention in which the activity of the α1,6-fucose modifying enzyme is decreased can be prepared by applying the method described in the item 1 to fertilized egg of a non-human animal of interest such as cattle, sheep, goat, pig, horse, mouse, rat, fowl, monkey, rabbit or the like.

A transgenic non-human animal in which the activity of the α1,6-fucose modifying enzyme is decreased can be prepared by transplanting the prepared fertilized egg cell into the oviduct or uterus of a pseudopregnant female by using the embryo transplantation method described in *Manipulating Mouse Embryo,* Second Edition or the like, followed by childbirth by the animal.

In a transgenic plant, the callus of the present invention in which the activity of the α1,6-fucose modifying enzyme is decreased can be prepared by applying the method described in the item 1 to a callus or cell of the plant of interest.

A transgenic plant in which the activity of α1,6-fucose modifying enzyme is decreased can be prepared by culturing the prepared callus using a medium comprising auxin and cytokinin to redifferentite it in accordance with a known method [*Tissue Culture,* 20 (1994); *Tissue Culture,* 21 (1995); *Trends in Biotechnology,* 15, 45 (1997)].

3. Method for producing antibody composition

The antibody composition can be obtained by expressing it in a host cell using the methods described in *Molecular Cloning, Second Edition; Current Protocols in Molecular Biology; Antibodies, A Laboratory Manual,* Cold Spring Harbor Laboratory, 1988 (hereinafter sometiems referred to as *"Antibodies"); Monoclonal Antibodies: Principles and Practice,* Third Edition, Acad. Press, 1993 (hereinafter sometiems referred to as *"Monoclonal Antibodies");* and *Antibody Engineering, A Practical Approach,* IRL Press at Oxford University Press (hereinafter sometiems referred to as *"Antibody Engineering"),* for example, as follows.

A cDNA of an antibody molecule is prepared.

Based on the prepared full length cDNA of an antibody molecule, an appropriate length of a DNA fragment comprising a moiety encoding the protein is prepared, if necessary.

A recombinant vector is prepared by inserting the DNA fragment or the full length cDNA into downstream of the promoter of an appropriate expression vector.

A transformant which produces the antibody molecule can be obtained by introducing the recombinant vector into a host cell suitable for the expression vector.

As the host cell, any of yeast, an animal cell, an insect cell, a plant cell or the like can be used, so long as it can express the gene of interest.

A cell such as yeast, animal cell, insect cell, plant cell or the like into which an enzyme relating to the modification of an *N*-glycoside-linked sugar chain which binds to the Fc region of the antibody molecule is introduced by a genetic engineering technique can also be used as the host cell.

The host cell used for the production of the antibody composition includes the cell of the present invention prepared in the above 1.

As the expression vector, a vector which is autonomously replicable in the host cell or can be integrated into the chromosome and comprises a promoter at such a position that the DNA encoding the antibody molecule of interest can be transferred is used.

The cDNA can be prepared from a human or non-human tissue or cell using, e.g., a probe primer specific for the antibody molecule of interest, in accordance with the methods described in "Preparation method of cDNA" in the item 1(1)(a).

When yeast is used as the host cell, the expression vector includes YEP13 (ATCC 37115), YEp24 (ATCC 37051), YCp50 (ATCC 37419) and the like.

Any promoter can be used, so long as it can function in yeast. Examples include a promoter of a gene of the glycolytic pathway such as a hexose kinase gene, PHO5 promoter, PGK promoter, GAP promoter, ADH promoter, gal 1 promoter, gal 10 promoter, heat shock protein promoter, MF α1 promoter, CUP 1 promoter and the like.

The host cell includes microorganisms belonging to the genus *Saccharomyces,* the genus *Schizosaccharomyces,* the genus *Kluyveromyces,* the genus *Trichosporon,* the genus *Schwanniomyces* and the like, such as *Saccharomyces cerevisiae, Schizosaccharomyces pombe, Kluyveromyces lactis, Trichosporon pullulans* and *Schwanniomyces alluvius.*

As the method for introducing the recombinant vector, any method can be used, so long as it can introduce DNA into yeast. Examples include electroporation [*Methods in Enzymology,* 194, 182 (1990)], the spheroplast method [*Proc. Natl. Acad. Sci. USA,* 84, 1929 (1978)], the lithium acetate method [*J. Bacteriol.,* 153, 163 (1983)], the method described in *Proc. Natl. Acad. Sci. USA,* 75, 1929 (1978) and the like.

When an animal cell is used as the host cell, the expression vector includes pcDNAI, pcDM8 (available from

Funakoshi), pAGE107 [Japanese Published Unexamined Patent Application No. 22979/91; *Cytotechnology,* 3, 133 (1990)], pAS3-3 (Japanese Published Unexamined Patent Application No. 227075/90), pCDM8 [*Nature,* 329, 840 (1987)], pcDNAI/Amp (manufactured by Invitrogen), pREP4 (manufactured by Invitrogen), PAGE103 [*J. Biochemistry,* 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SR$\alpha$ promoter and the like. Also, an enhancer of the IE gene of human CMV may be used together with the promoter.

The host cell includes a human cell such as Namalwa cell, a monkey cell such as COS cell, a Chinese hamster cell such as CHO cell or HBT5637 (Japanese Published Unexamined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology,* 3, 133 (1990)], the calcium phosphate method (Japanese Published Unexamined Patent Application No. 227075/90), the lipofection method [*Proc. Natl. Acad. Sci. USA,* 84, 7413 (1987)], the injection method [*Manipulating the Mouse Embryo, A Laboratory Manual*], a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method (*Manipulating Mouse Embryo,* Second Edition) and the like.

When an insect cell is used as the host, the protein can be expressed by the method described in *Current Protocols in Molecular Biology, Baculovirus Expression Vectors, A Laboratory Manual,* W.H. Freeman and Company, New York (1992), *Bio/Technology,* 6, 47 (1988) or the like.

The protein can be expressed by obtaining a recombinant virus in an insect cell culture supernatant and then infecting the insect cell with the recombinant virus.

The gene introducing vector used in the method includes pVL1392, pVL1393, pBlueBacIII (all manufactured by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [*Current Protocols in Molecular Biology, Baculovirus Expression Vectors, A Laboratory Manual,* W.H. Freeman and Company, New York (1992)], a *Trichoplusia ni* oocyte High 5 (manufactured by Invitrogen) and the like.

The method for the co-introducing the recombinant gene-introducing vector and the baculovirus for preparing the recombinant virus includes the calcium phosphate method (Japanese Published Unexamined Patent Application No. 227075/90), the lipofection method [*Proc. Natl. Acad. Sci. USA,* 84, 7413 (1987)] and the like.

When a plant cell is used as the host cell, the expression vector includes Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley and the like.

As the method for introducing the recombinant vector, any method can be used, so long as it can introduce DNA into a plant cell. Examples include a method using *Agrobacterium* (Japanese Published Unexamined Patent Application No. 140885/84, Japanese Published Unexamined Patent Application No. 70080/85, WO 94/00977), electroporation (Japanese Published Unexamined Patent Application No. 251887/85), a method using a particle gun (gene gun) (Japanese Patent No. 2606856, Japanese Patent No. 2517813) and the like.

As the method for expressing an antibody gene, secretion production, expression of a fusion protein of the Fc region with other protein and the like can be carried out in accordance with the method described in *Molecular Cloning, Second Edition* or the like, in addition to the direct expression.

When a gene is expressed by yeast, an animal cell, an insect cell or a plant cell into which a gene relating to the synthesis of a sugar chain is introduced, an antibody molecule to which a sugar or a sugar chain is added by the introduced gene can be obtained.

An antibody composition can be obtained by culturing the obtained transformant in a medium to produce and accumulate the antibody molecule in the culture and then recovering it from the resulting culture. The method for culturing the transformant using a medium can be carried out in accordance with a general method which is used for the culturing of host cells.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic material includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out generally under aerobic conditions such as shaking culture or submerged-aeration stirring culture. The culturing temperature is preferably 15 to 40°C, and the culturing time is generally 16 hours to 7 days. During the culturing, the pH is maintained at 3.0 to 9.0. The pH is adjusted using an inorganic or organic acid, an alkali solution, urea, calcium carbonate, ammonia or the like.

If necessary, an antibiotic such as ampicillin or tetracycline may be added to the medium during the culturing.

When a microorganism transformed with a recombinant vector obtained by using an inducible promoter as the promoter is cultured, an inducer may be added to the medium, if necessary. For example, when a microorganism transformed with a recombinant vector obtained by using *lac* promoter is cultured, isopropyl-β-D-thiogalactopyranoside may be added to the medium, and when a microorganism transformed with a recombinant vector obtained by using *trp* promoter is cultured, indoleacrylic acid may be added to the medium.

When a transformant obtained by using an animal cell as the host cell is cultured, the medium includes generally used RPMI 1640 medium [*The Journal of the American Medical Association,* 199, 519 (1967)], Eagle's MEM medium [*Science,* 122, 501 (1952)], Dulbecco's modified MEM medium [*Virology,* 8, 396 (1959)], 199 medium [*Proceeding of the Society for the Biological Medicine,* 73, 1 (1950)] and Whitten's medium [*Developmental Engineering Experimentation Manual-Preparation of Transgenic Mice* (Kodan-sha), edited by M. Katsuki (1987)], the media to which fetal calf serum, *etc.* is added, and the like.

The culturing is carried out generally at a pH of 6 to 8 and 30 to 40°C for 1 to 7 days in the presence of 5% $CO_2$.

If necessary, an antibiotic such as kanamycin or penicillin may be added to the medium during the culturing.

The medium for the culturing of a transformant obtained by using an insect cell as the host includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature,* 195, 788 (1962)] and the like.

The culturing is carried out generally at a medium pH of 6 to 7 and 25 to 30°C for 1 to 5 days.

In addition, antibiotics such as gentamicin may be added to the medium during the culturing, if necessary.

A transformant obtained by using a plant cell as the host cell can be cultured as a cell or after differentiating it into a plant cell or organ. The medium for culturing the transformant includes generally used Murashige and Skoog (MS) medium and White medium, the media to which a plant hormone such as auxin, cytokinin, *etc.* is added, and the like.

The culturing is carried out generally at a pH of 5 to 9 and 20 to 40°C for 3 to 60 days.

If necessary, an antibiotic such as kanamycin or hygromycin may be added to the medium during the culturing.

Accordingly, an antibody composition can be produced by culturing a transformant derived from yeast, an animal cell or a plant cell, which comprises a recombinant vector into which a DNA encoding an antibody molecule is inserted, in accordance with a general culturing method, to thereby produce and accumulate the antibody composition, and then recovering the antibody composition from the culture.

The method for producing an antibody composition includes a method of intracellular expression in a host cell, a method of extracellular secretion from a host cell, and a method of production on a host cell membrane outer envelope. The method can be selected by changing the host cell used or the structure of the antibody composition produced.

When the antibody composition of the present invention is produced in a host cell or on a host cell membrane outer envelope, it can be positively secreted extracellularly in accordance with the method of *Paulson et al.* [*J. Biol. Chem.,* 264, 17619 (1989)], the method of Lowe *et al.* [*Proc. Natl. Acad Sci. USA,* 86, 8227 (1989), *Genes Develop.,* 4, 1288 (1990)], the methods described in Japanese Published Unexamined Patent Application No. 336963/93 and Japanese Published Unexamined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, introducing the expression vector into the host cell and then expressing the antibody molecule.

Also, its production amount can be increased in accordance with the method described in Japanese Published Unexamined Patent Application No. 227075/90 by using a gene amplification system using a dihydrofolate reductase gene.

In addition, the antibody composition can also be produced by using a gene-introduced animal individual

(transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby produce and accumulate the antibody composition and then recovering the antibody composition from the animal or plant individual.

The method for producing an antibody composition by using an animal individual includes a method in which the antibody composition of interest is produced in an animal constructed by introducing a gene in accordance with a known method [*American Journal of Clinical Nutrition,* 63, 627S (1996); *Biol/Technology,* 9, 830 (1991)].

In the case of an animal individual, an antibody composition can be produced by rearing a transgenic non-human animal into which a DNA encoding an antibody molecule is introduced to thereby produce and accumulate the antibody composition in the animal, and then recovering the antibody composition from the animal. The place of the animal where the composition is produced and accumulated includes milk (Japanese Published Unexamined Patent Application No. 309192/88) and eggs of the animal. As the promoter used in this case, any promoter can be used, so long as it can function in an animal. Preferred examples include mammary gland cell-specific promoters such as α casein promoter, β casein promoter, β lactoglobulin promoter, whey acidic protein promoter and the like.

The method for producing an antibody composition by using a plant individual includes a method in which an antibody composition is produced by cultivating a transgenic plant into which a DNA encoding an antibody molecule is introduced by a known method [*Tissue Culture,* 20 (1994); *Tissue Culture,* 21 (1995); *Trends in Biotechnology,* 15, 45 (1997)] to produce and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted with ultrasonic oscillator, French press, Manton Gaulin homogenizer, dynomill or the like to obtain a cell-free extract. A purified product of the antibody composition can be obtained from a supernatant obtained by centrifuging the cell-free extract, by using a general enzyme isolation purification techniques such as solvent extraction; salting out and desalting with ammonium sulfate, *etc.;* precipitation with an organic solvent; anion exchange chromatography using a resin such as diethylaminoethyl (DEAE)-Sepharose or DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose, phenyl-Sepharose; gel filtration using a molecular sieve; affinity chromatography; chromatofocusing; electrophoresis such as isoelectric focusing; and the like which may be used alone or in combination.

Also, when the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized using a protein denaturing agent. The antibody composition is made into a normal three-dimensional structure by diluting or dialyzing the solubilized solution, and then a purified product of the antibody composition is obtained by the same isolation purification method.

When the antibody composition is secreted extracellularly, the antibody composition or derivatives thereof can be recovered from the culture supernatant. That is, the culture is treated by a technique such as centrifugation to obtain a soluble fraction, and a purified preparation of the antibody composition can be obtained from the soluble fraction by the same isolation purification method.

The thus obtained antibody composition includes an antibody, the fragment of the antibody, a fusion protein comprising the Fc region of the antibody, and the like.

As an example for obtaining the antibody composition, methods for producing a humanized antibody composition and an Fc fusion protein is described below in detail, but other antibody compositions can also be obtained in a manner similar to the method.

A. Preparation of humanized antibody composition

(1) Construction of vector for humanized antibody expression

A vector for humanized antibody expression is an expression vector for animal cell into which genes encoding the H chain and L chain C regions of a human antibody are inserted, which can be constructed by cloning each of genes encoding CH and CL of a human antibody into an expression vector for animal cell.

The C regions of a human antibody may be CH and CL of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding CH and CL of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad. Sci. USA,* 78, 1527 (1981), pSG1 β d2-4 [*Cytotechnology,* 4, 173 (1990)] and the like. The promoter and enhancer in the expression vector for animal cell includes SV40 early promoter and enhancer [*J. Biochem.,* 101, 1307 (1987)], Moloney mouse leukemia virus LTR promoter [*Biochem. Biophys. Res. Commun.,* 149, 960 (1987)], immunoglobulin H chain promoter [*Cell,* 41, 479 (1985)] and enhancer [*Cell,* 33, 717 (1983)] and the like.

The vector for humanized antibody expression may be either of a type in which genes encoding the H chain and L chain of an antibody exist on separate vectors or of a type in which both genes exist on the same vector (hereinafter referred to as "tandem type"). In respect of easiness of construction of a vector for humanized antibody expression, easiness of introduction into animal cells, and balance between the expression amounts of the H and L chains of an antibody in animal cells, a tandem type of the vector for humanized antibody expression is more preferred [*J. Immunol. Methods,* 167, 271 (1994)].

The constructed vector for humanized antibody expression can be used for expression of a human chimeric antibody and a human CDR-grafted antibody in animal cells.

(2) Preparation method of cDNA encoding V region of non-human animal antibody

cDNAs encoding VH and VL of a non-human animal antibody, such as a mouse antibody, can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding VH and a recombinant phage or recombinant plasmid comprising a cDNA encoding VL is isolated from the library by using a C region part or a V region part of an existing mouse antibody as the probe. Full nucleotide sequences of VH and VL of the mouse antibody of interest on the recombinant phage or recombinant plasmid are determined, and full length amino acid sequences of VH and VL are deduced from the nucleotide sequences.

As the non-human animal, any animal such as mouse, rat, hamster or rabbit can be used so long as a hybridoma cell can be produced therefrom.

The method for preparing total RNA from a hybridoma cell includes the guanidine thiocyanate-cesium trifluoroacetate method [*Methods in Enzymology,* 154, 3 (1987)] and the like, and the method for preparing mRNA from total RNA includes an oligo(dT)-immobilized cellulose column method [*Molecular Cloning, A Laboratory Manual,* Second Edition, Cold Spring Harbor Laboratory Press (1989)] and the like. In addition, examples of a kit for preparing mRNA from a hybridoma cell include Fast Track mRNA Isolation Kit (manufactured by Invitrogen), Quick Prep mRNA Purification Kit (manufactured by Pharmacia) and the like.

The method for synthesizing cDNA and preparing a cDNA library includes the usual methods [*Molecular Cloning, A Laboratory Manual,* Second Edition, Cold Spring Harbor Laboratory Press (1989), *Current Protocols in Molecular Biology,* Supplement 1-34], methods using a commercially available kit such as SuperScript™, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized by using mRNA extracted from a hybridoma cell as the template is inserted may be any vector so long as the cDNA can be inserted. Examples include ZAP Express [*Strategies,* 5, 58 (1992)], pBluescript II SK(+) [*Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics*, 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the H chain and L chain V regions of a non-human animal antibody from the cDNA library, a colony hybridization or a plaque hybridization using an isotope- or fluorescence-labeled probe can be used [*Molecular Cloning, A Laboratory Manual,* Second Edition, Cold Spring Harbor Laboratory Press (1989)]. The cDNA encoding VH and VL can also be prepared by preparing primers and carrying out polymerase chain reaction [hereinafter referred to as "PCR"; *Molecular Cloning, A Laboratory Manual,* Second Edition, Cold Spring

Harbor Laboratory Press (1989); *Current Protocols in Molecular Biology,* Supplement 1-34] using a cDNA synthesized from mRNA or a cDNA library as the template.

The nucleotide sequences of the cDNAs can be determined by digesting the selected cDNAs with appropriate restriction enzymes, cloning the DNA fragments into a plasmid such as pBluescript SK(-) (manufactured by Stratagene), carrying out the reaction of a generally used nucleotide sequence analyzing method such as the dideoxy method of *Sanger et al.* [*Proc. Natl. Acad. Sci. USA,* 74 5463 (1977)] or the like and then analyzing the clones using an automatic nucleotide sequence analyzer such as A.L.F. DNA Sequencer (manufactured by Pharmacia) or the like.

Whether or not the obtained cDNAs encode the full length amino acid sequences of VH and VL of the antibody containing a secretory signal sequence can be confirmed by deducing the full length amino acid sequences of VH and VL from the determined nucleotide sequence and comparing them with the full length amino acid sequences of VH and VL of known antibodies [*Sequences of Proteins of Immunological Interest,* US Dep. Health and Human Services (1991)].

(3) Analysis of amino acid sequence of V region of non-human animal antibody

Regarding the full length amino acid sequences of VH and VL of the antibody containing a secretory signal sequence, the length of the secretory signal sequence and the *N*-terminal amino acid sequences can be deduced and subgroups to which they belong can also be found, by comparing them with the full length amino acid sequences of VH and VL of known antibodies [*Sequences of Proteins of Immunological Interest,* US Dep. Health and Human Services, (1991)]. In addition, the amino acid sequences of each CDR pf VH and VL can also be found by comparing them with the amino acid sequences of VH and VL of known antibodies [*Sequences of Proteins of Immunological Interest,* US Dep. Health and Human Services, (1991)].

(4) Construction of vector for human chimeric antibody expression

A vector for human chimeric antibody expression can be constructed by cloning cDNAs encoding VH and VL of a non-human animal antibody into upstream of genes encoding CH and CL of a human antibody in the vector for expression of humanized antibody described in the item 3(1). For example, a vector for human chimeric antibody expression can be constructed by linking each of cDNAs encoding VH and VL of a non-human animal antibody to a synthetic DNA comprising nucleotide sequences at the 3'-terminals of VH and VL of a non-human animal antibody and nucleotide sequences at the 5'-terminals of CH and CL of a human antibody and also having a recognition sequence of an appropriate restriction enzyme at both terminals, and by cloning them into upstream of genes encoding CH and CL of a human antibody contained in the vector for expression of humanized antibody described in the item 3(1).

(5) Construction of cDNA encoding V region of human CDR-grafted antibody

cDNAs encoding VH and VL of a human CDR-grafted antibody can be obtained as follows. First, amino acid sequences of the frameworks (hereinafter referred to as "FR") of VH and VL of a human antibody for grafting CDR of VH and VL of a non-human animal antibody is selected. As the amino acid sequences of FRs of VH and VL of a human antibody, any amino acid sequences can be used so long as they are derived from a human antibody. Examples include amino acid sequences of FRs of VH and VL of human antibodies registered at databases such as Protein Data Bank, *etc.,* amino acid sequences common in each subgroup of FRs of the VH and VL of human antibodies [*Sequences of Proteins of Immunological Interest,* US Dep. Health and Human Services (1991)] and the like. In order to produce a human CDR-grafted antibody having enough activities, it is preferred to select an amino acid sequence having a homology as high as possible (at least 60% or more) with amino acid sequences of the H chain and L chain V regions of a non-human animal antibody of interest.

Next, the amino acid sequences of CDRs of VH and VL of the non-human animal antibody of interest are grafted to the selected amino acid sequences of FRs of VH and VL of a human antibody to design amino acid sequences of VH and VL of the human CDR-grafted antibody. The designed amino acid sequences are converted into DNA sequences by considering the frequency of codon usage found in nucleotide sequences of antibody genes [*Sequences of Proteins of Immunological Interest,* US Dep. Health and Human Services (1991)], and the DNA sequences encoding the amino acid sequences of VH and VL of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the vector for expression of humanized antibody described in the item 3 (1) by introducing recognition sequences of an appropriate restriction enzyme into the 5'-terminals of the synthetic DNA on both terminals. After the PCR, the amplified product is cloned into a plasmid such as pBluescript SK(-) (manufactured by Stratagene) and the nucleotide sequences are determined by the method in the item 3(2) to thereby obtain a plasmid having DNA sequences encoding the amino acid sequences of VH and VL of the desired human CDR-grafted antibody.

(6) Modification of amino acid sequence of V region of human CDR-grafted antibody

It is known that when a human CDR-grafted antibody is produced by simply grafting only CDRs in VH and VL of a non-human animal antibody into FRs in VH and VL of a human antibody, its antigen-binding activity is lower than that of the original non-human animal antibody [*BIO/TECHNOLOGY,* 9, 266 (1991)]. As the reason, it is considered that several amino acid residues of Frs other than CDRs directly or indirectly relate to antigen-binding activity in VH and VL of the original non-human animal antibody, and that they are changed to different amino acid residues of FRs in VH and VL of a human antibody. In order to solve the problem, in human CDR-grafted antibodies, among the amino acid sequences of FRs in VH and VL of a human antibody, an amino acid residue which directly relates to binding to an antigen, or an amino acid residue which indirectly relates to binding to an antigen by interacting with an amino acid residue in CDR or by maintaining the three-dimensional structure of an antibody is identified and modified to an amino acid residue which is found in the original non-human animal antibody to thereby increase the antigen binding activity which has been decreased [*BIO/TECHNOLOGY,* 9, 266 (1991)].

In the production of a human CDR-grafted antibody, how to efficiently identify the amino acid residues relating to the antigen binding activity in FR is most important, so that the three-dimensional structure of an antibody is constructed and analyzed by X-ray crystallography [*J. Mol. Biol.,* 112, 535 (1977)], computer-modeling [*Protein Engineering,* 7, 1501 (1994)] or the like. Although the information of the three-dimensional structure of antibodies has been useful in the production of a human CDR-grafted antibody, method for producing a human CDR-grafted antibody which can be applied to all antibodies has not been established yet. Therefore, various attempts must be currently be necessary, for example, several modified antibodies of each antibody are produced and the relationship between each of the modified antibodies and its antibody binding activity is examined.

The modification of the selected amino acid sequence of FRs in VH and VL of a human antibody can be accomplished using various synthetic DNA for modification according to PCR as described in the item 3(5). With regard to the amplified product obtained by the PCR, the nucleotide sequence is determined according to the method as described in the item 3(2) to thereby confirm whether the objective modification has been carried out.

(7) Construction of human CDR-grafted antibody expression vector

A human CDR-grafted antibody expression vector can be constructed by cloning the cDNAs encoding VH and VL of the human CDR-grafted antibody constructed in the items 3(5) and (6) into upstream of the gene encoding CH and CL of a human antibody in the vector for expression of humanized antibody described in the item 3(1). For example, recognizing sequences of an appropriate restriction enzyme are introduced into the 5'-terminals of both terminals of a synthetic DNA fragment, among the synthetic DNA fragments which are used in the items 3(5) and (6) for constructing the VH and VL of the human CDR-grafted antibody, so that they are cloned into upstream of the genes encoding CH and CL of a human antibody in the vector for expression of humanized antibody described in the item 3 (1) in such a manner that they can be expressed in a suitable form, to thereby construct the human CDR-grafted antibody expression vector.

(8) Stable production of humanized antibody

A transformant capable of stably producing a human chimeric antibody and a human CDR-grafted antibody (both hereinafter referred to as "humanized antibody") can be obtained by introducing the vectors for humanized antibody expression described in the items 3(4) and (7) into an appropriate animal cell.

The method for introducing a vector for humanized antibody expression into an animal cell includes electroporation [Japanese Published Unexamined Patent Application No. 257891/90, *Cytotechnology,* 3, 133 (1990)] and the like.

As the animal cell into which a vector for humanized antibody expression is introduced, any cell can be used so long as it is an animal cell which can produce the humanized antibody.

Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a syrian hamster kidney, a human myeloma cell such as Namalwa cell, and the like, and a Chinese hamster ovary cell CHO/DG44 cell, a rat myeloma YB2/0 cell and the cells described in the item 1 are preferred.

After introduction of the vector for humanized antibody expression, a transformant capable of stably producing the humanized antibody can be selected by using a medium for animal cell culture comprising an agent such as G418

sulfate (hereinafter referred to as "G418"; manufactured by SIGMA) and the like in accordance with the method described in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The humanized antibody can be produced and accumulated in the culture supernatant by culturing the obtained transformant in a medium. The amount of production and antigen binding activity of the humanized antibody in the culture supernatant can be measured by a method such as enzyme-linked immunosorbent assay [hereinafter referred to as "ELISA"; *Antibodies: A Laboratory Manual,* Cold Spring Harbor Laboratory, Chapter 14 (1998), *Monoclonal Antibodies: Principles and Practice,* Academic Press Limited (1996)] or the like. Also, the amount of the humanized antibody produced by the transformant can be increased by using a DHFR gene amplification system in accordance with the method described in Japanese Published Unexamined Patent Application No. 257891/90.

The humanized antibody can be purified from a culture supernatant of the transformant using a protein A column [*Antibodies, A Laboratory Manual,* Cold Spring Harbor Laboratory, Chapter 8 (1988), *Monoclonal Antibodies: Principles and Practice,* Academic Press Limited (1996)]. In addition, purification methods generally used for the purification of proteins can also be used. For example, the purification can be carried out through the combination of a gel filtration, an ion exchange chromatography and an ultrafiltration. The molecular weight of the H chain, L chain or antibody molecule as a whole of the purified humanized antibody can be measured, e.g., by polyacrylamide gel electrophoresis [hereinafter referred to as "SDS-PAGE"; *Nature,* 227, 680 (1970)], Western blotting [*Antibodies, A Laboratory Manual,* Chapter 12, (1988), *Monoclonal Antibodies*] or the like.

B. Preparation of Fc fusion protein

(1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.

The Fc region of a human antibody includes those containing a part of a hinge region and/or CH1 in addition to regions containing CH2 and CH3 regions. Also, it can be any Fc region so long as at least one amino acid of CH2 or CH3 may be deleted, substituted, added or inserted, and substantially has the binding activity to the Fcγ receptor.

As the genes encoding the Fc region of a human antibody and the protein to be fused, a chromosomal DNA comprising an exon and an intron can be used, and a cDNA can also be used. The method for linking the genes and the Fc region includes PCR using each of the gene sequences as the template (*Molecular Cloning,* Second Edition; *Current Protocols in Molecular Biology,* Supplement 1-34). As the expression vector for animal cell, any vector can be used, so long as a gene encoding the C region of a human antibody can be inserted thereinto and expressed therein. Examples include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad. Sci. USA,* 78, 1527 (1981), pSG1 β d2-4 [*Cytotechnology,* 4, 173 (1990)] and the like. The promoter and enhancer in the expression vector for animal cell include SV40 early promoter and enhancer [*J. Biochem.,* 101, 1307 (1987)], Moloney mouse leukemia virus LTR promoter [*Biochem. Biophys. Res. Commun.,* 149, 960 (1987)], immunoglobulin H chain promoter [*Cell,* 41, 479 (1985)] and enhancer [*Cell,* 33, 717 (1983)], and the like.

(2) Preparation of DNA encoding Fc region of human antibody and protein to be fused

A DNA encoding the Fc region of a human antibody and the protein to be fused can be obtained in the following manner.

A cDNA is synthesized from mRNA extracted from a cell or tissue which expresses the protein of interest to be fused with Fc. The synthesized cDNA is cloned into a vector such as a phage or a plasmid to obtain a cDNA library. A recombinant phage or recombinant plasmid comprising cDNA encoding the protein of interest is isolated from the library by using the gene sequence part of the protein of interest as the probe. A full nucleotide sequence of the antibody of interest on the recombinant phage or recombinant plasmid is determined, and a full length amino acid sequence is deduced from the nucleotide sequence.

As the non-human animal, any animal such as mouse, rat, hamster or rabbit can be used so long as a cell or tissue can be removed therefrom.

The method for preparing a total RNA from a cell or tissue includes the guanidine thiocyanate-cesium trifluoroacetate method [*Methods in Enzymology,* 154, 3 (1987)] and the like, and the method for preparing mRNA from total

RNA includes an oligo (dT)-immobilized cellulose column method (*Molecular Cloning,* Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods (*Molecular Cloning,* Second Edition; *Current Protocols in Molecular Biology,* Supplement 1-34); methods using a commercially available kit such as SuperScript™, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express [*Strategies,* 5, 58 (1992)], pBluescript II SK(+) [*Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the protein of interest from the cDNA library, a colony hybridization or a plaque hybridization using an isotope- or fluorescence-labeled probe can be used (*Molecular Cloning,* Second Edition). The cDNA encoding the protein of interest can also be prepared by preparing primers and using a cDNA synthesized from mRNA or a cDNA library as the template according to PCR.

The method for fusing the protein of interest with the Fc region of a human antibody includes PCR. For example, synthesized oligo DNAs (primers) are designed at the 5'-terminal and 3'-terminal of the gene sequence encoding the protein of interest, and PCR is carried out to prepare a PCR product. In the same manner, primers are designed for the gene sequence encoding the Fc region of a human antibody to be fused to prepare a PCR product. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequence of the DNA can be determined by digesting the gene sequence linked by the above method with appropriate restriction enzymes, cloning the DNA fragments into a plasmid such as pBluescript SK(-) (manufactured by Stratagene), carrying out analysis by using a generally used nucleotide sequence analyzing method such as the dideoxy method of Sanger *et al.* [*Proc. Natl. Acad Sci. USA,* 74, 5463 (1977)] or an automatic nucleotide sequence analyzer such as A.L.F. DNA Sequencer (manufactured by Pharmacia).

Whether or not the obtained cDNA encodes the full length amino acid sequences of the Fc fusion protein containing a secretory signal sequence can be confirmed by deducing the full length amino acid sequence of the Fc fusion protein from the determined nucleotide sequence and comparing it with the amino acid sequence of interest.

(3) Stable production of Fc fusion protein

A transformant capable of stably producing an Fc fusion protein can be obtained by introducing the Fc fusion protein expression vector described in the item (1) into an appropriate animal cell.

The method for introducing the Fc fusion protein expression vector into an animal cell include electroporation [Japanese Published Unexamined Patent Application No. 257891/90, *Cytotechnology,* 3, 133 (1990)] and the like.

As the animal cell into which the Fc fusion protein expression vector is introduced, any cell can be used, so long as it is an animal cell which can produce the Fc fusion protein.

Examples include mouse myeloma cells such as NS0 cell and SP2/0 cell, Chinese hamster ovary cells such as CHO/dhfr⁻ cell and CHO/DG44 cell, rat myeloma such as YB2/0 cell and IR983F cell, BHK cell derived from a syrian hamster kidney, a human myeloma cell such as Namalwa cell, and the like, and preferred are a Chinese hamster ovary cell CHO/DG44 cell, a rat myeloma YB2/0 cell and the host cells used in the method of the present invention described in the item 1.

After introduction of the Fc fusion protein expression vector, a transformant capable of stably producing the Fc fusion protein expression vector can be selected by using a medium for animal cell culture comprising an agent such as G418 and the like in accordance with the method described in Japanese Published Unexamined Patent Application No. 257891/90. The medium to culture animal cells includes RPMI 1640 medium (manufactured by Nissui

Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum to these media, and the like. The Fc fusion protein can be produced and accumulated in the culture supernatant by culturing the obtained transformant in a medium. The amount of production and antigen binding activity of the Fc fusion protein in the culture supernatant can be measured by a method such as ELISA. Also, the amount of the Fc fusion protein produced by the transformant can be increased by using a *dhfr* gene amplification system in accordance with the method described in Japanese Published Unexamined Patent Application No. 257891/90.

The Fc fusion protein can be purified from a culture supernatant culturing the transformant by using a protein A column or a protein G column (*Antibodies,* Chapter 8; *Monoclonal Antibodies*). In addition, purification methods generally used for the purification of proteins can also be used. For example, the purification can be carried out through the combination of a gel filtration, an ion exchange chromatography and an ultrafiltration. The molecular weight as a whole of the purified Fc fusion protein molecule can be measured by SDS-PAGE [*Nature,* 227, 680 (1970)], Western blotting (*Antibodies,* Chapter 12, *Monoclonal Antibodies*) or the like.

Thus, methods for producing an antibody composition using an animal cell as the host cell have been described, but, as described above, the antibody composition can also be produced by a yeast, an insect cell, a plant cell, an animal individual or a plant individual by the same methods on the animal cell.

When a cell has the ability to express an antibody molecule innately, an antibody composition of interest can be produced by preparing an antibody-producing cell using the method described in the item 1, culturing the cell and then purifying the antibody composition from the resulting culture.

4. Activity evaluation of antibody composition

As the method for measuring the amount of the purified antibody composition, the activity of the purified antibody to bind to an antigen and the effector function of the purified antibody composition, the known method described in *Monoclonal Antibodies, Antibody Engineering* and the like can be used.

For example, when the antibody composition is a humanized antibody, the binding activity to an antigen and the binding activity to an antigen-positive cultured clone can be measured by methods such as ELISA and an immunofluorescent method [*Cancer Immunol. Immunother.,* 36, 373 (1993)]. The cytotoxic activity against an antigen-positive cultured clone can be evaluated by measuring complement-dependent cytotoxic activity (hereinafter referred to as "CDC activity"), ADCC activity [*Cancer Immunol. Immunother.,* 36, 373 (1993)] and the like.

Also, safety and therapeutic effect of the antibody composition in human can be evaluated by using an appropriate model of animal species relatively close to human, such as *Macaca fascicularis.*

5. Analysis of sugar chains of antibody composition

The sugar chain structure binding to an antibody molecule expressed in various cells can be analyzed in accordance with the general analysis of the sugar chain structure of a glycoprotein. For example, the sugar chain which is bound to IgG molecule comprises a neutral sugar such as galactose, mannose or fucose, an amino sugar such as *N*-acetylglucosamine and an acidic sugar such as sialic acid, and can be analyzed by a method, such as a sugar chain structure analysis, using sugar composition analysis, two dimensional sugar chain mapping or the like.

(1) Analysis of neutral sugar and amino sugar compositions

The sugar chain composition binding to an antibody molecule can be analyzed by carrying out acid hydrolysis of sugar chains with trifluoroacetic acid or the like to release a neutral sugar or an amino sugar and measuring the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr.,* 6, 1577 (1983)].

The composition ratio can also be analyzed by a fluorescence labeling method using 2-aminopyridine. Specifically, the composition ratio can be calculated in accordance with a known method [*Agric. Biol. Chem.,* 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure binding to an antibody molecule can be analyzed by the two dimensional sugar

chain mapping method [*Anal. Biochem.*, 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with those of known sugar chains.

Specifically, sugar chains are released from an antibody by subjecting the antibody to hydrazinolysis, and the released sugar chain is subjected to fluorescence labeling with 2-aminopyridine (hereinafter referred to as "PA") [*J. Biochem.*, 95, 197 (1984)], and then the sugar chains are separated from an excess PA-treating reagent by gel filtration, and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo) or a literature [*Anal. Biochem.*, 171, 73 (1988)].

The structure deduced by the two dimensional sugar chain mapping method can be confirmed by further carrying out mass spectrometry such as MALDI-TOF-MS of each sugar chain.

6. Application of antibody composition obtained in the present invention

The antibody composition obtained in the present invention has high ADCC activity. An antibody having high ADCC activity is useful for preventing and treating various diseases including cancers, inflammatory diseases, immune diseases such as autoimmune diseases, allergies and the like, cardiovascular diseases and various diseases which are caused by such as virus and infections.

In the case of cancers, namely malignant tumors, cancer cells grow. General anti-tumor agents inhibit the growth of cancer cells. In contrast, an antibody having high ADCC activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is not sufficient, so that combination therapy with chemotherapy has been carried out [*Science*, 280, 1197 (1998)]. If higher anti-tumor effect is found by the antibody composition of the present invention alone, the dependency on chemotherapy will be decreased and side effects will be reduced.

In immune diseases such as inflammatory diseases, autoimmune diseases and allergies, *in vivo* reactions of the diseases are induced by the release of a mediator molecule by immunocytes, so that the allergy reaction can be suppressed by eliminating immunocytes using an antibody having high ADCC activity.

The cardiovascular diseases include arteriosclerosis and the like. The arteriosclerosis is treated using balloon catheter at present, but cardiovascular diseases can be prevented and treated by inhibiting growth of arterial cells in restricture after balloon catheter treatment using an antibody having high ADCC activity.

Various diseases including viral and bacterial infections can be prevented and treated by inhibiting proliferation of cells infected with a virus or bacterium using an antibody having high ADCC activity.

An antibody which recognizes a tumor-related antigen, an antibody which recognizes an allergy- or inflammation-related antigen, an antibody which recognizes cardiovascular disease-related antigen and an antibody which recognizes a viral or bacterial infection-related antigen are exemplified below.

The antibody which recognizes a tumor-related antigen includes anti-GD2 antibody [*Anticancer Res.*, 13, 331-336 (1993)], anti-GD3 antibody [*Cancer Immunol. Immunother.*, 36, 260-266 (1993)], anti-GM2 antibody [*Cancer Res.*, 54, 1511-1516 (1994)], anti-HER2 antibody [*Proc. Natl. Acad. Sci. USA*, 89, 4285-4289 (1992)], anti-CD52 antibody [*Proc. Natl. Acad. Sci. USA*, 89, 4285-4289 (1992)], anti-MAGE antibody [*British J. Cancer*, 83, 493-497 (2000)], anti-HM1.24 antibody [*Molecular Immunol.*, 36, 387-395 (1999)], anti-parathyroid hormone-related protein (PTHrP) antibody [*Cancer*, 88, 2909-2911 (2000)], anti- FGF8 antibody (*Proc. Natl Acad Sci. USA*, 86, 9911-9915 (1989), anti-basic fibroblast growth factor antibody and anti-FGF8 receptor antibody (*J. Biol. Chem*, 265, 16455-16463 (1990)], anti-basic fibroblast growth factor receptor antibody and anti-insulin-like growth factor antibody [*J. Neurosci. Res.*, 40, 647-659 (1995)], anti-insulin-like growth factor receptor antibody [*J. Neurosci. Res.*, 40, 647-659 (1995)], anti-PMSA antibody [*J. Urology*, 160, 2396-2401 (1998)], anti-vascular endothelial cell growth factor antibody [*Cancer Res.*, 57, 4593-4599 (1997)], anti-vascular endothelial cell growth factor receptor antibody [*Oncogene*, 19, 2138-2146 (2000)], anti- CA125 antibody, anti-17-1A antibody, anti-integrin $\alpha v\beta 3$ antibody, anti-CD33 antibody, anti-CD22 antibody, anti-HLA antibody, anti-HLA-DR antibody, anti-CD20 antibody, anti-CD 19 antibody, anti-EGF receptor antibody (*Immunology Today*, 21(8), 403-410 (2000)], anti-CD10 antibody [*American Journal of Clinical Pathology*, 113, 374-382 (2000)] and the like.

The antibody which recognizes an allergy- or inflammation-related antigen includes anti-interleukin 6 antibody [*Immunol. Rev.*, 127, 5-24 (1992)], anti-interleukin 6 receptor antibody [*Molecular Immunol.*, 31, 371-381 (1994)], anti-interleukin 5 antibody [*Immunol. Rev.*, 127, 5-24 (1992)], anti-interleukin 5 receptor antibody and anti-interleukin 4

antibody [*Cytokine,* 3, 562-567 (1991)], anti-interleukin 4 receptor antibody [*J. Immunol. Meth.,* 217, 41-50 (1998)], anti-tumor necrosis factor antibody [*Hybridoma,* 13, 183-190 (1994)], anti-tumor necrosis factor receptor antibody [*Molecular Pharmacol.,* 58, 237-245 (2000)], anti-CCR4 antibody [*Nature,* 400, 776-780 (1999)], anti-chemokine antibody [*J. Immuno. Meth.,* 174, 249-257 (1994)], anti-chemokine receptor antibody [*J. Exp. Med.,* 186, 1373-1381 (1997)], anti-IgE antibody, anti-CD23 antibody, anti-CD11a antibody [*Immunology Today,* 21 (8)., 403-410 (2000)], anti-CRTH2 antibody (*J Immunol.,* 162, 1278-1286 (1999)], anti-CCR8 antibody (WO99/25734), anti-CCR3 antibody (US6207155), and the like.

The antibody which recognizes a cardiovascular disease-related antigen includes anti-GpIIb/IIIa antibody [*J. Immunol.,* 152, 2968-2976 (1994)], anti-platelet-derived growth factor antibody [*Science,* 253, 1129-1132 (1991)], anti-platelet-derived growth factor receptor antibody [*J. Biol. Chem.,* 272, 17400-17404 (1997)] and anti-blood coagulation factor antibody [*Circulation,* 101, 1158-1164 (2000)] and the like.

The antibody which recognizes an antigen relating to autoimmune diseases includes an anti-auto-DNA antibody [*Immunol. Letters,* 72, 61-68 (2000)] and the like.

The antibody which recognizes a viral or bacterial infection-related antigen includes anti-gp120 antibody [*Structure,* 8, 385-395 (2000)], anti-CD4 antibody [*J. Rheumatology,* 25, 2065-2076 (1998)], anti-CCR4 antibody, anti-Vero toxin antibody [*J. Clin. Microbiol.,* 37, 396-399 (1999)], antibody against autoimmune diseases (psoriasis, rheumarthritis, Crohn's diseases, colitis ulcerosa, systemic erythematodes, disseminated sclerosis, *etc.*), anti-CD11a antibody, anti-ICAM3 antibody, anti-CD80 antibody, anti-CD2 antibody, anti-CD3 antibody, anti-CD4 antibody, anti-integrin $\alpha 4\beta 7$ antibody, anti-GD40L antibody, anti-IL-2 antibody [*Immunology Today,* 21(8), 403-410 (2000)], and the like.

These antibodies can be obtained from public organizations such as ATCC (The American Type Culture Collection), RIKEN Gene Bank at The Institute of Physical and Chemical Research, and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

The medicament comprising the antibody composition obtained in the present invention can be administered as a therapeutic agent alone, but generally, it is preferred to provide it as a pharmaceutical formulation produced by an appropriate method well known in the technical field of manufacturing pharmacy, by mixing it with at least one pharmaceutically acceptable carrier.

It is desirable to select a route of administration which is most effective in treatment. Examples include oral administration and parenteral administration, such as buccal, tracheal, rectal, subcutaneous, intramuscular or intravenous. In the case of an antibody preparation, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like.

Liquid preparations, such as emulsions and syrups, can be produced using, as additives, water; sugars such as sucrose, sorbitol and fructose; glycols, such as polyethylene glycol and propylene glycol; oils, such as sesame oil, olive oil and soybean oil; antiseptics, such as p-hydroxybenzoic acid esters; flavors, such as strawberry flavor and peppermint; and the like.

Capsules, tablets, powders, granules and the like can be produced using, as additive, excipients, such as lactose, glucose, sucrose and mannitol; disintegrating agents, such as starch and sodium alginate; lubricants, such as magnesium stearate and talc; binders, such as polyvinyl alcohol, hydroxypropylcellulose and gelatin; surfactants, such as fatty acid ester; plasticizers, such as glycerine; and the like.

The pharmaceutical preparation suitable for parenteral administration includes injections, suppositories, sprays and the like.

Injections can be prepared using a carrier, such as a salt solution, a glucose solution, a mixture of both thereof or the like. Also, powdered injections can be prepared by freeze-drying the antibody composition in the usual way and adding sodium chloride thereto.

Suppositories can be prepared using a carrier such as cacao butter, hydrogenated fat, carboxylic acid or the like.

Sprays can be prepared using the antibody composition as such or using the antibody composition together with a carrier which does not stimulate the buccal or airway mucous membrane of the patient and can facilitate absorption of the antibody composition by dispersing it as fine particles.

The carrier includes lactose, glycerol and the like. Depending on the properties of the antibody composition and the carrier, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day

and per adult.

Also, as the method for examining antitumor effect of the antibody composition against various tumor cells, *in vitro* tests include CDC activity measuring method, ADCC activity measuring method and the like, and *in vivo* tests include antitumor experiments using a tumor system in an experimental animal such as a mouse, and the like.

CDC activity and ADCC activity measurements and antitumor experiments can be carried out in accordance with the methods described in *Cancer Immunology Immunotherapy,* 36, 373 (1993); *Cancer Research,* 54, 1511 (1994) and the like.

The present invention will be described below in detail based on Examples; however, Examples are only simple illustrations, and the scope of the present invention is not limited thereto.

BRIEF DESCRIPTION OF THE DRAWINGS

Fig. 1 shows ADCC activities of anti-CCR4 chimeric antibodies produced by rat myeloma YB2/0 cell-derived clone KM2760#58-35-16 and clone 1-15 to CCR4/EL-4 cells. The ordinate and the abscissa show the cytotoxic activity and the antibody concentration, respectively. "□" and "Δ" show the activities of an anti-CCR4 chimeric antibody KM2760-1 produced by the clone KM2760#58-35-16 and an anti-CCR4 chimeric antibody KM2760-2 produced by the clone 1-15, respectively.

Fig. 2 shows elution patterns of PA-treated sugar chains prepared from antibodies produced by mfFUT8-6 and pAGE249-introduced clones, obtained by analyzing them by reverse phase HPLC. Fig. 2A and Fig. 2B show elution patterns of PA-treated sugar chains prepared from an antibody produced by mfFUT8-6-introduced clone and PA-treated sugar chains prepared from an antibody produced by pAGE249-introduced clone, respectively. The ordinate and the abscissa show the relative fluorescence intensity and the elution time, respectively.

Fig. 3 shows a photograph of the determined levels of FUT8 and β-actin transcription products in each host clone using RT-PCR. Using cDNAs prepared from the clone KM2760#58-35-16 capable of producing KM2760-1, the clone 1-15 capable of producing KM2760-2 and rat myeloma YB2/0 cell which was a parent cell were used as templates, PCR was carried out by using FUT8-specific primer set (SEQ ID NOs:13 and 14) or β-actin-specific primer set (SEQ ID NOs:11 and 12), and the obtained results by subjecting the reaction solution to agarose gel electrophoresis are given.

Fig. 4 shows construction of a plasmid ploxPPuro.

Fig. 5 shows construction of a plasmid pKOFUT8gE2-1.

Fig. 6 shows construction of a plasmid pKOFUT8gE2-2.

Fig. 7 shows construction of a plasmid pscFUT8gE2-3.

Fig. 8 shows construction of a plasmid pKOFUT8gE2-3.

Fig. 9 shows construction of a plasmid pKOFUT8gE2-4.

Fig. 10 shows construction of a plasmid pKOFUT8gE2-5.

Fig. 11 shows construction of a plasmid pKOFUT8Puro.

Fig. 12 shows a photograph of genome Southern analyses of the clone

1st.ΔFUT8 2-46-1 and the clone 2-46-H10 as α1,6-fucosyltransferase gene-disrupted CHO clones.

Fig. 13 shows genome Southern analyses of the clone 1st.ΔFUT8 2-46 and the clone 1st.ΔFUT8 2-46-H10 as α1,6-fucosyltransferase gene-disrupted CHO clones

Fig. 14 shows ADCC activities of an anti-CCR4 chimeric antibody purified from an FUT8 allele-disrupted clone. The ordinate and the abscissa show the cytotoxic activity and the antibody concentration, respectively. "▲" and "■" show the activities of a purified antibody derived from an anti-CCR4 chimeric antibody-producing CHO cell clone 5-03 and a purified antibody derived from clone 1st.ΔFUT8 2-46-1, respectively.

Fig. 15 shows construction of a plasmid pKOFUT8Neo.

Fig. 16 shows a photograph of genome Southern analysis of clones in which one copy of FUT8 allele of CHO/DG44 cell was disrupted.

Fig. 17 shows a photograph of genome Southern analysis of clones in which both FUT8 alleles of CHO/DG44 cell were disrupted.

Fig. 18 shows a photograph of genome Southern analysis of clones in which a drug-resistant gene was removed from both FUT8 alleles of CHO/DG44 cell.

Fig. 19 shows construction of a plasmid pBS-2B8L.

Fig. 20 shows construction of a plasmid pBS-2B8H and a plasmid pBS-28B8Hm.

Fig. 21 shows construction of a plasmid pKANTEX2B8P.

Fig. 22 shows ADCC activities of an anti-CD20 chimeric antibody purified from a FUT8 gene double knockout CHP/DG44 clone to human B lymphocyte cultured cell line Raji cell. The ordinate and the abscissa show the cytotoxic activity and the antibody concentration, respectively.

Fig. 23 shows construction of a plasmid CHfFUT8-pCR2.1.

Embodiments for Carrying Out the Invention

Example 1

Production of cell stably producing anti-CCR4 chimeric antibody:

Cells which capable of stably producing an anti-CCR4 chimeric antibody were prepared as follows by using a tandem type expression vector pKANTEX2160 for an anti-CCR4 chimeric antibody described in WO 01/64754.

(1) Preparation of antibody-producing cell using rat myeloma YB2/0 cell

After introducing 10 μg of the anti-CCR4 chimeric antibody expression vector pKANTEX2160 into $4\times10^6$ cells of rat myeloma YB2/0 cell (ATCC CRL 1662) by electroporation [*Cytotechnology,* 3, 133 (1990)], the cells were suspended in 40 ml of Hybridoma-SFM-FBS(5) [Hybridoma-SFM medium (manufactured by Invitrogen) supplemented with 5% FBS (manufactured by PAA Laboratories)] and dispensed in 200 μl/well into a 96 well culture plate (manufactured by Sumitomo Bakelite). After culturing at 37°C for 24 hours in a 5% $CO_2$ incubator, G418 was added to give a concentration of 1 mg/ml, followed by culturing for 1 to 2 weeks. Culture supernatant was recovered from wells in which growth of transformants showing G418 resistance was observed by the formation of colonies, and antigen binding activity of the anti-CCR4 chimeric antibody in the supernatant was measured by the ELISA described in the item (2) of Example 2.

Regarding the transformants in wells in which production of the anti-CCR4 chimeric antibody was observed in culture supernatants, in order to increase an amount of the antibody production using a DHFR gene amplification system, each of them was suspended in the Hybridoma-SFM-FBS(5) medium supplemented with 1 mg/ml G418 and 50 nM DHFR inhibitor MTX (manufactured by SIGMA) to give a density of 1 to $2\times10^5$ cells/ml, and the suspension was dispensed at 1 ml into wells of a 24 well plate (manufactured by Greiner). After culturing at 37°C for 1 to 2 weeks in a 5% $CO_2$ incubator, transformants showing 50 nM MTX resistance were induced. Antigen binding activity of the anti-CCR4 chimeric antibody in culture supernatants in wells in which growth of transformants was observed was measured by the ELISA described in the item (2) of Example 2.

Regarding the transformants in wells in which production of the anti-CCR4 chimeric antibody was observed in culture supernatants, the MTX concentration was increased by the same method, and a transformant capable of growing in the Hybridoma-SFM-FBS(5) medium supplemented with 200 nM MTX and capable of highly producing the anti-CCR4 chimeric antibody was finally obtained. The obtained transformant was made into a single cell (cloning) by limiting dilution twice, and the obtained cloned clone was named clone KM2760#58-35-16. In this case, using the method for determining the transcription product of an α1,6-fucosyltransferase (hereinafter referred to as "FUT8") gene described in WO00/61739, a clone producing a relatively small amount of the transcription product was selected and used as a suitable clone.

(2) Preparation of antibody-producing cell using CHO/DG44 cell

After introducing 4 μg of the anti-CCR4 chimeric antibody expression vector pKANTEX2160 into $1.6\times10^6$ cells of CHO/DG44 cell by electroporation [*Cytotechnology,* 3, 133 (1990)], the cells were suspended in 10 ml of IMDM-dFBS(10)-HT(1) [IMDM medium (manufactured by Invitrogen) supplemented with 10% dFBS (manufactured by Invitrogen) and 1 × concentration of HT supplement (manufactured by Invitrogen)] and dispensed in 100 μl/well into a 96 well culture plate (manufactured by Iwaki Glass). After culturing at 37°C for 24 hours in a 5% $CO_2$ incubator, the medium was changed to IMDM-dFBS(10) (IMDM medium supplemented with 10% of dialyzed FBS), followed by culturing for 1 to 2 weeks. Culture supernatant was recovered from wells in which the growth was observed due to formation of a transformant showing HT-independent growth, and an amount of production of the anti-CCR4 chimeric antibody in the supernatant was measured by the ELISA described in the item (2) of Example 2.

Regarding the transformants in wells in which production of the anti-CCR4 chimeric antibody was observed in culture supernatants, in order to increase an amount of the antibody production using a DHFR gene amplification system, each of them was suspended in the IMDM-dFBS(10) medium supplemented with 50 nM MTX to give a density of 1 to $2\times10^5$ cells/ml, and the suspension was dispensed in 0.5 ml into wells of a 24 well plate (manufactured by Iwaki Glass). After culturing at 37°C for 1 to 2 weeks in a 5% $CO_2$ incubator, transformants showing 50 nM MTX resistance were induced. Regarding the transformants in wells in which the growth was observed, the MTX concentration was increased to 200 nM by the same method, and a transformant capable of growing in the IMDM-dFBS(10) medium supplemented with 200 nM MTX and of producing the anti-CCR4 chimeric antibody in a large amount was finally obtained. The obtained transformant was named clone 5-03.

Example 2

Comparison of antibody composition produced by an antibody-producing cell in which expression of FUT8 gene has been decreased with an antibody composition produced by its parent cell:

ADCC activities were compared between an antibody composition produced by a cell in which genome is modified so as to have a decreased activity of the α1,6-fucose modifying enzyme with an antibody composition produced by its parent cell.

(1) Preparation of antibody compositions

As an antibody composition produced by an antibody-producing cell in which genome is modified so as to have a decreased activity of the α1,6-fucose modifying enzyme, an antibody composition KM2760-1 purified from culture supernatant of KM2760#58-35-16 in which the transcription product of FUT8 gene was low described in the item (1) of Example 1 was used.

An antibody composition produced by rat myeloma cell YB2/0 cell (ATCC CRL1662) which was a parent cell was prepared as follows.

After introducing 10 μg of the anti-CCR4 chimeric antibody expression vector pKANTEX2160 (described in WO 01/64754) into $4\times10^6$ cells of rat myeloma YB2/0 cell (ATCC CRL 1662) by electroporation [*Cytotechnology,* 3, 133 (1990)], the cells were suspended in 40 ml of Hybridoma-SFM-FBS(5) [Hybridoma-SFM medium (manufactured by Invitrogen) supplemented with 5% FBS (manufactured by PAA Laboratories)] and dispensed in 200 μl/well into a 96 well culture plate (manufactured by Sumitomo Bakelite). After culturing at 37°C for 24 hours in a 5% $CO_2$ incubator, G418 was added to give a concentration of 1 mg/ml, followed by culturing for 1 to 2 weeks. Culture supernatant was recovered from wells in which growth of transformants having G418 resistance was observed by the formation of colonies, and antigen binding activity of the anti-CCR4 chimeric antibody in the supernatant was measured by the ELISA described in the item (2) of this Example.

Regarding the transformants in wells in which production of the anti-CCR4 chimeric antibody was observed in culture supernatants, in order to increase an amount of the antibody production using a DHFR gene amplification system, each of them was suspended in the Hybridoma-SFM-FBS(5) medium supplemented with 1 mg/ml G418 and 50 nM DHFR inhibitor MTX (manufactured by SIGMA) to give a density of 1 to $2\times10^5$ cells/ml, and the suspension was dispensed at 1 ml into wells of a 24 well plate (manufactured by Greiner). After culturing at 37°C for 1 to 2 weeks in a 5% $CO_2$ incubator, transformants having 50 nM MTX resistance were induced. Antigen binding activity of the anti-CCR4 chimeric antibody in culture supernatants in wells in which growth of transformants was observed was measured by the ELISA described in the item (2) of this Example.

Regarding the transformants in wells in which production of the anti-CCR4 chimeric antibody was observed in culture supernatants, the MTX concentration was increased by the same method, and a transformant capable of growing in the Hybridoma-SFM-FBS(5) medium supplemented with 200 nM MTX and of producing the anti-CCR4 chimeric antibody in a large amount was finally obtained. The obtained clone was named clone 1-15. The clone 1-15 was not made into a single cell (cloning) by limiting dilution.

An anti-CCR4 chimeric antibody produced by the transformant clone 1-15 was purified as follows.

The anti-CCR4 chimeric antibody-expressing transformant the clone 1-15 was suspended in Hybridoma-SFM (manufactured by Invitrogen) medium supplemented with 200 nM MTX and 5% of Daigo's GF21 (manufactured by Wako Pure Chemical Industries) to give a density of $2\times10^5$ cells/ml and subjected to fed-batch shaking culturing by using a spinner bottle (manufactured by Iwaki Glass) in a constant temperature chamber of 37°C. After culturing for 8 to 10 days, the anti-CCR4 chimeric antibody was purified from the culture supernatant recovered by using Prosep-A (manufactured by Millipore) column and gel filtration. The purified anti-CCR4 chimeric antibody was named clone KM2760-2.

(2) Antibody-binding activity of antibody compositions

Antibody-binding activity of the two antibody composition obtained in the item (1) of this Example was measured by ELISA shown below.

Compound 1 (SEQ ID NO:6) was selected as a human CCR4 extracellular region peptide capable of reacting with the anti-CCR4 chimeric antibody. In order to use it in the activity measurement by ELISA, a conjugate with BSA (bovine serum albumin) (manufactured by Nacalai Tesque) was prepared by the following method and used as the antigen. That is, 100 ml of a DMSO solution comprising 25 mg/ml SMCC [4-(N-maleimidomethyl)-cyclohexane-1-carboxylic acid *N*-hydroxysuccinimide ester] (manufactured by Sigma) was added dropwise to 900 ml of a 10 mg BSA-containing PBS solution under stirring by using a vortex, followed by gently stirring for 30 minutes. To a gel filtration column such as NAP-10 column equilibrated with 25 ml of PBS, 1 ml of the reaction solution was applied and then

eluted with 1.5 ml of PBS and the resulting eluate was used as a BSA-SMCC solution (BSA concentration was calculated by the absorbance at 280 nM). Next, 250 ml of PBS was added to 0.5 mg of Compound 1 and then completely dissolved by adding 250 ml of DMF, and the BSA-SMCC solution was added thereto under vortex, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-Compound 1 solution.

The prepared conjugate was dispensed at 0.05 μg/ml and 50 μl/well into a 96 well EIA plate (manufactured by Greiner) and incubated for adhesion at 4°C overnight. After washing each well with PBS, 1% BSA-PBS was added thereto in 100 μl/well and allowed to react at room temperature to block the remaining active groups. After washing each well with PBS containing 0.05% Tween 20 (hereinafter referred to as "Tween-PBS"), a culture supernatant of a transformant was added at 50 μl/well and allowed to react at room temperature for 1 hour. After the reaction, each well was washed with Tween-PBS, and then a peroxidase-labeled goat anti-human IgG(γ) antibody solution (manufactured by American Qualex) diluted 6000 times with 1% BSA-PBS as the secondary antibody was added at 50 μl/well and allowed to react at room temperature for 1 hour. After the reaction and subsequent washing with Tween-PBS, the ABTS substrate solution was added at 50 μl/well for color development, and 20 minutes thereafter, the reaction was stopped by adding a 5% SDS solution at 50 μl/well. Thereafter, the absorbance at 415 nm was measured.

As a result of the above measurement by the ELISA, the binding activity to the CCR4 partial peptide was similar between the purified KM2760-1 and KM2760-2.

(3) ADCC activity of purified KM2760-1 and 2760-2.

ADCC activity of the purified KM2760-1 and KM2760-2 was measured as follows.

The ADCC activity of an anti-CCR4 chimeric antibody against CCR4/EL-4 cell (WO 01/64754) which is a human CCR4-high expression cell.

(a) Preparation of target cell suspension

After $1.5\times10^6$ cells of a human CCR4-expressing cell, CCR4/EL-4 cell, described in WO 01/64754 were prepared, a 5.55 MBq equivalent of a radioactive substance $Na_2{}^{51}CrO_4$ was added thereto, followed by reaction at 37°C for 1.5 hours to thereby label the cells with a radioisotope. After the reaction, the cells were washed three times by suspension in a medium and subsequent centrifugation, resuspended in the medium and then incubated at 4°C for 30 minutes on ice for spontaneous dissociation of the radioactive substance. After centrifugation, the cells were adjusted to give a density of $2\times10^5$ cells/ml by adding 15 ml of the medium and used as a target cell suspension.

(b) Preparation of human effector cell suspension

From a healthy doner, 60 ml of peripheral blood was collected, 0.6 ml of heparin sodium (manufactured by Shimizu Pharmaceutical) was added thereto, followed by gently mixing. The mixture was centrifuged (800 g, 20 minutes) to isolate a mononuclear cell layer by using Lymphoprep (manufactured by AXIS SHIELD) in accordance with the manufacture's instructions. The cells were washed by centrifuging (1,400 rpm, 5 minutes) three times with a medium and then re-suspended in the medium to give a density of $5\times10^6$ cells/ml and used as a human effector cell suspension.

(c) Measurement of ADCC activity

The target cell suspension prepared in the item (1) was dispensed at 50 μl ($1\times10^4$ cells/well) into each well of a 96 well U-bottom plate (manufactured by Falcon). Next, 100 μl of the human effector cell suspension prepared in the item (2) was added thereto ($5\times10^5$ cells/well, ratio of the human effector cells to the target cells was 50 : 1). Furthermore, each of the anti-CCR4 chimeric antibodies was added thereto to give a final concentration of 0.0001 to 10 μg/ml, followed by reaction at 37°C for 4 hours. After the reaction, the plate was centrifuged and the amount of $^{51}Cr$ in the supernatant was measured by using a γ-counter. An amount of the spontaneously dissociated $^{51}Cr$ was calculated by carrying out the same procedure using the medium alone instead of the human effector cell suspension and antibody solution, and measuring the amount of $^{51}Cr$ in the supernatant. An amount of the total dissociated $^{51}Cr$ was calculated by carrying out the same procedure using a 1 mol/L hydrochloric acid solution instead of the antibody solution and human effector cell suspension, and measuring the amount of $^{51}Cr$ in the supernatant. The ADCC activity (%) was calculated based on equation (I).

$$\text{ADCC activity (\%)} = \frac{{}^{51}\text{Cr in sample supernatant - spontaneously released } {}^{51}\text{Cr}}{\text{total released } {}^{51}\text{Cr - spontaneously released } {}^{51}\text{Cr}} \times 100 \qquad \text{(I)}$$

As a result of the measurement according to the above method, the activities were significantly different between KM2760-1 and KM2760-2, and the activity of KM2760-2 was significantly less than that of KM2760-1 (Fig. 1).

(4) Sugar chain analysis of antibody composition

Sugar chains of KM2760-1 and KM2760-2 were analyzed as follows.
Each of KM2760-1 and KM2760-2 was subjected to hydrazinolysis to cleave sugar chains from proteins [*Method of Enzymology,* 83, 263 (1982)]. After removing hydrazine by evaporation under a reduced pressure, *N*-acetylation was carried out by adding an aqueous ammonium acetate solution and acetic anhydride. After freeze-drying, fluorescence labeling by 2-aminopyridine [*J. Biochem.,* 95, 197 (1984)] was carrying out. A fluorescence-labeled sugar chain group (PA-treated sugar chain group) was separated from excess reagents by using Superdex Peptide HR 10/30 column (manufactured by Pharmacia). The sugar chain fractions were dried by using a centrifugation concentrator and used as a purified PA-treated sugar chain group. Next, the purified PA-treated sugar chain group was subjected to reverse phase HPLC analysis by using a CLC-ODS column (manufactured by Shimadzu) (Fig. 2).
Using a sodium phosphate buffer (pH 3.8) as buffer A and a sodium phosphate buffer (pH 3.8) + 0.5% 1-butanol as buffer B, the analysis was carried out by the following gradient.

| Time (minute) | 0 | 80 | 90 | 90.1 | 120 |
|---|---|---|---|---|---|
| Buffer B (%) | 0 | 60 | 60 | 0 | 0 |

Peaks (1) to (8) shown in Fig. 2 have the following structures.

(1)

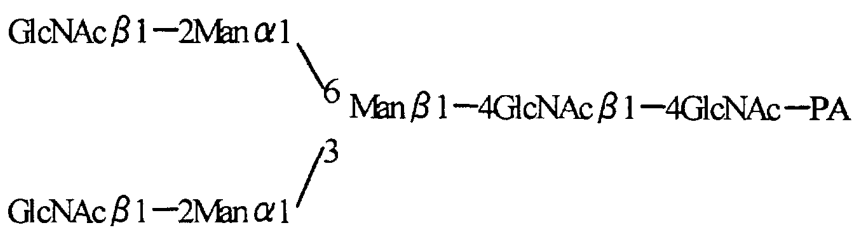

(2)

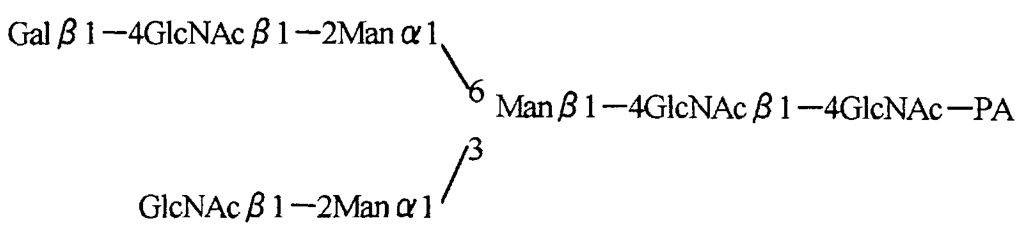

(3)

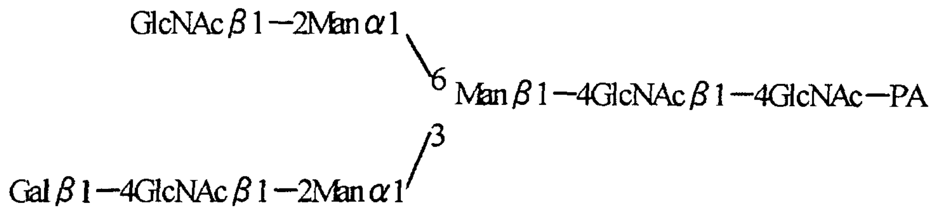

(4)

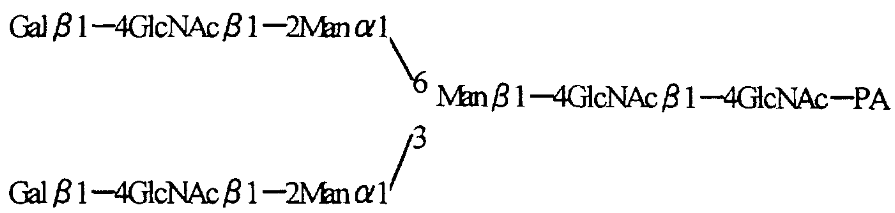

(5)

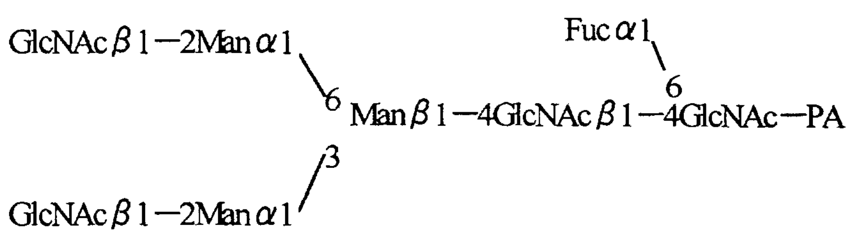

(6)

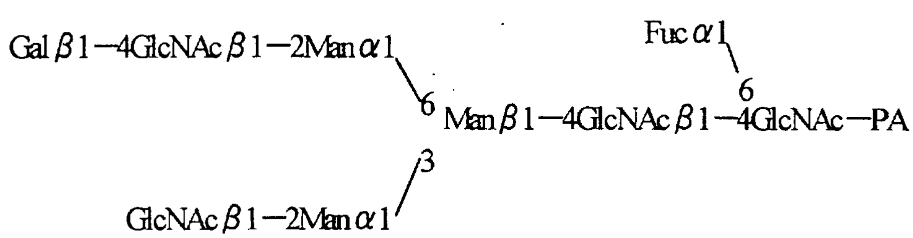

(7) 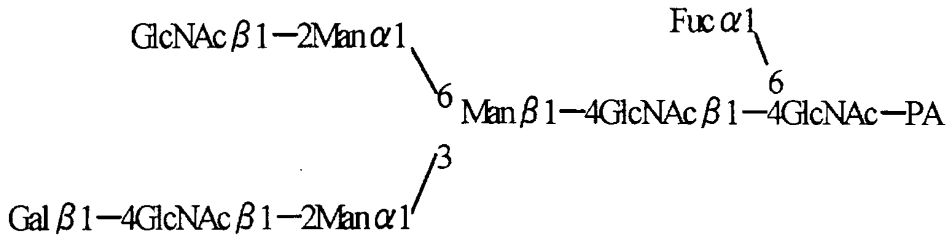

(8) 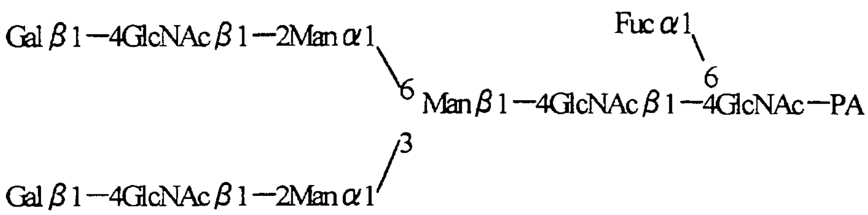

GlcNAc, Gal, Man, Fuc and PA indicate *N*-acetylglucosamine, galactose, mannose, fucose and a pyridylamino group, respectively.

In Fig. 2, the ratio of the α1,6-fucose-free sugar chain group was calculated from the area occupied by the peaks (1) to (4) among (1) to (8), and the ratio of the α1,6-fucose-bound sugar chain group was calculated from the area occupied by the peaks (5) to (8) among (1) to (8).

The ratio of the α1,6-fucose-free sugar chain of KM2760-1 differed from that of KM2760-2.

(5) Expression level of α1,6-fucosyltransferase gene of antibody-producing cells

Fig. 3 shows results of determined levels of transcriptional products of α1,6-fucosyltransferase and β-actin genes of rat myeloma YB2/0 cell which was a parent cell, the clone KM2760#58-35-16 capable of producing KM2760-1 and the clone 1-15 capable of KM2760-2 which were measured according to the method described in Example 8 of WO 00/61739. The amount of production of α1,6-fucosyltransferase gene of the clone 1-15 was similar to that of rat myeloma YB2/0 cell which was a parent cell, but the amount of production of the clone KM2760#58-35-16 was clearly less than the other clones.

Based on the above results, it was confirmed that the antibody-producing cell in which genome is modified so as to have more decreased or deleted activity of the α1,6-fucose modifying enzyme than its parent cell can produce an antibody composition having higher ADCC activity than an antibody composition produced by the parent cell.

Example 3

Preparation of CHO cell in which FUT8 gene is disrupted and production of antibody using the cell:

A CHO cell from which the genome region comprising the CHO cell FUT8 gene exon 2 was deleted was prepared and the ADCC activity of an antibody produced by the cell was evaluated.

1. Construction of Chinese hamster FUT8 gene exon 2 targeting vector plasmid pKOFUT8Puro

(1) Construction of plasmid ploxPPuro

A plasmid ploxPPuro was constructed by the following procedure (Fig. 4).

In 35 μl of NEBuffer 4 (manufactured by New England Biolabs), 1.0 μg of a plasmid pKOSelectPuro (manufactured by Lexicon) was dissolved, and 20 units of a restriction enzyme *Asc*I (manufactured by New England Biolabs) were added thereto, followed by digestion at 37°C for 2 hours. After the digestion, the solution was subjected to 0.8%

(w/v) agarose gel electrophoresis to purify a DNA fragment of about 1.5 Kb containing a puromycin-resistant gene expression unit.

On the other hand, 1.0 μg of a plasmid ploxP described in Japanese Published Unexamined Patent Application No. 314512/99 was dissolved in 35 μl of NEBuffer 4 (manufactured by New England Biolabs), and 20 units of a restriction enzyme *Asc*I (manufactured by New England Biolabs) were added thereto, followed by digestion at 37°C for 2 hours. After the digestion, the solution was subjected to 0.8% (w/v) agarose gel electrophoresis to purify a DNA fragment of about 2.0 Kb.

The obtained *Asc*I-*Asc*I fragment (4.5 μl, about 1.5 Kb) derived from the plasmid pKOSelectPuro, 0.5 μl of the *Asc*I-*Asc*I fragment (about 2.0 Kb) derived from the plasmid ploxP and 5.0 μl of Ligation High (manufactured by Toyobo) were mixed, followed by ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as ploxPPuro.

(2) Construction of plasmid pKOFUT8gE2-1

A plasmid pKOFUT8gE2-1 was constructed by the following procedure by using the plasmid pFUT8fgE2-2 obtained in Reference Example (2) having a genome region comprising exon 2 of Chinese hamster FUT8 (Fig. 5).

In 35 μl of NEBuffer 1 (manufactured by New England Biolabs) containing 100 μg/ml of BSA (manufactured by New England Biolabs), 2.0 μg of the plasmid pFUT8fgE2-2 was dissolved, and 20 units of a restriction enzyme *Sac*I (manufactured by New England Biolabs) were added thereto, followed by digestion at 37°C for 2 hours. A DNA fragment was recovered from the reaction solution by ethanol precipitation and dissolved in 35 μl of NEBuffer 2 (manufactured by New England Biolabs) containing 100 μg/ml of BSA (manufactured by New England Biolabs), and 20 units of a restriction enzyme *Eco*RV (manufactured by New England Biolabs) were added thereto, followed by digestion at 37°C for 2 hours. After the digestion, the solution was subjected to 0.8% (w/v) agarose gel electrophoresis to purify a DNA fragment of about 1.5 Kb.

Separately, 1.0 μg of a plasmid LITMUS28 (manufactured by New England Biolabs) was dissolved in 35 μl of NEBuffer 1 (manufactured by New England Biolabs) containing 100 μg/ml of BSA (manufactured by New England Biolabs), and 20 units of a restriction enzyme *Sac*I (manufactured by New England Biolabs) were added thereto, followed by digestion at 37°C for 2 hours. A DNA fragment was recovered from the reaction solution by ethanol precipitation and dissolved in 35 μl of NEBuffer 2 (manufactured by New England Biolabs) containing 100 μg/ml of BSA (manufactured by New England Biolabs), and 20 units of a restriction enzyme *Eco*RV (manufactured by New England Biolabs) were added thereto, followed by digestion at 37°C for 2 hours. After the digestion, the solution was subjected to 0.8% (w/v) agarose gel electrophoresis to purify a DNA fragment of about 2.8 Kb.

The obtained *Eco*RV-*Sac*I fragment (4.5 μl, about 1.5 Kb) derived from the plasmid pFUT8fgE2-2, 0.5 μl of the *Eco*RV-*Sac*I fragment (about 2.8 Kb) derived from the plasmid LITMUS28 and 5.0 μl of Ligation High (manufactured by Toyobo) were mixed, followed by ligation at 16°C for 30 minutes. *E. coli* DH5α strain was transformed using the reaction solution, and a plasmid DNA was isolated in accordance with a known method from the obtained ampicillin-resistant clones. Herein, the plasmid is referred to as pKOFUT8gE2-1.

(3) Construction ofplasmid pKOFUT8gE2-2

A plasmid pKOFUT8gE2-2 was constructed by the following procedure by using the plasmid pKOFUT8gE2-1 obtained in the item (2) (Fig. 6).

In 30 μl of NEBuffer 2 (manufactured by New England Biolabs) containing 100 μg/ml of BSA (manufactured by New England Biolabs), 2.0 μg of the plasmid pKOFUT8gE2-1 was dissolved, and 20 units of a restriction enzyme *Eco*RV (manufactured by New England Biolabs) were added thereto, followed by digestion at 37°C for 2 hours. A DNA fragment was recovered from the reaction solution by ethanol precipitation and dissolved in 30 μl of NEBuffer 1 (manufactured by New England Biolabs) containing 100 μg/ml of BSA (manufactured by New England Biolabs), and 20 units of a restriction enzyme *Kpn*I (manufactured by New England Biolabs) were added thereto, followed by digestion at 37°C for 2 hours. After the digestion, the solution was subjected to 0.8% (w/v) agarose gel electrophoresis to purify a DNA fragment of about 1.5 Kb.

Separately, 1.0 μg of the plasmid ploxPPuro was dissolved in 30 μl of NEBuffer 4 (manufactured by New England Biolabs), and 20 units of a restriction enzyme *Hpa*I (manufactured by New England Biolabs) were added thereto, followed by digestion at 37°C for 2 hours. A DNA fragment was recovered from the reaction solution by ethanol precipitation and dissolved in 30 μl of NEBuffer 1 (manufactured by New England Biolabs) containing 100 μg/ml of BSA (manufactured by New England Biolabs), and 20 units of a restriction enzyme *Kpn*I (manufactured by New England Biolabs) were added thereto, followed by digestion at 37°C for 2 hours. After the digestion, the solution was subjected to 0.8% (w/v) agarose gel electrophoresis to purify a DNA fragment of about 3.5 Kb.

After 4.0 μl of the obtained *Eco*RV-*Kpn*I fragment (about 1.5 Kb) derived from the plasmid pKOFUT8gE2-1, 1.0 μl of the *Hpa*I-*Kpn*I fragment (about 3.5 Kb) derived from the plasmid ploxPPuro and 5.0 μl of Ligation High (manufactured by Toyobo) were mixed, the mixture was allowed to react for ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as pKOFUT8gE2-2.

(4) Construction of plasmid pscFUT8gE2-3

A plasmid pscFUT8gE2-3 was constructed by the following procedure by using the plasmid pFUT8fgE2-4 obtained in Reference Example (2) having a genome region comprising exon 2 of Chinese hamster FUT8 (Fig. 7).
In 35 μl of NEBuffer 1 (manufactured by New England Biolabs), 2.0 μg of the plasmid pFUT8fgE2-4 was dissolved, and 20 units of a restriction enzyme *Hpa*II (manufactured by New England Biolabs) were added thereto, followed by digestion at 37°C for 2 hours. A DNA fragment was recovered from the reaction solution by ethanol precipitation, and then the DNA termini were changed to blunt ends by using Blunting High (manufactured by Toyobo) in accordance with the manufacture's instructions. The DNA fragment was recovered by carrying out phenol/chloroform extraction and ethanol precipitation and dissolved in 35 μl of NEBuffer 2 (manufactured by New England Biolabs), and 20 units of a restriction enzyme *Hind*III (manufactured by New England Biolabs) were added thereto, followed by digestion at 37°C for 2 hours. After the digestion, the solution was subjected to 0.8% (w/v) agarose gel electrophoresis to purify a DNA fragment of about 3.5 Kb.
Separately, 1.0 μg of a plasmid LITMUS39 (manufactured by New England Biolabs) was dissolved in 35 μl of NEBuffer 2 (manufactured by New England Biolabs), and the solution was mixed with 20 units of a restriction enzyme *Eco*RV (manufactured by New England Biolabs) and 20 units of a restriction enzyme *Hind*III (manufactured by New England Biolabs) and subjected to digestion at 37°C for 2 hours. After the digestion, the solution was subjected to 0.8% (w/v) agarose gel electrophoresis to purify a DNA fragment of about 2.8 Kb.
The obtained *Hpa*II-*Hind*III fragment (4.0 μl, about 3.5 Kb) derived from the plasmid pFUT8fgE2-4, 1.0 μl of the *Eco*RV-*Hind*III fragment (about 2.8 Kb) derived from the plasmid LITMUS39 and 5.0 μl of Ligation High (manufactured by Toyobo) were mixed, followed by ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as pscFUT8gE2-3.

(5) Construction of plasmid pKOFUT8gE2-3

A plasmid pKOFUT8gE2-3 was constructed by the following procedure by using the plasmid pFUT8fgE2-4 obtained in Reference Example (2) having a genome region comprising exon 2 of Chinese hamster FUT8 (Fig. 8).
In 35 μl of NEBuffer for *Eco*RI (manufactured by New England Biolabs), 2.0 μg of the plasmid pFUT8fgE2-4 was dissolved, and 20 units of a restriction enzyme *Eco*RI (manufactured by New England Biolabs) and 20 units of a restriction enzyme HindIII (manufactured by New England Biolabs) were added thereto, followed by digestion at 37°C for 2 hours. After the digestion, the solution was subjected to 0.8% (w/v) agarose gel electrophoresis to purify a DNA fragment of about 1.8 Kb.
Separately, 1.0 μg of a plasmid pBluescript II KS(+) (manufactured by Stratagene) was dissolved in 35 μl of NEBuffer for *Eco*RI (manufactured by New England Biolabs), and 20 units of a restriction enzyme *Eco*RI (manufactured by New England Biolabs) and 20 units of a restriction enzyme *Hind*III (manufactured by New England Biolabs) were added thereto, followed by digestion at 37°C for 2 hours. After the digestion, the solution was subjected to 0.8% (w/v) agarose gel electrophoresis to purify a DNA fragment of about 3.0 Kb.
The obtained *Hind*III-*Eco*RI fragment (4.0 μl, about 1.8 Kb) derived from the plasmid pFUT8fgE2-4, 1.0 μl of the *Hind*III-*Eco*RI fragment (about 3.0 Kb) derived from the plasmid pBluescript II KS(+) and 5.0 μl of Ligation High (manufactured by Toyobo) were mixed, followed by ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as pKOFUT8gE2-3.

(6) Construction of plasmid pKOFUT8gE2-4

A plasmid pKOFUT8gE2-4 was constructed by the following procedure by using the plasmids pscFUT8fgE2-3 and pKOFUT8gE2-3 obtained in the items (4) and (5) (Fig. 9).
In 35 μl of NEBuffer for *Sal*I (manufactured by New England Biolabs) containing 100 μg/ml BSA (manufactured by New England Biolabs), 1.0 μg of the plasmid pscFUT8gE2-3 was dissolved, and 20 units of a restriction enzyme *Sal*I (manufactured by New England Biolabs) were added thereto, followed by digestion at 37°C for 2 hours. A DNA fragment was recovered from the reaction solution by ethanol precipitation and dissolved in 30 μl of NEBuffer 2 (man-

ufactured by New England Biolabs) containing 100 μg/ml of BSA (manufactured by New England Biolabs), and 20 units of a restriction enzyme *Hind*III (manufactured by New England Biolabs) were added thereto, followed by digestion at 37°C for 2 hours. After the digestion, the solution was subjected to 0.8% (w/v) agarose gel electrophoresis to purify a DNA fragment of about 3.6 Kb.

Separately, 1.0 μg of the plasmid pKOFUT8gE2-3 was dissolved in 35 μl of NEBuffer for *Sal*I (manufactured by New England Biolabs), and 20 units of a restriction enzyme *Sal*I (manufactured by New England Biolabs) were added thereto, followed by digestion at 37°C for 2 hours. A DNA fragment was recovered from the reaction solution by ethanol precipitation and dissolved in 35 μl of NEBuffer 2 (manufactured by New England Biolabs), and 20 units of a restriction enzyme *Hind*III (manufactured by New England Biolabs) were added thereto, followed by digestion at 37°C for 2 hours. After the digestion, 35 μl of 1 mol/l Tris-HCl buffer (pH 8.0) and 3.5 μl of *E. coli* C15-derived alkaline phosphatase (manufactured by Takara Shuzo) were added thereto, followed by reaction at 65°C for 30 minutes to dephosphorylate the DNA termini. After the dephosphorylation treatment, a DNA fragment was recovered by carrying out phenol/chloroform extraction and ethanol precipitation and dissolved in 10 μl of sterile water.

The obtained *Sal*I-*Hind*III fragment (4.0 μl, about 3.1 Kb) derived from the plasmid pscFUT8gE2-3, 1.0 μl of the *Sal*I-*Hind*III fragment (about 4.8 Kb) derived from the plasmid pKOFUT8gE2-3 and 5.0 μl of Ligation High (manufactured by Toyobo) were mixed, followed by ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as pKOFUT8gE2-4.

(7) Construction of plasmid pKOFUT8gE2-5

A plasmid pKOFUT8gE2-5 was constructed by the following procedure by using the plasmids pKOFUT8gE2-2 and pKOFUT8gE2-4 obtained in the items (3) and (6) (Fig. 10).

In 30 μl of NEBuffer 4 (manufactured by New England Biolabs), 1.0 μg of the plasmid pKOFUT8gE2-2 was dissolved, and 20 units of a restriction enzyme *Sma*I (manufactured by New England Biolabs) were added thereto, followed by digestion at 25°C for 2 hours. A DNA fragment was recovered from the reaction solution by ethanol precipitation and dissolved in 30 μl of NEBuffer 2 (manufactured by New England Biolabs), and 20 units of a restriction enzyme *Bam*HI (manufactured by New England Biolabs) were added thereto, followed by digestion at 37°C for 2 hours. After the digestion, 30 μl of 1 mol/l Tris-HCl buffer (pH 8.0) and 3.0 μl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment was recovered by carrying out phenol/chloroform extraction and ethanol precipitation and dissolved in 10 μl of sterile water.

Separately, 1.0 μg of the plasmid pKOFUT8gE2-4 was dissolved in 30 μl of NEBuffer 4 (manufactured by New England Biolabs), and 20 units of a restriction enzyme *Sma*I (manufactured by New England Biolabs) were added thereto, followed by digestion at 25°C for 2 hours. A DNA fragment was recovered from the reaction solution by ethanol precipitation and dissolved in 30 μl of NEBuffer 2 (manufactured by New England Biolabs), and 20 units of a restriction enzyme *Bam*HI (manufactured by New England Biolabs) were added thereto, followed by digestion at 37°C for 2 hours. After the digestion, the solution was subjected to 0.8% (w/v) agarose gel electrophoresis to purify a DNA fragment of about 5.2 Kb.

The obtained *Sma*I-*Bam*HI fragment (0.5 μl, about 5.0 Kb) derived from the plasmid pKOFUT8gE2-2, 4.5 μl of the *Sma*I-*Bam*HI fragment (about 5.4 Kb) derived from the plasmid pKOFUT8gE2-4 and 5.0 μl of Ligation High (manufactured by Toyobo) were mixed, followed by ligation at 16°C for 15 hours. *E. coli* DH5α strain was transformed by using the reaction solution, and a plasmid DNA was isolated in accordance with a known method from the obtained ampicillin-resistant clones. Herein, the plasmid is referred to as pKOFUT8gE2-5.

(8) Construction of plasmid pKOFUT8Puro

A plasmid pKOFUT8Puro was constructed by the following procedure by using the plasmid pKOFUT8gE2-5 obtained in the item (7) (Fig. 11).

In 50 μl of NEBuffer 4 (manufactured by New England Biolabs), 1.0 μg of a plasmid pKOSelectDT (manufactured by Lexicon) was dissolved, and 16 units of a restriction enzyme *Rsr*II (manufactured by New England Biolabs) were added thereto, followed by digestion at 37°C for 2 hours. After the digestion, the solution was subjected to 0.8% (w/v) agarose gel electrophoresis to purify a DNA fragment of about 1.2 Kb comprising a diphtheria toxin expression unit.

Separately, 1.0 μg of the plasmid pKOFUT8gE2-5 was dissolved in 50 μl of NEBuffer 4 (manufactured by New England Biolabs), and 16 units of a restriction enzyme *Rsr*II (manufactured by New England Biolabs) were added thereto, followed by digestion at 37°C for 2 hours. After the digestion, 30 μl of 1 mol/l Tris-HCl buffer (pH 8.0) and 3.0 μl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment

was recovered by carrying out phenol/chloroform extraction and ethanol precipitation and dissolved in 10 μl of sterile water.

The obtained *Rsr*II-*Rsr*II fragment (1.0 μl, about 1.2 Kb) derived from the plasmid pKOSelectDT, 1.0 μl of the *Rsr*II-*Rsr*II fragment (about 10.4 Kb) derived from the plasmid pKOFUT8gE2-5, 3.0 μl of sterile water and 5.0 μl of Ligation High (manufactured by Toyobo) were mixed, followed by ligation at 16°C for 30 minutes. *E. coli* DH5α strain was transformed by using the reaction solution, and a plasmid DNA was isolated in accordance with a known method from the obtained ampicillin-resistant clones. Herein, the plasmid is referred to as pKOFUT8Puro.

2. Preparation of CHO cell in which one copy of the genome region containing FUT8 gene exon 2 was disrupted

(1) Introduction of targeting vector

A Chinese hamster FUT8 genome region targeting vector pKOFUT8Puro constructed in the item 1 of this Example was introduced into the clone 5-03 prepared in the item (2) of Example 1.

A gene of the plasmid pKOFUT8Puro was introduced into the clone 5-03 as described below in accordance with electroporation [*Cytotechnology,* 3, 133 (1990)]. First, 150 μg of the plasmid pKOFUT8Puro was dissolved in 1.8 ml of NEBuffer for *Sal*I (manufactured by New England Biolabs), and 600 units of a restriction enzyme *Sal*I (manufactured by New England Biolabs) were added thereto, followed by digestion at 37°C for 5 hours to obtain a linear fragment. The reaction solution was extracted with phenol/chloroform extraction, followed by ethanol precipitation, and the recovered linear plasmid was made into a 1 μg/μl aqueous solution. Separately, the clone 5-03 was suspended in a K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After mixing 200 μl of the cell suspension ($1.6\times10^6$ cells) with 4 μl (4 μg) of the linear plasmid, an entire volume of the cell-DNA mixture was transferred into Gene Pulser Cuvette (inter-electrode distance, 2 mm) (manufactured by BIO-RAD) and then the electroporation was carried out by using a cell fusion apparatus Gene Pulser (manufactured by BIO-RAD) at 350 V pulse voltage and 250 μF electric capacity. After carrying out the electroporation by using 30 cuvettes in the same manner, the cell suspension was suspended in IMDM medium (manufactured by Life Technologies) supplemented with 10% fetal bovine serum (manufactured by Life Technologies) and 1 × concentration HT supplement (manufactured by Life Technologies) and inoculated into 30 adhesion cell culture dishes of 10 cm in diameter (manufactured by Falcon). After culturing at 37°C for 24 hours in 5% $CO_2$, the culture supernatant was removed, and IMDM medium (manufactured by Life Technologies) supplemented with 15 μg/ml puromycin (manufactured by SIGMA) and 10% fetal bovine dialyzed serum (manufactured by Life Technologies) was dispensed at 10 ml. After culturing for 10 days while repeating the medium exchange at intervals of 3 to 4 days, puromycin-resistant clones were obtained.

(2) Preparation of targeting vector-introduced clones

Arbitrary 900 colonies were obtained as follows among the puromycin-resistant clones obtained in the item (1).

First, culture supernatant was removed from the 10 cm dish in which colony of puromycin-resistant clones were formed and 7 ml of a phosphate buffer was added to the dish which was subsequently set under a stereoscopic microscope. Next, each colony was scratched and sucked up using Pipetteman (manufactured by GILSON) and transferred into a 96 well round-bottom plate (manufactured by Falcon). After a trypsin-treatment, each clone was inoculated into a 96 well flat-bottom plate for adhesion cell culture use (manufactured by Iwaki Glass) and cultured for 1 week in IMDM medium (manufactured by Life Technologies) supplemented with 15 μg/ml puromycin (manufactured by SIGMA) and 10% fetal bovine dialyzed serum (manufactured by Life Technologies).

After culturing, each clone in the plate was subjected to trypsin treatment and then mixed with two volumes of a freezing medium (20% DMSO, 40% fetal bovine serum, 40% IMDM). A half of the mixture was inoculated into a 96 well flat-bottom plate for adhesion cell culture (manufactured by Iwaki Glass) as a replica plate, while the remaining half of the mixture was subjected to cryopreservation as master plates. The replica plate was cultured for 1 week in IMDM medium (manufactured by Life Technologies) supplemented with 15 μg/ml puromycin (manufactured by SIGMA) and 10% fetal bovine dialyzed serum (manufactured by Life Technologies).

(3) Diagnosis of homologous recombination by genome PCR

Diagnosis of homologous recombination in the 900 clones obtained in the item (2) was carried out by genome PCR.

First, genomic DNA of each clone was prepared from the replica plate prepared in the item (2) in accordance with a known method [*Analytical Biochemistry,* 201, 331 (1992)] and dissolved overnight in 30 μl of a TE-RNase buffer (pH 8.0) (10 mmol/l Tris-HCl, 1 mmol/l EDTA, 200 μg/ml RNase A). Also, a primer (represented by SEQ ID NO:15)

which binds to a sequence outside the targeting vector homologous region among the FUT8 genome region obtained in Reference Example and a primer (represented by SEQ ID NO:16) which binds to the loxP sequence in the vector were designed.

Using a DNA polymerase ExTaq (manufactured by Takara Shuzo), 25 μl of a reaction solution [ExTaq buffer (manufactured by Takara Shuzo), 0.2 mmol/l dNTPs and 0.5 μmol/l gene-specific primers (SEQ ID NOs:15 and 16)] containing 10 μl of each the above-prepared genomic DNA solution were prepared, and polymerase chain reaction (PCR) was carried out. The PCR was carried out by heating at 94°C for 3 minutes and subsequent 38 cycles of heating at 94°C for 1 minute, 60°C for 1 minute and 72°C for 2 minutes as one cycle.

After the PCR, the reaction solution was subjected to 0.8% (w/v) agarose gel electrophoresis, and a specifically amplifying fragment of about 1.7 Kb containing a border region between the CHO cell genome region and the targeting vector homologous region was identified as a positive clone. One positive clone was found by the method.

(4) Diagnosis of homologous recombination by genome Southern blotting

Diagnosis of homologous recombination in the one clone in which positive signal was confirmed in the item (3) was carried out by genome Southern blotting.

Among the master plates cryo-preserved in the item (2), a 96 well plate containing the positive clone found in the item (3) was selected and incubated at 37°C for 10 minutes in 5% $CO_2$. After the incubation, cells were collected from a well corresponding to the positive clone and inoculated into a 24 well flat bottom plate for adhesion cell (manufactured by Greiner). After culturing for 1 week in IMDM medium (manufactured by Life Technologies) supplemented with 15 μg/ml of puromycin (manufactured by SIGMA) and 10% fetal bovine dialyzed serum (manufactured by Life Technologies), the cells were inoculated into a 6 well flat bottom plate for adhesion cell (manufactured by Greiner). A genomic DNA of each of the clones was prepared from the plate in accordance with a known method [*Nucleic Acids Research,* 3, 2303 (1976)] and dissolved overnight in 150 μl of a TE-RNase buffer (pH 8.0) (10 mmol/l Tris-HCl, 1 mmol/l EDTA, 200 μg/ml RNase A).

In 120 μl of NEBuffer 3 (manufactured by New England Biolabs), 12 μg of the obtained genomic DNA was dissolved, and 25 unites of a restriction enzyme *Pst*I (manufactured by New England Biolabs) were added thereto, followed by digestion at 37°C overnight. A DNA fragment was recovered from the reaction solution by ethanol precipitation, dissolved in 20 μl of TE buffer (pH 8.0, 10 mmol/l Tris-HCl, 1 mmol/l EDTA) and then subjected to 0.8% (w/v) agarose gel electrophoresis. After the electrophoresis, the genomic DNA was transferred onto a nylon membrane in accordance with a known method [*Proc. Natl. Acad. Sci. USA,* 76, 3683 (1979)]. After completion of the transfer, the nylon membrane was heated at 80°C for 2 hours.

Separately, a probe used in the Southern blotting was prepared as follows. First, primers (SEQ ID NOs:9 and 10) which bind to a sequence outside the targeting vector homologous region with the FUT8 genome region obtained in Reference Example (2) were designed. Next, using a DNA polymerase ExTaq (manufactured by Takara Shuzo), 20 μl of a reaction solution [ExTaq buffer (manufactured by Takara Shuzo), 0.2 mmol/l dNTPs and 0.5 μmol/l gene-specific primers (SEQ ID NOs:9 and 10] containing 4.0 ng of the plasmid pFUT8fgE2-2 obtained in Reference Example (2) was prepared, and polymerase chain reaction (PCR) was carried out. The PCR was carried out by heating at 94°C for 1 minute and subsequent 25 cycles of heating at 94°C for 30 seconds, 55°C for 30 seconds and 74°C for 1 minute as one cycle. After the PCR, the reaction solution was subjected to 1.75% (w/v) agarose gel electrophoresis to purify a probe DNA fragment of about 230 bp. The obtained probe DNA solution (5 μl) was labeled with a radioisotope using 1.75 MBq of [$\alpha$-$^{32}$P]dCTP and Megaprime DNA Labeling System, dCTP (manufactured by Amersham Pharmacia Biotech).

The hybridization was carried out as follows. First, the nylon membrane was sealed in a roller bottle, and prehybridization was carried out at 65°C for 3 hours by adding 15 ml of a hybridization solution [5 × SSPE, 50 × Denhaldt's solution, 0.5% (w/v) SDS, 100 μg/ml salmon sperm DNA]. Next, the $^{32}$P-labeled probe DNA was heat-denatured and put into the bottle. Then, the nylon membrane was heated at 65°C overnight.

After the hybridization, the nylon membrane was soaked in 50 ml of 2×SSC-0.1% (w/v) SDS and heated at 65°C for 15 minutes. After repeating the washing step twice, the membrane was soaked in 50 ml of 0.2 × SSC-0.1% (w/v) SDS and heated at 65°C for 15 minutes. After the washing, the nylon membrane was exposed to an X-ray film at -80°C for two nights for development.

By the restriction enzyme *Pst*I treatment, a DNA fragment of about 4.4 Kb is formed from a wild type FUT8 allele. On the other hand, a DNA fragment of about 6.0 Kb is formed from an allele in which homologous recombination with a targeting vector was generated.

By the method, such specific fragments of about 4.4 Kb and about 6.0 Kb were found from the positive clone genomic DNA in the item (3). Since the quantitative ratio of both fragments was 1 : 1, it was confirmed that the clone is a clone in which one copy of the FUT8 allele was disrupted. Hereinafter, the clone is referred to as clone 1st.ΔFUT8 2-46.

3. Deletion of drug-resistant gene from CHO cell in which one copy of FUT8 gene was disrupted

(1) Introduction of Cre recombinase expression vector

A Cre recombinase expression vector pBS185 (manufactured by Life Technologies) was introduced into the clone 1st.ΔFUT8 2-46 prepared in the item 2 of this Example.

The plasmid pBS 185 was introduced into the clone 1st.ΔFUT8 2-46 as follows in accordance with electroporation [*Cytotechnology,* 3, 133 (1990)]. First, the clone 1st.ΔFUT8 2-46 was suspended in a K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After mixing 200 μl of the cell suspension ($1.6\times10^6$ cells) with 4 μg of the plasmid pBS185, an entire volume of the cell-DNA mixture was transferred into Gene Pulser Cuvette (inter-electrode distance, 2 mm) (manufactured by BIO-RAD) and then the gene transfer was carried out by using a cell fusion apparatus Gene Pulser (manufactured by BIO-RAD) at 350 V pulse voltage and 250 μF electric capacity. After the gene transfer, the cell suspension was suspended in 10 ml of IMDM medium (manufactured by Life Technologies) supplemented with 10% fetal bovine serum (manufactured by Life Technologies) and 1 × concentration HT supplement (manufactured by Life Technologies) and further diluted 20,000-fold by using the same medium. The cells were inoculated into 7 adhesion cell culture dishes of 10 cm in diameter (manufactured by Falcon) and then cultured at 37°C for 24 hours in 5% $CO_2$. After culturing, the culture supernatant was removed and IMDM medium (manufactured by Life Technologies) supplemented with 10% fetal bovine dialyzed serum (manufactured by Life Technologies) was dispensed at 10 ml. Culturing was carried out for 10 days while repeating the medium exchange at intervals of 3 to 4 days.

(2) Preparation of Cre recombinase expression vector-introduced clone

Arbitrary 400 colonies were obtained as follows among the clones obtained in the item (1).

First, culture supernatant was removed from the 10 cm dish and 7 ml of a phosphate buffer was added to the dish which was subsequently set under a stereoscopic microscope. Next, each colony was scratched and sucked up by using Pipetteman (manufactured by GILSON) and transferred into a 96 well round-bottom plate (manufactured by Falcon). After a trypsin-treatment, each clone was inoculated into a 96 well flat-bottom plate for adhesion cell culture (manufactured by Iwaki Glass) and cultured for 1 week in IMDM medium (manufactured by Life Technologies) supplemented with 10% fetal bovine dialyzed serum (manufactured by Life Technologies).

After culturing, each clone in the plate was treated with trypsin and then mixed with two volumes of a freezing medium (20% DMSO, 40% fetal bovine serum, 40% IMDM). A half of the mixture was inoculated into a 96 well flat-bottom plate for adhesion cell culture use (manufactured by Iwaki Glass) to prepare a replica plate, while the remaining half was subjected to cryopreservation as a master plate.

Next, the replica plate was cultured for 6 days in IMDM medium (manufactured by Life Technologies) supplemented with 15 μg/ml puromycin (manufactured by SIGMA) and 10% fetal bovine dialyzed serum (manufactured by Life Technologies). A positive clone from which the puromycin-resistant gene interposed between loxP sequences was deleted by the expression of Cre recombinase dies out in the presence of puromycin. By the selection method, 91 positive clones were found.

(3) Diagnosis of drug-resistant gene deletion by genome Southern blotting

Diagnosis of drug-resistant gene deletion by genome Southern blotting was carried out on optional 6 clones among the positive clones found in the item (2).

Among the master plates cryo-preserved in the item (2), 96 well plates containing the 6 positive clones were selected and incubated at 37°C for 10 minutes in 5% $CO_2$. After the incubation, cells were collected from a well corresponding to each positive clone and inoculated into 24 well flat bottom plates for adhesion cell use (manufactured by Greiner). After culturing for 1 week in IMDM medium (manufactured by Life Technologies) supplemented with 10% fetal bovine dialyzed serum (manufactured by Life Technologies), the cells were inoculated into 6 well flat bottom plates for adhesion cell use (manufactured by Greiner). A genomic DNA of each of the clones was prepared from the plates in accordance with a known method [*Nucleic Acids Research,* 3, 2303 (1976)] and dissolved overnight in 150 μl of a TE-RNase buffer (pH 8.0) (10 mmol/l Tris-HCl, 1 mmol/l EDTA, 200 μg/ml RNase A).

In 120 μl of NEBuffer for *Bam*HI (manufactured by New England Biolabs), 12 μg of the obtained genomic DNA was dissolved, and 20 unites of a restriction enzyme *Bam*HI (manufactured by New England Biolabs) were added thereto, followed by digestion at 37°C overnight. A DNA fragment was recovered from the reaction solution by ethanol precipitation, dissolved in 20 μl of TE buffer (pH 8.0) (10 mmol/l Tris-HCl, 1 mmol/l EDTA) and then subjected to 0.4% (w/v) agarose gel electrophoresis. After the electrophoresis, the genomic DNA was transferred onto a nylon membrane in accordance with a known method [*Proc. Natl. Acad. Sci. USA,* 76, 3683 (1979)]. After completion of the transfer,

the nylon membrane was heated at 80°C for 2 hours.

Separately, a probe used in the Southern blotting was prepared as follows. First, primers (SEQ ID NOs:9 and 10) which bind to a sequence outside the targeting vector homologous region among the FUT8 genome region obtained in Reference Example were designed. Next, polymerase chain reaction (PCR) was carried out by using a DNA polymerase ExTaq (manufactured by Takara Shuzo), by preparing 20 μl of a reaction solution [ExTaq buffer (manufactured by Takara Shuzo), 0.2 mmol/l dNTPs and 0.5 μmol/l gene-specific primers (SEQ ID NOs:9 and 10)] containing 4.0 ng of the plasmid pFUT8fgE2-2 obtained in Reference Example (2). The PCR was carried out by heating at 94°C for 1 minute and subsequent 25 cycles of heating at 94°C for 30 seconds, 55°C for 30 seconds and 74°C for 1 minute as one cycle. After the PCR, the reaction solution was subjected to 1.75% (w/v) agarose gel electrophoresis to purify a probe DNA fragment of about 230 bp. Using 1.75 MBq of [α-$^{32}$P]dCTP and Megaprime DNA Labeling System, dCTP (manufactured by Amersham Pharmacia Biotech), 5 μl of the obtained probe DNA solution was radioisotope-labeled.

The hybridization was carried out as follows. First, the nylon membrane was sealed in a roller bottle, and pre-hybridization was carried out at 65°C for 3 hours by adding 15 ml of a hybridization solution [5 × SSPE, 50 × Denhaldt's solution, 0.5% (w/v) SDS, 100 μg/ml salmon sperm DNA]. Next, the $^{32}$P-labeled probe DNA was heat-denatured and put into the bottle and the nylon membrane was heated overnight at 65°C.

After the hybridization, the nylon membrane was soaked in 50 ml of 2 × SSC-0.1% (w/v) SDS and heated at 65°C for 15 minutes. After repeating the washing step twice, the membrane was soaked in 50 ml of 0.2 × SSC-0.1% (w/v) SDS and heated at 65°C for 15 minutes. After washing the nylon membrane, it was exposed to an X-ray film two nights at -80°C for development.

By the treatment with restriction enzyme *Bam*HI described above, a DNA fragment of about 25.5 Kb was formed from a wild type FUT8 allele. Also, a DNA fragment of about 20.0 Kb was formed from an allele in which homologous recombination with a targeting vector was generated. Furthermore, when the puromycin-resistant gene (about 1.5 Kb) was deleted from the allele in which homologous recombination was generated, a DNA fragment of about 18.5 Kb was formed by the same treatment.

By the method, the specific fragments of about 25.5 Kb and about 18.5 Kb were found from the genomic DNA of 5 clones among the 6 clones. Since the quantitative ratio of both fragments was 1 : 1, it was shown that the puromycin-resistant gene was deleted from the clones in which one copy of the FUT8 genome region was disrupted. Hereinafter, the clone is referred to as 1st.ΔFUT8 2-46-1. Also, results of the genome Southern blotting of the clone 1st.ΔFUT8 2-46-1, the clone 1st.ΔFUT8 2-46 and the clone 5-03 are shown in Fig. 12. Also, the clone 1st.ΔFUT8 2-46-1, as a name of 2-46-1, has been deposited on September 26, 2001, as FERM BP-7755 in International Patent Organism Depositary, National Institute of Advanced Industrial Science and Technology (Tsukuba Central 6, 1, Higashi 1-Chome Tsukuba-shi, Ibaraki-ken 305-8566 Japan).

4. Purification of antibody produced by FUT8 gene-disrupted clone

The clone 1st.ΔFUT8 2-46-1 obtained in the item 3 of this Example by disrupting one copy of the FUT8 allele was suspended in IMDM medium (manufactured by Life Technologies) supplemented with 15 μg/ml of puromycin (manufactured by SIGMA) and 10% fetal bovine dialyzed serum (manufactured by Life Technologies) to give a density of 3 × 10$^5$ cells/ml, and then 60 ml in total of the suspension was inoculated into two T182 flasks for adhesion cell culture use (manufactured by Greiner). After culturing for 3 days, the supernatant was discarded and changed to a total of 60 ml of EXCELL301 medium (manufactured by JRH Biosciences).

After culturing at 37°C for 7 days in a 5% $CO_2$ incubator, the number of intact cells was counted to confirm that their viability was almost the same (each 30% or less), and then each cell suspension was recovered. The cell suspension was centrifuged at 3,000 rpm at 4°C for 10 minutes, and the recovered supernatant was centrifuged at 10,000 rpm at 4°C for 1 hour and then filtered by using 150 ml capacity PES Filter Unit (manufactured by NALGENE) having a pore diameter of 0.22 μm.

Prosep-A High Capacity (manufactured by bioPROCESSING) was packed in a 0.8 cm diameter column to a thickness of 2 cm and washed with 10 ml of 0.1 mol/l citrate buffer (pH 3.0) and 10 ml of 1 mol/l glycine/NaOH-0.15 mol/l NaCl buffer (pH 8.6) in this order to effect equilibrate the carrier. Next, 100 ml of each of the culture supernatant was passed through the column and washed with 50 ml of 1 mol/l glycine/NaOH-0.15 mol/l NaCl buffer (pH 8.6). After washing it, the antibody absorbed to Prosep-A was eluted by using 2.5 ml of 0.1 mol/l citrate buffer (pH 3.0), the eluate was fractionated at 500 μl and each fraction was neutralized by mixing with 100 μl of 2 mol/l Tris-HCl (pH 8.5). Two fractions containing the antibody at a high concentration (1.2 ml in total) were selected by the BCA method [*Anal. Biochem.,* 150, 76 (1985)], combined and then dialyzed against 10 mol/l citrate-0.15 mol/l NaCl buffer (pH 6.0) at 4°C for a whole day and night. After the dialysis, the antibody solution was recovered and subjected to sterile filtration by using Millex GV having a pore size of 0.22 μm (manufactured by MILLIPORE).

5. ADCC activity of antibody composition produced by FUT8 gene-disrupted clone

In order to evaluate ADCC activity of the anti-CCR4 antibody purified in the item 4 of this Example, the ADCC activity was measured by using the CCR4-positive clone CCR4/EL-4 described in WO 01/34754.

After $1\times10^6$ cells of the CCR4/EL-4 clone subcultured in RPMI1640 medium (manufactured by Life Technologies) supplemented with 10% fetal bovine serum (manufactured by Life Technologies) (hereinafter referred to as "RPMI1640-FBS(10)") were suspended in 500 μl of RPMI1640-FBS(10), 3.7 MBq of $Na_2{}^{51}CrO_4$ was added thereto, followed by culturing at 37°C for 90 minutes to label the cells with a radioisotope. After centrifugation at 1,200 rpm for 5 minutes, the supernatant was discarded and the target cells were suspended in 5 ml of RPMI1640-FBS(10). The washing step was repeated three times and then the cell suspension was incubated for 30 minutes on ice for spontaneous dissociation of the radioactive substance. The washing step was again repeated twice and then the cells were suspended in 5 ml of RPMI1640-FBS(10) to thereby prepare $2.0\times10^5$ cells/ml of a target cell suspension.

Separately, 30 ml of venous blood was collected from a healthy doner, gently mixed with 0.5 ml of heparin sodium (manufactured by Shimizu Pharmaceutical) and then mixed with 30 ml of physiological saline (manufactured by Otsuka Pharmaceutical). After mixing them, 10 ml of the mixture was gently overlaid on 4 ml of Lymphoprep (manufactured by NYCOMED PHARMA AS) and centrifuged at room temperature at 2,000 rpm for 30 minutes. The separated mononuclear cell fractions were collected from the centrifugation tubes, combined and then suspended in 30 ml of RPMI1640-FBS(10). After centrifugation at room temperature at 1,200 rpm for 15 minutes, the supernatant was discarded and the cells were suspended in 20 ml of RPMI1640-FBS(10). The washing step was repeated twice and then $2.5\times10^6$ cells/ml of an effector cell suspension was prepared by using RPMI1640-FBS(10).

The target cell suspension was dispensed at 50 μl ($1\times10^4$ cells/well) into each well of a 96 well U-bottom plate (manufactured by Falcon). Subsequently, the effector cell suspension was dispensed at 100 μl ($2.5\times10^5$ cells/well) into each well to thereby adjust the ratio of the effector cells to the target cells to 25 : 1. Next, using RPMI1640-FBS (10), a series of dilution solution of 0.01 μg/ml, 0.1 μg/ml, 1 μg/ml and 10 μg/ml was prepared from each of the anti-CCR4 antibodies obtained in the item 4 of this Example, and the diluted solutions were dispensed at 50 μl into the wells to give final concentrations of 0.0025 μg/ml, 0.025 μg/ml, 0.25 μg/ml and 2.5 μg/ml, respectively. After the reaction at 37°C for 4 hours in 5% $CO_2$, the plate was centrifuged at 1,200 rpm for 5 minutes. Into a 12 mm diameter RIA tube (manufactured by IWAKI), 75 μl of the supernatant in each well was batched off and the amount of the dissociated $^{51}Cr$ was measured by using MINAX-γ auto-gamma counter 5550 (manufactured by PACKARD).

Also, the amount of the spontaneously dissociated $^{51}Cr$ was calculated by carrying out the same reaction in a reaction mixture in which 150 μl of RPMI1640-FBS(10) was added, instead of the effector cell suspension and antibody solution. The amount of the total dissociated $^{51}Cr$ was calculated by carrying out the same reaction in a reaction mixture in which 100 μl of 1 N hydrochloric acid and 50 μl of RPMI1640-FBS(10) were added, instead of the effector cell suspension and antibody solution. Using these values, the ADCC activity was calculated based on equation (I) described in the item (3) of Example 2.

Fig. 13 shows ADCC activity of each of the anti-CCR4 antibodies. The antibody obtained from the clone 1st. ΔFUT8 2-46-1 in which one copy of the FUT8 allele was disrupted showed a significantly higher ADCC activity than the antibody produced by the clone 5-03 which is the CHO clone before gene disruption. Also, changes in the antigen binding activity of these antibodies were not observed. Based on the results, it was confirmed that the ADCC activity of produced antibodies can be improved by disrupting the FUT8 allele in host cells.

Example 4

Preparation of high drug-resistant clone from CHO cell in which one copy of FUT8 gene was destroyed:

It is generally known that a clone in which both alleles were destroyed is obtained by culturing a cell in which one allele of a genomic gene obtained by a homologous recombination technique using a target vector is destroyed, in a medium in which the agent concentration used for positive selection in selecting a target vector-inserted clone is increased to about 10 times, and then isolating a clone resistant to the drug [*Manipulating the Mouse Embryo, A Laboratory Manual; Gene Targeting, A Practical Approach,* IRL Press at Oxford University Press (1993), *Preparation of Mutant Mice using ES Cells*].

Thus, a transformant in which 2 copies of the FUT8 gene were destroyed was prepared as follows according to a known method [*Molecular and Cellular Biology,* 12, 2391 (1992)] using the clone 1st.ΔFUT8 2-46 obtained in the item 2(3) of Example 3.

(1) Preparation of high concentration puromycin-resistant clone

The clone 1st.ΔFUT8 2-46 was suspended in an amount of $1\times10^8$ cells in IMDM medium (manufactured by

Life Technologies) supplemented with 10% dialyzed fetal bovine serum (manufactured by Life Technologies), inoculated into 20 dishes of a 10 cm dish for adhered cell culture (manufactured by Falcon) and then cultured at 37°C for 24 hours in the presence of 5% $CO_2$. After culturing, the supernatants were discarded and IMDM medium (manufactured by Life Technologies) supplemented with 150 μg/ml puromycin (manufactured by SIGMA) and 10% dialyzed fetal bovine serum (manufactured by Life Technologies) was dispensed at 10 ml. While repeating this medium exchange at intervals of 3 to 4 days, culturing was carried out for 12 days.

The formed 90 drug-resistant colonies were collected by .the following procedure. First, culture supernatant was discarded from the 10 cm dish and replaced with 7 ml of a phosphate buffer and then the dish was placed under a stereoscopic microscope. Next, the colonies were peeled off and sucked up by using Pipetteman (manufactured by GILSON) and transferred into 96 well round bottom plates (manufactured by Falcon). After a trypsin treatment, each clone was inoculated into 96 well flat bottom plates for adhered cell (manufactured by Iwaki Glass) and cultured for 1 week in IMDM medium (manufactured by Life Technologies) supplemented with 150 μg/ml puromycin (manufactured by SIGMA) and 10% dialyzed fetal bovine serum (manufactured by Life Technologies).

After culturing, each clone on the plate was subjected to a trypsin treatment and mixed with two volumes of a medium for freezing (20% DMSO, 40% fetal bovine serum, 40% IMDM). A half volume thereof was inoculated into a 96 well flat bottom plate for adhered cell (manufactured by Iwaki Glass) to prepare a replicate, and the remaining half volume was subjected to cryopreservation as the master plate. The replicate was cultured for 1 week in IMDM medium (manufactured by Life Technologies) supplemented with 15 mg/ml puromycin (manufactured by SIGMA) and 10% dialyzed fetal bovine serum (manufactured by Life Technologies).

(2) Diagnosis of homologous recombination by genome Southern blotting

Diagnosis of homologous recombination by genome Southern blotting was carried out for all of the drug-resistant clones obtained in the item ( I ) by the following procedure.

After the replicate prepared in the item (1) was treated with trypsine, all of the clones were inoculated into 24 well flat bottom plates for adhered cell (manufactured by Greiner). All of the clones were cultured for 1 week in IMDM medium (manufactured by Life Technologies) supplemented with 150 μg/ml puromycin (manufactured by SIGMA) and 10% dialyzed fetal bovine serum (manufactured by Life Technologies), treated with trypsin and inoculated into 6 well flat bottom plates for adhered cell (manufactured by Greiner). A genomic DNA of each clone was prepared from the plate according to a known method [*Nucleic Acids Research,* 3, 2303 (1976)] and dissolved overnight in 150 μl of TE-RNase buffer (pH 8.0) (10 mmol/l Tris-HCl, 1 mmol/l EDTA, 200 mg/ml RNase A).

In 120 μl of NE Buffer for BamHI (manufactured by New England Biolabs), 12 μg of the genomic DNA prepared in the above was dissolved, and 25 units of a restriction enzyme *Bam*HI (manufactured by New England Biolabs) were added thereto, followed by digestion at 37°C. DNA fragments were recovered from the reaction solution by the ethanol precipitation method, dissolved in 20 μl of TE buffer (pH 8.0, 10 mmol/l Tris-HCl, 1 mmol/l EDTA) and then subjected to 0.6% (w/v) agarose gel electrophoresis. After the electrophoresis, the genomic DNA was transferred on a nylon membrane according to a known method [*Proc. Natl. Acad. Sci. USA,* 76, 3683 (1979)]. After the transfer, the nylon membrane was subjected to heat treatment at 80°C for 2 hours.

On the other hand, a probe for Southern blotting was prepared as follows. Using a DNA polymerase ExTaq (manufactured by Takara Shuzo), polymerase chain reaction (PCR) was carried out by preparing 20 μl of a reaction solution [ExTaq buffer (manufactured by Takara Shuzo), 0.2 mmol/l dNTPs, 0.5 μmol/l of the above gene-specific primers (SEQ ID NOs:9 and 10)]. PCR was carried out by heating at 94°C for 1 minute and subsequent 25 cycles of at 94°C for 30 seconds, 55°C for 30 seconds and 74°C for 1 minute as one cycle. After the PCR, the reaction solution was subjected to 1.75% (w/v) agarose gel electrophoresis to purify a probe DNA fragment of about 230 bp. Using 1.75 MBq of [$\alpha^{32}$P]dCTP and Megaprime DNA Labeling System, dCTP (manufactured by Amersham Pharmacia Biotech), 5 μl of the obtained probe DNA solution was subjected to radioisotope-labeling.

The hybridization was carried out as follows. First, the above nylon membrane was sealed in a roller bottle, and pre-hybridization was carried out at 65°C for 3 hours by adding 15 ml of a hybridization solution [5 × SSPE, 50 × Denhardt's solution, 0.5% (w/v) SDS, 100 mg/ml salmon sperm DNA]. Next, the $^{32}$P-labeled probe DNA was heat-denatured, put into the bottle and heated overnight at 65°C.

After the hybridization, the nylon membrane was soaked in 50 ml of 2 × SSC-0.1% (w/v) SDS and heated at 65°C for 15 minutes. After repeating this washing step twice, the membrane was soaked in 50 ml of 0.2 × SSC-0.1% (w/v) SDS and heated at 65°C for 15 minutes. After the washing, the nylon membrane was developed by exposing it to an X-ray film at -80°C two nights.

A DNA fragment of about 25.5 Kb is obtained from the wild type FUT8 allele by the above restriction enzyme *Bam*HI treatment. On the other hand, a DNA fragment of about 20.0 Kb is formed from the allele resulting from homologous recombination with the targeting vector by the same restriction enzyme treatment.

According to this method, a clone showing only the above homologous recombination region-specific fragment

of about 14.0 Kb was found (Fig. 14). This clone is named clone 2-46-H10.

Example 5

Preparation of CHO/DG44 cells in which all FUT8 genes existing on the genome were disrupted:
CHO/DG44 clone in which the genome region containing the translation initiation codon of the FUT8 both alleles was deleted was prepared.

1. Construction of Chinese Hamster FUT8 gene exon 2 targeting vector plasmid pKOFUT8Neo

A plasmid pKOFUT8Neo was constructed by substituting the puromycin-resistant gene expression unit contained in the targeting vector plasmid pKOFUT8Puro obtained in paragraph 1 of Example 3 with a neomycin-resistant gene expression unit by the following procedure (Fig. 15).
In 50 μl of NEBuffer 4 (manufactured by New England Biolabs), 1.0 μg of the plasmid pKOSelectNeo (manufactured by Lexicon) was dissolved, and 16 units of a restriction enzyme *Asc*I (manufactured by New England Biolabs) were added thereto, followed by digestion at 37°C for 2 hours. From the reaction solution, DNA fragments were collected using the ethanol precipitation method and dissolved in 50 μl of NEBuffer 4 (manufactured by New England Biolabs) containing 100 μg/ml BSA (manufactured by New England Biolabs), and 20 units of a restriction enzyme *Apa*LI (manufactured by New England Biolabs) was added thereto, followed by digestion at 25°C for 2 hours. After the digestion, the obtained liquid was subjected to 0.8 % (w/v) agarose gel electrophoresis and the DNA fragment of about 1.6 Kb containing the neomycin-resistant gene expression unit was purified.
Separately, 1.0 μg of the plasmid pKOFUT8Puro was dissolved in 50 μl of NEBuffer 4 (manufactured by New England Biolabs), 16 units of a restriction enzyme *Asc*I (manufactured by New England Biolabs) were added thereto, followed by digestion at 37°C for 2 hours. After the digestion, 30 μL of 1 mol/l Tris-HCl buffer of pH 8.0 and 3.0 μl of alkaline phosphatase derived from *E. coli* C15 (manufactured by Takara Shuzo) were added thereto, and the reaction was carried out at 65°C for 1 hour to dephosphorylate the DNA termini. After the dephosphorylation, a phenol/chloroform extraction and an ethanol precipitation were carried out and the recovered DNA fragments were dissolved in 10 μl of sterilized water.
After 1.0 μl of an *Asc*I-*Asc*I fragment (about 1.6 Kb) derived from the plasmid pKOSelectNeo and 1.0 μl of an *Asc*I-*Asc*I fragment (about 10.1 Kb) derived from the plasmid pKOFUT8Puro obtained in the above, 3.0 μl of sterilized water and 5.0 μl of Ligation High (manufactured by Toyobo) were mixed, and the reaction was carried out at 16°C for 30 minutes for ligation. Using the reaction solution, *E. coli* DH5α strain was transformed, and each plasmid DNA was isolated from with the obtained ampicillin resistant clone according to a known method. This plasmid is referred to as pKOFUT8Neo.

2. Preparation of CHO/DG44 cell in which one copy of the FUT8 gene on the genome was disrupted

(1) Preparation of targeting vector-introduction clone

In CHO/DG44 cell derived from Chinese Hamster ovary in which dihydrofolic acid reductase gene (*dhfr*) was deficient [*Somatic Cell and Molecular Genetics,* 12, 555 (1986)], the Chinese Hamster FUT8 genome region targeting vector pKOFUT8Neo constructed in the item 1 of this Example was introduced. The gene introduction into the CHO/DG44 cell of the plasmid pKOFUT8Neo was carried out by electroporation [*Cytotechnology,* 3, 133 (1990)] according to the following procedure. First, 280 μg of the plasmid pKOFUT8Neo were dissolved in 1.2 ml NEBuffer for *Sal*I (manufactured by New England Biolabs) containing 100 μg/ml BSA (manufactured by New England Biolabs), then 400 units of a restriction enzyme *Sal*I (manufactured by New England Biolabs) were added thereto, and the linearization was carried out by digestion at 37°C for 5 hours. The reaction solution was extracted with phenol/chloroform extraction, followed by ethanol precipitation, and the recovered linear plasmid was made into a 1 μg/μl aqueous solution. Separately, the CHO/DG44 cell was suspended in K-PBS buffer (137 mmol/l KCl, 2.7 mmol/l NaCl, 8.1 mmol/l $Na_2HPO_4$, 1.5 mmo/l $KH_2PO_4$, 4.0 mmol/l $MgCl_2$) to give a density of $8\times10^7$ cells/ml. After 200 μl of the cell suspension ($1.6\times10^6$ cells) were mixed with 4 μl (4 μg) of the above described linearized plasmid, the whole amount of the cell-DNA mixture liquid was transferred into Gene Pulser Cuvette (electrode distance: 2 mm) (manufactured by BIO-RAD) and the gene introduction was carried out at a pulse voltage of 350 V and a capacity of 250 μF by using Gene Pulser cell fusion device (manufactured by BIO-RAD). The gene-introduced cell suspension was suspended in IMDM medium (manufactured by Invitrogen) supplemented with 10% fetal bovine serum (manufactured by Invitrogen) and 1 × concentration HT supplement (manufactured by Invitrogen), and inoculated onto 10 cm dishes for adhesion cell culture (manufactured by Falcon). After culturing at 5% $CO_2$ and 37°C for 24 hours, the culture supernatant was removed, and IMDM medium (manufactured by Invitrogen) supplemented with 600 μg/ml G418 (manufactured by Nacalai Tesque), 1 × concentration

HT supplement (manufactured by Invitrogen) and 10% fetal bovine serum (manufactured by Invitrogen) was dispensed into each well at 10 ml/well. The culturing was carried out for 15 day while the culture medium exchange was repeated every 3 to 4 days to obtain G418-resistant clones.

(2) Diagnosis of homologous recombination by genome PCR

The diagnosis of the homologous recombination of the G418-resistant clone obtained in the item (1) was carried out by genome PCR according to the following procedure.

The G418-resistant clones obtained in 96 well plates were treated with trypsin, and then mixed with the 2-fold amount of a freezing culture medium (20% DMSO, 40% fetal bovine serum, 40% IMDM). The half amount of the mixture was inoculated onto a flat bottom 96 well plates for adhesion cell (manufactured by Asahi Technoglass) as a replica plate, while the remaining half was used for cryopreservation as a master plate.

The replica plate was incubated for 1 week in IMDM medium (manufactured by Invitrogen) supplemented with 600 μg/ml G418 (manufactured by Nacalai Tesque), 1 × concentration HT supplement (manufactured by Invitrogen) and 10% fetal bovine serum (manufactured by Invitrogen), a genomic DNA of each clone was prepared according to a known method [*Analytical Biochemistry,* 201, 331 (1992)], and each of them was dissolved overnight in 30 μl of TE-RNase buffer (pH 8.0) (10 mmol/l Tris-HCl, 1 mmol/l ETDA, 200 μg/ml RNase A).

A following polymerase chain reaction (PCR) was carried out as follows by using primers (SEQ ID NO:13 or 15) combining with the sequence of the part which exceeded the targeting vector homologous region within the FUT8 genome region obtained by Reference Example, and primers (SEQ ID NO:14 or 16) combining with the inner-vector sequence. Specifically, 25 μl of a reaction solution containing 10 μl of each of the above prepared genomic DNA solution [DNA polymerase ExTaq (manufactured by Takara Shuzo), ExTaq buffer (manufactured by Takara Shuzo), 0.2 mmol/l dNTPs, 0.5 μmol/l of the above described gene specific primer (the forward primer is shown by SEQ ID NO:13 or 15, the reverse primer is shown by SEQ ID NO:14 or 16)] was prepared and the PCR was carried out by heating at 94°C for 3 minutes and subsequent heating at 94°C for 1 minute at 94°C, 60°C for 1 minute and 72°C for 2 minutes as one cycle.

After the PCR, the reaction solution was subjected to 0.8 % (w/v) agarose gel electrophoresis and the observed specific amplification of about 1.7 Kb, including the boundary part of the CHO cell genome region and the targeting vector homologous region, was determined as a positive clone (50-10-104).

(3) Diagnosis of homologous recombination by genome Southern blotting

The diagnosis of homologous recombination of the positive confirmed clone obtained in the item (2) was carried out by genome Southern blotting according to the following procedure.

Among the master plates stored in a frozen state, a 96 well plate containing the positive clone found in the item (2) was selected and allowed to stand for 10 minutes at 5% $CO_2$ and 37°C. After the standing, cells from the wells corresponding to the positive clone were inoculated onto a flat bottom 24 well plate for adherent cell (manufactured by Greiner). After culturing for 1 week in IMDM medium (manufactured by Invitrogen) supplemented with 600 μg/ml G418 (manufactured by Nacalai Tesque), 1 × concentration HT supplement (manufactured by Invitrogen) and 10% fetal bovine serum (manufactured by Invitrogen), the cells were inoculated onto a flat bottom 6 well plate for adherent cell (manufactured by Greiner). A genomic DNA of each clone was prepared from the plate according to a known method [*Nucleic Acids Research,* 3, 2303, (1976)] and dissolved overnight in 150 μl of TE-RNase buffer solution (pH 8.0) (10 mmol/l Tris-HCl, 1 mmol/l ETDA, 200 μg/ml RNase A).

In 120 μl of NEBuffer for *Bam*HI (manufactured by New England Biolabs) containing 100 μg/ml BSA (manufactured by New England Biolabs), 12 μg of the above prepared genomic DNA was dissolved, and 25 units of a restriction enzyme *Bam*HI (manufactured by New England Biolabs) were added thereto, followed by digestion overnight at 37°C. From the reaction solution, the DNA fragments were collected by the ethanol precipitation method, dissolved in 20 μl of TE buffer solution (pH 8.0) (10 mmol/l Tris-HCl, 1 mmol/l EDTA) and subjected to 0.6 % (w/v) agarose gel electrophoresis. After the electrophoresis, the genomic DNA was transferred onto a nylon membrane by a known method [*Proc. Natl. Acad. Sci. USA,* 76, 3683, (1979)]. After the transfer, the nylon membrane was subjected to a heat treatment at 80°C for 2 hours.

Separately, the probe for Southern blotting was prepared as follows. First, PCR was carried out according to the following procedure using the primer (SEQ ID NOs:9 and 10) combining with the sequence of the part which exceeded the targeting vector homologous region within the FUT8 genome region obtained by Reference Example. Specifically, 20 μl of a reaction solution [DNA polymerase ExTaq (manufactured by Takara Shuzo), ExTaq buffer (manufactured by Takara Shuzo), 0.2 mmol/l dNTPs, 0.5 μmol/l of the above described gene specific primer (SEQ ID NOs: 9 and 10)] containing 4.0 ng of the plasmid pFUT8fgE2-2 obtained in Reference Example (2) were prepared and PCR was carried out by 25 cycles of heating at 94°C for 1 minute, 94°C for 30 seconds, 55°C for 30 seconds and 74°C for

1 minute as one cycle. After the PCR, the reaction solution was subjected to 1.75% (w/v) agarose gel electrophoresis and a probe DNA fragment of about 230 bp was purified. To 5 μl of the probe DNA solution, radiation labeling was carried out by using [$\alpha$-$^{32}$P]dCTP 1.75 MBq and Megaprime DNA Labeling system, dCTP (manufactured by Amersham Pharmacia Biotech).

Hybridization was carried out as follows. First, the above-described nylon membrane was encapsulated in a roller bottle, and 15 ml of a hybridization solution [5 × SSPE, 50 × Denhalt's solution, 0.5 % (w/v) SDS, 100 μg/ml salmon sperm DNA] was added thereto, followed by hybridization at 65°C for 3 hours. Next, the probe DNA labeled with $^{32}$P was heat-degenerated and poured into a bottle, followed by heating overnight at 65°C.

After the hybridization, the nylon membrane was soaked into 50 ml of 0.2 × SSC-0.1 % (w/v) SDS and heated at 65°C for 15 minutes. After the above-described washing operation was repeated twice, the membrane was soaked into 50 ml of 0.2 × SSC-0.1% (w/v) SDS and heated at 65°C for 15 minutes. After the washing, the nylon membrane was developed by exposing it to an X-ray film at -80°C.

By the above-described treatment with a restriction enzyme *Bam*HI, a DNA fragment of about 25.5 Kb was obtained from the wild type FUT8 allele. On the other hand, by the same restriction enzyme treatment, a DNA fragment of about 20.0 Kb was obtained from the allele in which a homologous recombination with the targeting vector had occurred.

Based on the present method, the above-described specific fragments of about 25.5 Kb and about 20.0 Kb were found from the genomic DNA of the positive clone 50-10-104 (Fig. 16). Since the quantity ratio of both fragments was 1 : 1, it was confirmed that the clone is a hemi-knockout clone in which one copy of the FUT8 allele was disrupted.

3. Preparation of CHO/DG44 cell in which FUT8 gene on genome was double knocked out

(1) Preparation of targeting vector-introduced clone

The Chinese hamster FUT8 genome region targeting vector pKOFUT8Puro constructed in the item 1 of Example 3 was introduced in the FUT8 hemi-knockout clone 50-10-104 obtained in the item 2 of this Example.

The gene of the plasmid pKOFUT8Puro was introduced by electroporation [*Cytotechnology,* 3, 133 (1990)] by the following procedure. First, 440 μg of the plasmid pKOFUT8Puro was dissolved in 2.4 ml of NEBuffer for *Sal*I (manufactured by New England Biolabs) containing 100 μg/ml BSA (manufactured by New England Biolabs), 800 units of a restriction enzyme *Sal*I (manufactured by New England Biolabs) were added thereto, and the linearization was carried out by digestion at 37°C for 5 hours. The reaction solution was extracted with phenol/chloroform extraction, followed by ethanol precipitation, and the recovered linear plasmid was made into a 1 μg/μl aqueous solution. Separately, 50-10-104 was suspended in K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8 \times 10^7$ cells/ml. After 200 μl of the cell suspension ($1.6 \times 10^6$ cells) was mixed with 4 μl (4 μg) of the above-described linearized plasmid, the whole amount of the cell-DNA mixture liquid was transferred into Gene Pulser Cuvette (electrode distance: 2 mm) (manufactured by BIO-RAD) and the gene introduction was carried out at a pulse voltage of 350 V and a capacity of 250 μF using Gene Pulser cell fusion device (manufactured by BIO-RAD). The gene-introduced cell suspension was suspended in IMDM medium (manufactured by Invitrogen) supplemented with 10% fetal bovine serum (manufactured by Invitrogen) and 1 × concentration HT supplement (manufactured by Invitrogen), and inoculated onto a 10 cm Dish for adherent cell culturing (manufactured by Falcon). After the cells were incubated for 24 hours at 5 % $CO_2$ and 37°C, the culture supernatant was removed and IMDM medium (manufactured by Invitrogen) supplemented with 15 μg/ml puromycin (manufactured by SIGMA), 1 × concentration HT supplement (manufactured by Invitrogen) and 10% fetal bovine serum (manufactured by Invitrogen) was dispensed at 10 ml. The culturing was carried out for 15 days while the culture medium exchange process was repeated every 7 days to obtain a drug-resistant clone.

(2) Diagnosis of homologous recombination by genome Southern blotting

The diagnosis of the homologous recombination of the drug-resistant clone obtained in the item (1) was carried out by genome Southern blotting concerning according to the following procedure.

The culture supernatant was removed from the 10 cm dish in which the puromycin-resistant clone was found, and 7 ml of a phosphate buffer was injected and then transferred under a stereoscopic microscope. Next, the colony was scratched off and sucked up with Pipetman (manufactured by GILSON) and collected in a round-bottom 96 well plate (manufactured by Falcon). After trypsin treatment, each clone was inoculated onto a flat-bottom 96 well plate for adhesion cell (manufactured by Asahi Technoglass) and cultured for 1 week in IMDM medium (manufactured by Invitrogen) supplemented with 15 μg/ml puromycin (manufactured by SIGMA), 1 × concentration HT supplement (manufactured by Invitrogen) and 10% fetal bovine serum (manufactured by Invitrogen).

After culturing, each clone of the plate was treated with trypsin, and then they were seeded on a flat-bottom

24 well plate for adhesion cell (manufactured by Greiner). After culturing for 1 week in IMDM medium (manufactured by Invitrogen) supplemented with 15 μg/ml puromycin (manufactured by SIGMA), 1 × concentration HT supplement (manufactured by Invitrogen) and 10% fetal bovine serum (manufactured by Invitrogen), the clone was inoculated onto a flat-bottom 6 well plate for adhesion cell (manufactured by Greiner). From the plate, a genomic DNA of each clone was prepared according to a known method [*Nucleic Acids Research,* 3, 2303, (1976)] and dissolved overnight in 150 μl of TE-RNase buffer (pH 8.0) (10 mmol/l Tris-HCl, 1 mmol/l ETDA, 200 μg/ml RNase A).

In 120 μl of NEBuffer for *Bam*HI (manufactured by New England Biolabs) containing 100 μg/ml BSA (manufactured by New England Biolabs), 12 μg of the above prepared genomic DNA was dissolved and 25 units of a restriction enzyme *Bam*HI (manufactured by New England Biolabs) were added, followed by digestion overnight at 37°C. From the reaction solution, the DNA fragments were collected by the ethanol precipitation method, dissolved in 20 μl of TE buffer solution (pH 8.0) (10 mmol/l Tris-HCl, 1 mmol/l EDTA) and subjected to 0.6 % (w/v) agarose gel electrophoresis. After the electrophoresis, the genomic DNA was transferred onto a nylon membrane in accordance with a known method [*Proc. Natl. Acad. Sci. USA,* 76, 3683, (1979)]. After the transfer, the nylon membrane was heated at 80°C for 2 hours.

Separately, the probe used for Southern blotting was prepared as follows. First, the PCR was carried out according to the following procedure using the primer (SEQ ID NOs:11 and 12) combining with the sequence of the part which exceeded the targeting vector homologous region within the FUT8 genome region. Specifically, 20 μl of a reaction solution [DNA polymerase ExTaq (manufactured by Takara Shuzo), ExTaq buffer (manufactured by Takara Shuzo), 0.2 mmol/l dNTPs, 0.5 μmol/l of the above-described gene specific primer (SEQ ID NOs:11 and 12)] containing 4.0 ng of the plasmid pFUT8fgE2-2 obtained in Reference Example (2) was prepared and the PCR was carried out by heating at 94°C for 1 minute and subsequent 25 cycles of a reaction at 94°C for 30 seconds, 55°C for 30 seconds and 74°C for 1 minute as one cycle. After the PCR, the reaction solution was subjected to 1.75 % (w/v) agarose gel electrophoresis and a probe DNA fragment of about 230 bp was purified. To 5 μl of the probe DNA solution, the radiation labeling was carried out by using [$\alpha$-$^{32}$P]dCTP 1.75 MBq and Megaprime DNA Labeling system, dCTP (manufactured by Amersham Pharmacia Biotech).

Hybridization was carried out as follows. First, the above described nylon membrane was encapsulated into a roller bottle, 15 ml of the hybridization solution [5 × SSPE, 50 × Denhalt's solution, 0.5 % (w/v) SDS, 100 μg/ml salmon sperm DNA] was added thereto and prehybridization was carried out at 65°C for 3 hours. Then, the probe DNA labeled with $^{32}$P was heat degenerated and poured into a bottle and heated overnight at 65°C.

After the hybridization, the nylon membrane was soaked into 50 ml of 0.2 × SSC-0.1% (w/v) SDS and heated at 65°C for 15 minutes. The above-described washing operation is repeated twice, and then the membrane is washed with 50 ml of 0.2 × SSC-0.1% (w/v) SDS and heated at 65°C for 15 minutes. After the washing, the nylon membrane was developed by exposing it to an X-ray film at -80°C.

By the above- described treatment with a restriction enzyme *Bam*HI, a DNA fragment of about 25.5 Kb was obtained from the wild type FUT8 allele. On the other hand, by the same restriction enzyme treatment, a DNA fragment of about 20.0 Kb was obtained from the alleles in which a homologous recombination with the targeting vector had occurred.

By the present method, only fragments of about 20.0 kb specific for the homologous recombination region were found from the genomic DNA of the drug-resistant clone WK704 (Fig. 17). Since the specific fragment to the wild type allele had disappeared (the part indicated by an arrow), it was confirmed that the clone is a clone in which all FUT8 alleles existing on the genome were disrupted.

4. Removal of the drug-resistant gene from FUT8 gene-double knockout CHO/DG44 cell

(1) Introduction of Cre recombinase expression vector

To the WK704 among the FUT8 double knockout clone prepared in the item 3 of this Example, the Cre recombinase expression vector pBS185 (manufactured by Life Technologies) was introduced.

The gene introduction of the plasmid pBS 185 into each FUT8 double knockout clone was carried out by electroporation [*Cytotechnology,* 3, 133 (1990)] according to the following procedure. First, each FUT8 double knockout clone was suspended in K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) were mixed with 4 μg of the plasmid pBS185, the whole amount of the cell-DNA mixture liquid was transferred into Gene Pulser Cuvette (electrode distance: 2 mm) (manufactured by BIO-RAD) and the gene introduction was carried out at a pulse voltage of 350 V and a capacity of 250 μF using Gene Pulser cell fusion device (manufactured by BIO-RAD). After the introduction, each cell suspension was suspended in 10 ml of IMDM medium (manufactured by Invitrogen) supplemented with 10 % fetal bovine serum (manufactured by Invitrogen) and 1 × concentration HT supplement (manufactured by Invitrogen), and further diluted 20,000-folds with the same culture medium. Each of the diluted solution was

inoculated onto 7 dishes of a 10 cm dish for adhesion cell culture (manufactured by Falcon), and incubated for 10 days at 5 % $CO_2$ and 37°C to form colony.

(2) Preparation of Cre recombinase expression vector introduction clone

Optional clones were collected from the colony obtained by the gene introduction to WK704 according to the following procedure. First, the culture supernatant was removed from the 10 cm dish, 7 ml of a phosphate buffer was injected and then transferred under a stereoscopic microscope. Then, the colony was scratched off and sucked up with Pipetman (manufactured by GILSON) and collected in a round-bottom 96 well plate (manufactured by Falcon). After trypsin treatment, each clone was inoculated onto a flat-bottom 96 well plate for adhesion cell (manufactured by Iwaki Glass) and cultured for 1 week in IMDM medium (manufactured by Invitrogen) supplemented with 10% fetal bovine serum (manufactured by Invitrogen) and 1 × concentration HT supplement (manufactured by Invitrogen).

After culturing, each clone of the above plate was treated with trypsin and mixed with the 2-fold amount of a freezing culture medium (20% DMSO, 40% fetal bovine serum, 40% IMDM). The half amount thereof was inoculated onto a flat bottom 96 well plate for adhesion cell (manufactured by Asahi Technoglass) as a replica plate, on the other hand, the remaining half was submitted to cryopreservation as a master plate.

Then, the replica plate was incubated for 7 days in IMDM medium (manufactured by Invitrogen) supplemented with 600 μg/ml G418 (manufactured by Nacalai Tesque), 15 μg/ml puromycin (manufactured by SIGMA), 10% fetal bovine serum (manufactured by Invitrogen) and 1 × concentration HT supplement (manufactured by Invitrogen). According to the expression of the Cre recombinase, the positive clones from which the drug-resistant gene on both alleles between the loxP sequences had been removed became extinct under the existence of G418 and puromycin. The positive clones were found with this negative selection technique.

(3) Diagnosis of removal of drug-resistant gene by genome Southern blotting

The diagnosis of the removal of the drug-resistant gene of the positive clone (4-5-C3) obtained in the item (2) was carried out by genome Southern blotting according to the following procedure.

Among the master plates stored in a frozen state in the item (2), the 96 well plate containing the above positive clone was selected and allowed to stand for 10 minutes at 5% $CO_2$ and 37°C. After the standing, cells from the wells corresponding to the above clone were inoculated onto a flat bottom 24 well plate for adhesion cell (manufactured by Greiner). After culturing for 1 week in IMDM medium (manufactured by Invitrogen) supplemented with 10% fetal bovine serum (manufactured by Invitrogen) and 1 × concentration HT supplement (manufactured by Invitrogen). Then, they were inoculated onto a flat bottom 6 well plate used for adhesion cell (manufactured by Greiner). From the plate, a genomic DNA of each clone was prepared according to a known method [*Nucleic Acids Research,* 3, 2303, (1976)] and dissolved overnight in 150 μl of TE-RNase buffer solution (pH 8.0) (10 mmol/l Tris-HCl, 1 mmol/l ETDA, 200 μg/ml RNase A).

In 120 μl of NE Buffer 2 (manufactured by New England Biolabs) containing 100 μg/ml BSA (manufactured by New England Biolabs), 12 μg of the above prepared genomic DNA was dissolved, and 20 units of a restriction enzyme *Nhe*I (manufactured by New England Biolabs) were added thereto, followed by digestion overnight at 37°C. From the reaction solution, the DNA fragments were collected by the ethanol precipitation method, dissolved in 20 μl of TE buffer (pH 8.0) (10 mmol/l Tris-HCl, 1 mmol/l EDTA) and subjected to 0.6 % (w/v) agarose gel electrophoresis. After the electrophoresis, the genomic DNA was transferred onto a nylon membrane by a known method [*Proc. Natl. Acad. Sci. USA,* 76, 3683, (1979)]. After the transfer, the nylon membrane was heated at 80°C for 2 hours.

Separately, the probe, which is used for Southern blotting, was prepared as follows. First, PCR was carried out according to the following procedure using the primer (SEQ ID NO:11 or 12) combining with the sequence of the part which exceeded the targeting vector homologous region within the FUT8 genome region. Specifically, 20 μl of a reaction solution [DNA polymerase ExTaq (manufactured by Takara Shuzo), ExTaq buffer (manufactured by Takara Shuzo), 0.2 mmol/l dNTPs, 0.5 μmol/l of the above-described gene specific primer (SEQ ID NOs:1 and 12)] containing 4.0 ng of the plasmid pFUT8fgE2-2 obtained in Reference Example (2) was prepared and the PCR was carried out by heating at 94°C for 1 minute and subsequent 25 cycles of a reaction at 94°C for 30 minutes, 55°C for 30 minutes and 74°C for 1 minute as one cycle. After the PCR, the reaction solution was subjected to 1.75 % (w/v) agarose gel electrophoresis and a probe DNA fragment of about 230 bp was purified. To 5 μl of the probe DNA solution, the radiation labeling was carried out by using [$\alpha$-$^{32}$P]dCTP 1.75 MBq and Megaprime DNA Labeling system, dCTP (manufactured by Amersham Pharmacia Biotech).

Hybridization was carried out as follows. First, the above described nylon membrane was encapsulated into a roller bottle, 15 ml of the hybridization solution [5 × SSPE, 50 × Denhalt's solution, 0.5 % (w/v) SDS, 100 μg/ml salmon sperm DNA] was added and the hybridization was carried out at 65°C for 3 hours. Then, the probe DNA labeled with $^{32}$P was heat degenerated and poured into a bottle and heated overnight at 65°C.

After the hybridization, the nylon membrane was soaked into 50 ml of 2×SSC-0.1% (w/v) SDS and heated at 65°C for 15 minutes. After the above-described washing operation was repeated twice, the membrane was soaked into 50 ml of 0.2 × SSC-0.1 % (w/v) SDS and heated at 65°C for 15 minutes. After the washing, the nylon membrane was developed by exposing it to an X-ray film at -80°C.
By the above-described treatment with a restriction enzyme *Nhe*I, a DNA fragment of about 8.0 Kb was obtained from the wild type FUT8 allele. Furthermore, by the same restriction enzyme treatment, a DNA fragment of about 9.5 Kb was obtained from the alleles in which a homologous recombination with the targeting vector had occurred. Moreover, when the neomycin resistant gene (about 1.6 kb) or puromycin resistant gene (about 1.5 kb) was removed from the alleles in which a homologous recombination had occurred, a DNA fragment of about 8.0 Kb was obtained by the same treatment.
By the present method, only fragment of about 8.0 Kb specific for the homologous recombination region removed drug-resistant gene was found from the genomic DNA of the tested positive clone 4-5-C3 (positive clone in the item 4(3) of Example 5) (Fig. 18).

Example 6

Expression of antibody molecule in CHO/DG44 cell in which FUT8 allele was double knocked out:

1. Preparation of anti-CD20 chimeric antibody expression vector

(1) Establishment of cDNA encoding VL of anti-CD20 mouse monoclonal antibody

The cDNA (SEQ ID NO:17) encoding the amino acid sequence of VL in anti-CD20 mouse monoclonal antibody 2B8 described in WO94/11026 was constructed using PCR as follows.
First, the binding nucleotide sequence (including a restriction enzyme recognizing site for cloning to a vector for humanized antibody expression) of the amplification primer for PCR was added to the 5'-terminal and 3'-terminal of the nucleotide sequence of VL described in WO94/11026. The designed nucleotide sequence was divided into total 6 nucleotide sequences from the 5'-terminal side with about 100 bases each (the adjacent nucleotide sequences were adjusted in such a manner that their ends have a common sequence of about 20 bases at their termini) and 6 synthetic DNA of SEQ ID NOs:19, 20, 21, 22, 23 and 24 were prepared (consignment to GENSET company) in alternate order of a sense chain and an antisense chain.
Each oligonucleotide was added to 50 μL of a reaction solution [KOD DNA Polymerase affixture PCR Buffer #1 (manufactured by Toyobo), 0.2 mM dNTPs, 1 mM magnesium chloride, 0.5 μM M13 primer M4 (manufactured by Takara Shuzo), 0.5μM M13 primer RV (manufactured by Takara Shuzo)] to give a final concentration of 0.1 μM. Using a DNA thermal cycler GeneAmp PCR System 9600 (manufactured by Perkin Elmer), the reaction solution was heated at 94°C for 3 minutes, subsequent 25 cycles of heating at 94°C for 30 seconds, 55°C for 30 seconds and 74°C for 1 minute as one cycle after 2.5 units of KOD DNA Polymerase (manufactured by Toyobo) were added, and further heated at 72°C for 10 minutes. Then, 25 μL of the reaction solution was subjected to agarose gel electrophoresis by using a QIAquick Gel Extraction Kit (manufactured by QIAGEN) to collect a PCR product of about 0.44 kb of VL.
Then, 0.1 μg of DNA obtained from plasmid pBluescriptII SK(-) (manufactured by Stratagene) by a restriction enzyme *Sma*I (manufactured by Takara Shuzo) and about 0.1 μg of the PCR product obtained above were added to sterilized water to give a total volume of 7.5 μL, and 7.5 μL of solution I of a TAKARA ligation kit ver. 2 (manufactured by Takara Shuzo) and 0.3 μL of a restriction enzyme *Sma*I (manufactured by Takara Shuzo) were added thereto, followed by reaction at 22°C for 2 hours. Using the thus obtained recombinant plasmid DNA solution, *E. coli* DH5α strain (manufactured by Toyobo) was transformed. From the clones of the transformant, each plasmid DNA was prepared and was allowed to react by using a BigDye Terminator Cycle Sequencing Ready Reaction Kit v2.0 (manufactured by Applied Biosystems) according to the attached manufacture's instruction, and then the nucleotide sequence was analyzed by a DNA sequencer ABI PRISM 377 of the same company. Thus, a plasmid pBS-2B8L having the nucleotide sequence of interest shown in Fig. 19 was obtained.

(2) Construction of cDNA coding VH of anti-CD20 mouse monoclonal antibody

cDNA (SEQ ID NO:18) encoding the amino acid sequence of VH of anti-CD20 mouse monoclonal antibody 2B8 described in WO94/11026 was constructed using PCR as follows.
First, the binding nucleotide sequence (including a restriction enzyme recognition sequence for cloning to a vector for humanized antibody expression) of the amplification primer for PCR was added to the 5'-terminal and 3'-terminal of the nucleotide sequence of VH described in WO94/11026. The designed nucleotide sequence was divided into total 6 nucleotide sequences from the 5'-terminal side with about 100 bases each (the adjacent nucleotide sequences were adjusted in such a manner that their ends have a common sequence of about 20 bases at their termini) and 6 synthetic DNA of SEQ ID NOs:25, 26, 27, 28, 29 and 30 were prepared (consignment to GENSET company) in alternate order of a sense chain and an antisense chain.

Each oligonucleotide was added to 50 µL of the reaction solution [KOD DNA Polymerase affixture PCR Buffer #1 (manufactured by Toyobo), 0.2 mM dNTPs, 1 mM magnesium chloride, 0.5 µM M13 primer M4 (manufactured by Takara Shuzo), 0.5 µM M13 primer RV (manufactured by Takara Shuzo)] to give a final concentration of 0.1 µM, and, using a DNA thermal cycler GeneAmp PCR System 9600 (manufactured by Perkin Elmer), heated at 94°C for 3 minutes, subsequent 25 cycles of heating at 94°C for 30 seconds, 55°C for 30 seconds and 74°C for 1 minute as one cycle after 2.5 units KOD DNA Polymerase (manufactured by Toyobo) was added thereto, and further heated at 72°C for 10 minutes. After 25 µL of the reaction solution was subjected to agarose gel electrophoresis using a QIAquick Gel Extraction Kit (manufactured by QIAGEN) to collect a PCR product of about 0.49 kb ofVH.

Then, 0.1 µg of the DNA obtained from the plasmid pBluescriptII SK(-) (manufactured by Stratagene) by a restriction enzyme *Sma*I (manufactured by Takara Shuzo) and about 0.1 µg of the PCR product obtained as described above were added to sterilized water to give a total volume of 7.5 µL, and 7.5 µL solution I of a TAKARA ligation kit ver. 2 (manufactured by Takara Shuzo Co) and 0.3 µL of a restriction enzyme *Sma*I (manufactured by Takara Shuzo) were added thereto, followed by reaction overnight at 22°C.

Using the thus obtained recombinant plasmid DNA solution, *E. coli* DH5α strain (manufactured by Toyobo) was transformed. Each plasmid DNA from the clones of the transformant was prepared and was allowed to react by using a BigDye Terminator Cycle Sequencing Ready Reaction Kit v2.0 (manufactured by Applied Biosystems) according to the attached manufacture's instruction, and then the nucleotide sequence was analyzed by a DNA sequencer ABI PRISM 377 of the same company. Thus, a plasmid pBS-2B8H having the nucleotide sequence of interest shown in Fig. 20 was obtained.

Then, the synthetic DNA represented by SEQ ID NO:31 was designed in order to substitute the amino acid residue at position 14 from Ala to Pro, and the substitution was carried out as follows by PCR using a LA PCR *in vitro* Mutagenesis Primer Set for pBluescriptII (manufactured by Takara Shuzo). After 50 µL of a reaction solution [LA PCR Buffer II (manufactured by Takara Shuzo), 2.5 units TAKARA LA Taq, 0.4 mM dNTPs, 2.5 mM magnesium chloride, 50 nM T3 BcaBEST Sequencing primer (manufactured by Takara Shuzo), 50 nM of the above-described primer for introducing mutation (SEQ ID NO:31, produced by GENSET)] containing 1 ng of the above-described plasmid pBS-2B8H was prepared and, using a DNA thermal cycler GeneAmp PCR System 9600 (manufactured by Perkin Elmer), the reaction solution was allowed to react by 25 cycles of heating at 94°C for 30 seconds, 55°C for 2 minutes and 72°C for one and a half minute as one cycle. Then, 30 µL of the reaction solution was subjected to agarose gel electrophoresis, and a PCR product of about 0.44 kb was collected using a QIAquick Gel Extraction Kit (manufactured by QIAGEN) and made to a 30 µL aqueous solution. Furthermore, PCR was carried out in the same manner using 50 µL of a reaction solution [LA PCR Buffer II (manufactured by Takara Shuzo Co., Ltd.), 2.5 units of TAKARA LA Taq, 0.4 mM dNTPs, 2.5 mM magnesium chloride, 50 nM T7 BcaBEST Sequencing primer (manufactured by Takara Shuzo Co., Ltd.), 50 nM MUT B1 primer (manufactured by Takara Shuzo Co., Ltd.)] containing 1 ng of the above-described plasmid pBS-2B8H. Then, 30 µL of the reaction solution was subjected to agarose gel electrophoresis, and a PCR product of about 0.63 kb was collected using a QIAquick Gel Extraction Kit (manufactured by QIAGEN) and made to a 30 µL aqueous solution. Subsequently, 0.5 µL of the above obtained PCR product of about 0.44 kb and the PCR product of about 0.63 kb, respectively, were added to 47.5 µL of a reaction solution [LA PCR Buffer II (manufactured by Takara Shuzo), 0.4 mM dNTPs, 2.5 mM magnesium chloride] and, using a DNA thermal cycler GeneAmp PCR System 9600 (manufactured by Perkin Elmer), the reaction solution was allowed to react by heating at 90°C for 10 minutes, subsequent cooling to 37°C over 60 minutes, and then maintaining the temperature at 37°C for 15 minutes to thereby carry out annealing of DNA. After 2.5 units of TAKARA LA Taq (manufactured by Takara Shuzo) were added and allowed to react at 72°C for 3 minutes, 10 pmol of T3 BcaBEST Sequencing primer (manufactured by Takara Shuzo) and T7 BcaBEST Sequencing primer (manufactured by Takara Shuzo), respectively, were added, and the reaction solution was made to 50 µL and allowed to react by 10 cycles of heating at 94°C for 30 seconds, 55°C for 2 minutes and 72°C for one and a half minute as one cycle. Then, 25 µL of the reaction solution was purified with QIA quick PCR purification kit (manufactured by QIAGEN) and half the amount was allowed to react at 37°C for 1 hour using 10 units of a restriction enzyme *Kpn*I (manufactured by Takara Shuzo Co., Ltd.) and 10 units of a restriction enzyme *Sac*I (manufactured by Takara Shuzo Co., Ltd.). The reaction solution was fractionated by agarose gel electrophoresis and a *Kpn*I-*Sac*I fragment of about 0.59 kb was collected.

Next, 1 µg of pBluescriptII SK(-) (manufactured by Stratagene) was allowed to reacted at 37°C for 1 hour using 10 units of a restriction enzyme *Kpn*I (manufactured by Takara Shuzo) and 10 units of a restriction enzyme *Sac*I (manufactured by Takara Shuzo), and then, the reaction solution was fractionated by agarose gel electrophoresis to collect a *Kpn*I-*Sac*I fragment of about 2.9 kb.

The *Kpn*I-*Sac*I fragment derived from the PCR product obtained as described above and the *Kpn*I-*Sac*I fragment derived from the plasmid pBluescriptII SK(-) were ligated by using solution I of DNA Ligation Kit Ver.2 (manufactured by Takara Shuzo) according to the attached manufacture's instruction. Using the thus obtained recombinant plasmid DNA solution, *E. coli* DH5α strain (manufactured by Toyobo) were transformed. Each plasmid DNA was prepared from the clones of the transformant and allowed to react by using a BigDye Terminator Cycle Sequencing Ready Reaction Kit v2.0 (manufactured by Applied Biosystems) according to the attached manufacture's instruction, and then, the nucleotide sequence was analyzed with a DNA sequencer ABI PRISM 377 of the same company.

Thus, a plasmid pBS-2B8Hm having the nucleotide sequence of interest shown in Fig. 20 was obtained.

(3) Construction of expression vector of anti-CD20 human chimeric antibody.

Using vector pKANTEX93 for humanized antibody expression [*Mol. Immunol.,* 37, 1035 (2000)] and plasmid pBS-2B8L and pBS-2B8Hm obtained in the items (1) and (2), the expression vector pKANTEX2B8P of the anti-CD20 human chimeric antibody (hereinafter referred to as "anti-CD20 chimeric antibody") was constructed as follows.

After 2 μg of the plasmid pBS-2B8L obtained in the item (1) was allowed to react at 55°C for 1 hour using 10 units of a restriction enzyme *Bsi*WI (manufactured by New England Biolabs), further reaction was carried out at 37°C for 1 hour using 10 units of a restriction enzyme *Eco*RI (manufactured by Takara Shuzo). The reaction solution was fractionated by agarose gel electrophoresis to collect a *Bsi*WI-*Eco*RI fragment of about 0.41 kb.

Then, 2 μg of the vector pKANTEX93 for humanized antibody expression was allowed to react at 55°C for 1 hour using 10 units of a restriction enzyme *Bsi*WI (manufactured by New England Biolabs), and then further reaction was carried out at 37°C for 1 hour using 10 units of a restriction enzyme *Eco*RI (manufactured by Takara Shuzo). The reaction solution was fractionated by agarose gel electrophoresis to collect a *Bsi*WI-*Eco*RI fragment of about 12.75 kb.

Next, the above obtained *Bsi*WI-*Eco*RI fragment derived from plasmid pBS-2B8L and the *Bsi*WI-*Eco*RI fragment derived from the plasmid pKANTEX93 were ligated by using solution I of DNA Ligation Kit Ver.2 (manufactured by Takara Shuzo) according to the attached manufacture's instruction. Using the thus obtained recombinant plasmid DNA solution, *E. coli* DH5α strain (manufactured by Toyobo) was transformed to obtain a plasmid pKANTEX2B8-L shown in Fig. 21.

Then, 2 μg of the plasmid pBS-2B8Hm obtained in the item (2) was allowed to react at 37°C for 1 hour by using 10 units of a restriction enzyme *Apa*I (manufactured by Takara Shuzo Co., Ltd.), and then, further reaction was carried out at 37°C for 1 hour by using 10 units of a restriction enzyme *Not*I (manufactured by Takara Shuzo Co., Ltd.). The reaction solution was fractionated by agarose gel electrophoresis to collect an *Apa*I-*Not*I fragment of about 0.45 kb.

Next, 3 μg of the plasmid pKANTEX2B8-L was allowed to react at 37°C for 1 hour by using 10 units of a restriction enzyme *Apa*I (manufactured by Takara Shuzo Co., Ltd.), and then further reaction was carried out at 37°C for 1 hour by using 10 units of a restriction enzyme *Not*I (manufactured by Takara Shuzo Co., Ltd.). The reaction solution was fractionated by agarose gel electrophoresis to collect an *Apa*I-*Not*I fragment of about 13.16 kb.

Then, the above obtained *Apa*I-*Not*I fragment derived from plasmid pBS-2B8Hm and the *Apa*I-*Not*I fragment derived from plasmid pKANTEX2B8-L were ligated by using solution I of DNA Ligation Kit Ver.2 (manufactured by Takara Shuzo) according to the attached manufacture's instruction. Using the recombinant plasmid DNA solution obtained in this manner, *E*. DH5α strain (manufactured by Toyobo) was transformed and each plasmid DNA was prepared from the clone of the transformant.

Using the obtained plasmid, the nucleotide sequence was analyzed by using a BigDye Terminator Cycle Sequencing Ready Reaction Kit v2.0 (manufactured by Applied Biosystems) with DNA sequencer 377 of the same company. As a result, it was confirmed that a plasmid pKANTEX2B8P cloned with the DNA of interest shown in Fig. 21 was obtained.

2. Expression of anti-CD20 chimeric antibody

The expression vector pKANTEX2B8P of the anti-CD20 antibody obtained in the item 1 of this Example was introduced into the FUT8 gene double knockout clone WK704 prepared in the item 3 of Example 5.

The gene introduction into WK704 of the plasmid pKANTEX2B8P was carried out by electroporation [*Cytotechnology,* 3*,* 133 (1990)] by the procedure as follows. First, 10 μg of the plasmid pKANTEX2B8P was dissolved in 100 μl of NEBuffer 4 (manufactured by New England Biolabs), and 40 units of a restriction enzyme *Aat*II (manufactured by New England Biolabs) were added thereto, then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended into K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a concentration of $8\times10^7$ cells/ml. After 200 μl of the cell suspension ($1.6\times10^6$ cells) were combined with 4 μl (4 μg) of the above-described linearized plasmid, the total cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene intro-

duction, the cell suspension was suspended in IMDM medium (manufactured by Invitrogen) supplemented with 10 % fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml IMDM medium (manufactured by Invitrogen) supplemented with 10% fetal bovine dialysis serum (manufactured by Invitrogen) were filled therein. The culturing was carried out for 15 days while the medium exchange process was repeated every 3 to 4 days, and a transformant WK704-2B8P was obtained. Furthermore, the clone WK704-2B8P, as a name of WK704-2B8P, has been deposited on March 20, 2003, as FERM BP-8337 in International Patent Organism Depositary, National Institute of Advanced Industrial Science and Technology (Tsukuba Central 6, 1, Higashi 1-Chome Tsukuba-shi, Ibaraki-ken 305-8566 Japan).

3. Expression of anti-ganglioside GD3 chimeric antibody

The vector plasmid pKANTEX641 for expression of the anti-ganglioside GD3 chimeric antibody was introduced into the FUT8 gene double knockout clone WK704 prepared in the item 3 of Example 5 and a stable expression clone of anti-GD3 chimeric antibody was prepared. The pKANTEX641 is a derivative comprising the vector plasmid pChi641LHGM4 for expression of the anti-GD3 chimeric antibody described in WO00/61739 and the vector pKANTEX93 for expression of humanized antibody [*Mol. Immunol.,* 37, 1035 (2000)] in which an *Eco*RI-*Hind*III fragment containing a tandem antibody expression unit obtained from pChi641 LHGM4 is ligated with an *Eco*RI-*Hind*III fragment containing the origin of replication obtained from pKANTEX93.

The gene introduction into WK704 of the plasmid pKANTEX641 was carried out by electroporation [*Cytotechnology,* 3, 133 (1990)] by the procedure as follows. First, 10 μg of the plasmid pKANTEX641 was dissolved in 100 μl of NEBuffer 4 (manufactured by New England Biolabs), and 40 units of a restriction enzyme *Aat*II (manufactured by New England Biolabs) were added thereto, and then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended into K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) was mixed with 4 μl (4 μg) of the above-described linearized plasmid, the whole cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene introduction, the cell suspension was suspended in IMDM medium (manufactured by Invitrogen) supplemented with 10 % fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml of IMDM medium (manufactured by Invitrogen) supplemented with 10% fetal bovine dialysis serum (manufactured by Invitrogen) were filled therein. The culturing was carried out for 15 days while the medium exchange process was repeated every 3 to 4 days, and a transformant WK704-2871 was obtained. Furthermore, the clone WK704-2871, as a name of WK704-2871, has been deposited on March 20, 2003, as FERM BP-8336 in International Patent Organism Depositary, National Institute of Advanced Industrial Science and Technology (Tsukuba Central 6, 1, Higashi 1-Chome Tsukuba-shi, Ibaraki-ken 305-8566 Japan).

4. Expression of the anti-CCR4 chimeric antibody

The vector pKANTEX2160 for expression of anti-CCR4 chimeric antibody described in W001/64754 was introduced into the FUT8 gene double knockout clone WK704 prepared in the item 3 of Example 5 and a stable expression clone of the anti CCR4 chimeric antibody was prepared.

The gene introduction into the WK704 of the plasmid pKANTEX2160 was carried out the electroporation technique [*Cytotechnology,* 3, 133 (1990)] by the procedure as follows. First, 15 μg of the plasmid pKANTEX2160 were dissolved in 100 μl of NEBuffer 4 (manufactured by New England Biolabs), 40 units of a restriction enzyme *Aat*II (manufactured by New England Biolabs) were added thereto, and then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended in K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) was combined with 4 μl (4 μg) of the above-described linearized plasmid, the whole cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene introduction, the cell suspension was suspended in IMDM medium (manufactured by Invitrogen) supplemented with 10% fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml IMDM medium (manufactured by Invitrogen) supplemented with 10 % fetal bovine dialysis serum (manufactured by Invitrogen) were filled therein. The culturing was carried out for 15 days while this medium exchange process was repeated every 3 to 4 days, and a transformant WK704-2760 was obtained. Furthermore, the clone WK704-2760, as a name of WK704-2760, has been deposited on March 20, 2003, as FERM BP-8335 in International Patent Organism Depositary, National Institute of Advanced Industrial Science and Technology (Tsukuba Central 6, 1, Higashi 1-Chome Tsukuba-shi, Ibaraki-ken 305-8566 Japan).

5. Purification of antibody molecule

The clone WK704-2B8P for expression of anti-CD20 antibody obtained in the item 2 of this Example was suspended in IMDM medium (manufactured by Invitrogen) supplemented with 10% fetal bovine dialysis serum (manufactured by Invitrogen) to give a density of $3\times10^5$ cells/ml and a total volume of 300 ml was inoculated onto 10 bottles of T182 flasks for adhesion cell culture (manufactured by Greiner). The clone WK704-2871 for expression of anti-GD3 antibody obtained in the item 3 of this Example and the clone WK704-2760 for expression of anti-CCR4 antibody obtained in the item 4 of this Example were inoculated in the same manner. After culturing for 3 days, all culture supernatants of each clone were removed and exchanged to EXCELL301 medium (manufactured by JRH Biosciences). They were cultured for 7 days at 37°C in a 5% $CO_2$ incubator, and then each cell suspension was collected. Each of all collected cell suspensions was centrifuged for 10 minutes at 3000 rpm and 4°C to recover a supernatant, and then the supernatant was filtered with a PES membrane having a pore size of 0.22 µm and a volume of 500 ml (manufactured by Asahi Technoglass).

In a column having a diameter of 0.8 cm, 0.5 ml of Mab Select (manufactured by Amersham Pharmacia Biotech) was packed and then 3.0 ml purified water and 3.0 ml of 0.2 mol/l boric acid - 0.15 mol/l NaCl buffer (pH 7.5) were filled into the tube successively. Furthermore, the carrier was equilibrated by successive cleaning with 2.0 ml of 0.1 mol/l citrate buffer (pH 3.5) and 1.5 ml of 0.2 mol/l boric acid - 0.15 mol/l NaCl buffer (pH 7.5). Then, after 300 ml of the above-described supernatant was packed into the column, it was washed with 3.0 ml of 0.2 mol/l boric acid - 0.15 mol/l NaCl buffer (pH 7.5). After the washing, the antibody absorbed on the carrier was eluted by using 1.25 ml of 0.1 mol/l citrate buffer (pH 3.5). After 250 µl of the first eluted fraction was disposed, 1 ml of the next eluted fraction was recovered and neutralized by mixing with 200 µl of 2 mol/l Tris-HCl (pH 8.5). The obtained eluted solution was dialyzed overnight at 4°C by using 10 mol/l citric acid - 0.15 mol/l NaCl buffer (pH 6.0). After the dialysis, the antibody solution was recovered and was sterilized and filtered by using Millex GV having a pore size of 0.22 µm (manufactured by MILLIPORE).

Example 7

*In vitro* cytotoxic activity (ADCC activity) of antibody composition produced by CHO/DG44 cell in which FUT8 allele was double knocked out:

In order to evaluate the *in vitro* cytotoxic activity of the anti-CD20 antibody purified in Example 6, the ADCC activity was measured as follows.

(1) Preparation of target cell suspension

A human B lymphocyte cultured cell line Raji cell (JCRB9012) cultured in RPMI1640-FCS(10) medium [PRMI1640 medium (manufactured by GIBCO BRL) supplemented with 10% FCS] was washed with RPMI1640-FCS (5) medium [PRMI1640 medium (manufactured by GIBCO BRL) supplemented with 5 % FCS] by centrifuge separation and suspension. Then, the suspension was prepared with RPMI1640-FCS(5) medium to give a density of $2\times10^6$ cells/ml as the target cell suspension.

(2) Preparation of effector cell suspension

After 50 ml venous blood of a healthy individual was collected, 0.5 ml of heparin sodium (manufactured by Shimizu Seiyaku) was added thereto, followed by mixing gently. The mixture was centrifuged (800 g, 20 minutes) with Lymphoprep (manufactured by AXIS SHIELD) according to the manufacture's instruction to separate a mononuclear cell phase. The cells were washed 3 times with RPMI1640-FCS(5) medium by centrifugal separation and re-suspended to give a density of $4\times10^6$ cells/ml by using the same medium, and the resulting suspension was used as effector cell suspension.

(3) Measurement of the ADCC activity

To each well of a 96 well U-shape bottom plate (manufactured by Falcon), 50 μl ($1\times10^4$ cells/well) of the target cell suspension prepared in the above (1) was dispensed. Then, 50 μl ($2\times10^5$ cells/well, the ratio of the effector cells and target cells becomes 20 : 1) of the effector cell suspension prepared in the above (2) was added. Moreover, various anti CD20 chimeric antibodies were added to give a final concentration of 0.3 to 3000 ng/ml and a total volume of 150 μl, and the reaction was carried out at 37°C for 4 hours. After the reaction, the plates were centrifuged and the lactate dehydrogenase (LDH) activity in the supernatant was measured by CytoTox96 Non-Radioactive Cytotoxicity Assay (manufactured by Promega). The spontaneously released LDH amount of the target cells was calculated by carrying out the same procedure as described above, except for using only medium instead of the effector cell suspension and the antibody solution and measuring the LDH activity in the supernatant. The absorbance data of the spontaneously release of the effector cells was obtained by carrying out the same procedure as described above, except for using only medium instead of the effector cell suspension and the antibody solution. The total free LDH amount involved in all targeted cytoclasis was calculated by the measurement of the LDH activity in the supernatant, conducting the same procedure as described above, except for using only medium instead of the effector cell suspension and the antibody solution, and adding 15 μl of 9% Triton X-100 solution 45 minutes before the end of the reaction. The ADCC activity was calculated according to the following formula (II) by using these values.

$$\text{ADCC activity (\%)} = \frac{\begin{pmatrix}\text{LDH amount in}\\\text{sample supernatant}\end{pmatrix} - \begin{pmatrix}\text{spontaneously released}\\\text{LDH amount}\end{pmatrix}}{\begin{pmatrix}\text{total released}\\\text{LDH amount}\end{pmatrix} - \begin{pmatrix}\text{spontaneously released}\\\text{LDH amount}\end{pmatrix}} \times 100 \quad \text{(II)}$$

The ADCC activity of each anti-CD20 antibody is shown in Fig. 22. The antibodies obtained from FUT8 gene double knockout clone WK704-2B8P showed a higher ADCC activity than commercially available Rituxan™ in all antibody concentrations and the maximum cytotoxic activity value was also higher. Rituxan™ is an anti-CD20 chimeric antibody produced by using CHO cell, as the host cell, in which the FUT8 gene was not disrupted. Furthermore, as a result that the ADCC activity of each of the antibodies obtained from the FUT8 gene double knockout clone WK704-2871 and the clone WK704-2760 was measured, it was shown that a higher cytotoxic activity than an antibody produced by a usual CHO cell line in which FUT8 gene was not disrupted was obtained in the same manner as in the case of the anti-CD20 antibody. Based on the above results, it was found that an antibody having a higher cytotoxic activity can be prepared by using a host cell in which the FUT8 allele was disrupted, in comparison with the case of using host cells in which FUT8 gene was not disrupted.

Example 8

Sugar chain analysis of antibody composition produced by CHO/DG44 cell in which FUT8 allele is double knocked out:

The sugar chain analysis of the anti-CD20 antibody, anti-GD3 antibody and anti-CCR4 antibody produced by the FUT8 gene double knockout clone obtained in Example 6 was carried out according to the method described in the item (4) of Example 2. Furthermore, regarding the sugar chain composition of the antibodies, a monosaccharide composition analysis was carried out according to a known method *[*Journal of Liquid Chromatography, 6*,* 1577, (1983)]. As the result, a sugar chain structure containing fucose was not found in any of the antibodies obtained from FUT8 gene double knockout clone WK704. Based on the above result, it was clarified that the function in which 1-position of fucose is bound to 6-position of *N*-acetylglucosamine in the reducing end through α-bond in a complex *N*-glycoside-linked sugar chain can be completely deleted by disruption of the FUT8 allele in the host cell.

Reference Example

Preparation of CHO cell FUT8 gene:

(1) Preparation of CHO cell FUT8 cDNA sequence

From a single-stranded cDNA prepared from CHO/DG44 cells on the 2nd day of culturing in the item (1) of Example 8 of WO 00/61739, Chinese hamster FUT8 cDNA was obtained by the following procedure (Fig. 23).

First, a forward primer specific for a 5'-terminal untranslated region (represented by SEQ ID NO:7) and a

reverse primer specific for a 3'-terminal untranslated region (represented by SEQ ID NO:8) were designed from a mouse FUT8 cDNA sequence (GenBank, AB025198).

Next, 25 μl of a reaction solution [ExTaq buffer (manufactured by Takara Shuzo), 0.2 mmol/l dNTPs, 4% DMSO and 0.5 μmol/l specific primers (SEQ ID NOs:7 and 8)] containing l μl of the CHO/DG44 cell-derived cDNA was prepared and PCR was carried out by using a DNA polymerase ExTaq (manufactured by Takara Shuzo). The PCR was carried out by heating at 94°C for 1 minute, subsequent 30 cycles of a reaction at 94°C for 30 seconds, at 55°C for 30 seconds and at 72°C for 2 minutes as one cycle, and further heating at 72°C for 10 minutes.

After the PCR, the reaction solution was subjected to 0.8% agarose gel electrophoresis, and a specific amplified fragment of about 2 Kb was purified. Into a plasmid pCR2.1, 4 μl of the DNA fragment was employed to insert in accordance with the instructions attached to TOPO TA Cloning Kit (manufactured by Invitrogen), and *E. coli* DH5α strain was transformed with the reaction solution. Plasmid DNAs were isolated in accordance with a known method from cDNA-inserted 8 clones among the obtained kanamycin-resistant colonies.

The nucleotide sequence of each cDNA inserted into the plasmid was determined using DNA Sequencer 377 (manufactured by Parkin Elmer) and BigDye Terminator Cycle Sequencing FS Ready Reaction Kit (manufactured by Parkin Elmer) in accordance with the method of the manufacture's instructions. It was confirmed by the method that all of the inserted cDNAs encode a sequence containing the full ORF of CHO cell FUT8. Among these, a plasmid DNA containing absolutely no reading error of bases by the PCR in the sequences was selected. Herein, the plasmid is referred to as CHfFUT8-pCR2.1. The determined nucleotide sequence and the amino acid sequence of the cDNA of CHO FUT8 are represented by SEQ ID NOs:1 and 4, respectively.

(2) Preparation of CHO cell FUT8 genome sequence

Using the ORF full length cDNA fragment of CHO cell FUT8 obtained in the item (1) as a probe, a CHO cell FUT8 genome clone was obtained in accordance with a known genome screening method described, e.g., in *Molecular Cloning, Second Edition, Current Protocols in Molecular Biology, A Laboratory Manual*, Second Edition (1989). Next, after digesting the obtained genome clone using various restriction enzymes, the Southern hybridization was carried out by using an *Afa*I-*Sau*3AI fragment (about 280 bp) containing initiation codon of the CHO cell FUT8 cDNA as a probe, and then a *Xba*I-*Xba*I fragment (about 2.5 Kb) and a *Sac*I-*Sac*I fragment (about 6.5 Kb) were selected from restriction enzyme fragments showing positive reaction, inserted into pBluescript II KS(+) (manufactured by Stratagene), respectively.

The nucleotide sequence of each of the obtained genome fragments was determined using DNA Sequencer 377 (manufactured by Parkin Elmer) and BigDye Terminator Cycle Sequencing FS Ready Reaction Kit (manufactured by Parkin Elmer) in accordance with the method of the manufacture's instructions. Thereby, it was confirmed that the *Xba*I-*Xba*I fragment encodes a sequence of an upstream intron of about 2.5 Kb containing exon 2 of the CHO cell FUT8, and the *Sac*I-*Sac*I fragment encodes a sequence of a downstream intron of about 6.5 Kb containing exon 2 of the CHO cell FUT8. Herein, the plasmid containing *Xba*I-*Xba*I fragment is referred to as pFUT8fgE2-2, and the plasmid containing *Sac*I-*Sac*I fragment is referred to as pFUT8fgE2-4. The determined nucleotide sequence (about 9.0 Kb) of the genome region containing exon 2 of the CHO cell FUT8 is represented by SEQ ID NO:3.

INDUSTRIAL APPLICABILITY

The present invention provides a cell in which genome is modified so as to have a more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell, a production process of an antibody using the cell, a tnrasgenic non-human animal or plant prepared by using the cell, an antibody composition produced by the production process, and a medicament comprising the antibody composition.

Free Text in Sequence Listing

SEQ ID NO:7 - Explanation of synthetic sequence: synthetic DNA
SEQ ID NO:8 - Explanation of synthetic sequence: synthetic DNA
SEQ ID NO:9 - Explanation of synthetic sequence: synthetic DNA
SEQ ID NO:10 - Explanation of synthetic sequence: synthetic DNA
SEQ ID NO:11 - Explanation of synthetic sequence: synthetic DNA
SEQ ID NO:12 - Explanation of synthetic sequence: synthetic DNA

SEQ ID NO:13 - Explanation of synthetic sequence: synthetic DNA
SEQ ID NO:14 - Explanation of synthetic sequence: synthetic DNA
SEQ ID NO:15 - Explanation of synthetic sequence: synthetic DNA
SEQ ID NO:16 - Explanation of synthetic sequence: synthetic DNA
SEQ ID NO:19 - Explanation of synthetic sequence: synthetic DNA
SEQ ID NO:20 - Explanation of synthetic sequence: synthetic DNA
SEQ ID NO:21 - Explanation of synthetic sequence: synthetic DNA
SEQ ID NO:22 - Explanation of synthetic sequence: synthetic DNA
SEQ ID NO:23 - Explanation of synthetic sequence: synthetic DNA
SEQ ID NO:24 - Explanation of synthetic sequence: synthetic DNA
SEQ ID NO:25 - Explanation of synthetic sequence: synthetic DNA
SEQ ID NO:26 - Explanation of synthetic sequence: synthetic DNA
SEQ ID NO:27 - Explanation of synthetic sequence: synthetic DNA
SEQ ID NO:28 - Explanation of synthetic sequence: synthetic DNA
SEQ ID NO:29 - Explanation of synthetic sequence: synthetic DNA
SEQ ID NO:30 - Explanation of synthetic sequence: synthetic DNA
SEQ ID NO:31 - Explanation of synthetic sequence: synthetic DNA

## Claims

**1.** A cell in which genome is modified so as to have a more decreased or deleted activity of an enzyme relating to modification of a sugar chain in which 1-position of fucose is bound to 6-position of *N*-acetylglucosamine in the reducing end through α-bond in a complex *N*-glycoside-linked sugar chain than its parent cell.

**2.** The cell according to claim 1, wherein a genomic gene encoding an enzyme relating to modification of a sugar chain in which 1-position of fucose is bound to 6-position of *N*-acetylglucosamine in the reducing end through α-bond in a complex *N*-glycoside-linked sugar chain is knocked out.

**3.** The cell according to claim 1 or 2, wherein all of alleles on a genome encoding an enzyme relating to modification of a sugar chain in which 1-position of fucose is bound to 6-position of *N*-acetylglucosamine in the reducing end through α-bond in a complex *N*-glycoside-linked sugar chain are knocked out.

**4.** The cell according to any one of claims 1 to 3, wherein the enzyme relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

**5.** The cell according to claim 4, wherein the α1,6-fucosyltransferase is a protein encoded by a DNA selected from the group consisting of the following (a) to (d):

(a) a DNA comprising the nucleotide sequence represented by SEQ ID NO:1;
(b) a DNA comprising the nucleotide sequence represented by SEQ ID NO:2;
(c) a DNA which hybridizes with a DNA comprising the nucleotide sequence represented by SEQ ID NO:1 under stringent conditions and having an α1,6-fucosyltransferase activity;
(d) a DNA which hybridizes with a DNA comprising the nucleotide sequence represented by SEQ ID NO:2 under stringent conditions and having an α1,6-fucosyltransferase activity.

**6.** The cell according to claim 4, wherein the α1,6-fucosyltransferase is a protein selected from the group consisting of the following (a), (b), (c), (d), (e) and (f):

(a) a protein comprising the amino acid sequence represented by SEQ ID NO:4;
(b) a protein comprising the amino acid sequence represented by SEQ ID NO:5;
(c) a protein which comprises an amino acid sequence in which at least one amino acid is deleted, substituted, inserted and/or added in the amino acid sequence represented by SEQ ID NO:4 and has an α1,6-fucosyltransferase activity;
(d) a protein which comprises an amino acid sequence in which at least one amino acid is deleted, substituted, inserted and/or added in the amino acid sequence represented by SEQ ID NO:5 and has an α1,6-fucosyltransferase activity;
(e) a protein which comprises an amino acid sequence having a homology of 80% or more with the amino acid sequence represented by SEQ ID NO:4 and has an α1,6-fucosyltransferase activity;
(f) a protein which comprises an amino acid sequence having a homology of 80% or more with the amino acid sequence represented by SEQ ID NO:5 and has an α1,6-fucosyltransferase activity.

**7.** The cell according to any one of claims 1 to 6, which is resistant to a lectin which recognizes a sugar chain in which 1-position of fucose is bound to 6-position of *N*-acetylglucosamine in the reducing end through α-bond in a complex *N*-glycoside-linked sugar chain.

**8.** The cell according to claim 7, which is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

**9.** The cell according to any one of claims 1 to 8, which is selected from the group consisting of the following (a) to (j):

(a) a CHO cell derived from a Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-AgI4 cell;
(e) a BHK cell derived from a Syrian hamster kidney tissue;
(f) a hybridoma cell which produces an antibody;
(g) a human leukemic cell line Namalwa cell;
(h) an embryonic stem cell;
(i) a fertilized egg cell;
(j) a plant cell.

**10.** The cell according to any one of claims 1 to 9, which contains a gene encoding an antibody molecule.

**11.** The cell according to claim 10, wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

**12.** The cell according to claim 10 or 11, wherein the antibody molecule belongs to an IgG class.

**13.** The cell according to any one of claims 1 to 12, which produces an antibody composition having a higher antibody-dependent cell-mediated cytotoxic activity than an antibody composition produced by its parent cell.

**14.** The cell according to claim 13, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**15.** The cell according to claim 14, wherein the sugar chain in which fucose is not bound is a sugar chain in which 1-position of the fucose is not bound to 6-position of *N*-acetylglucosamine in the reducing end through $\alpha$-bond in a complex *N*-glycoside-linked sugar chain.

**16.** The cell according to any one of claims 13 to 15, wherein the ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.

**17.** The cell according to any one of claims 13 to 16, wherein the antibody composition having a higher antibody-dependent cell-mediated cytotoxic activity is an antibody composition which has no sugar chain in which fucose is bound to *N*-acetylglucosamine in the reducing end in the sugar chain.

**18.** A process for producing an antibody composition, which comprises using the cell according to any one of claims 10 to 17.

**19.** A process for producing an antibody composition, which comprises culturing the cell according to any one of claims 10 to 18 in a medium to form and accumulate an antibody composition in the culture; and recovering the antibody

composition from the culture.

**20.** The process according to claim 18 or 19, wherein the antibody composition is an antibody composition having a higher antibody-dependent cell-mediated cytotoxic activity than an antibody composition produced by its parent cell.

**21.** The process according to claim 20, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**22.** The process according to claim 21, wherein the sugar chain in which fucose is not bound is a sugar chain in which 1-position of the fucose is not bound to 6-position of *N*-acetylglucosamine in the reducing end through $\alpha$-bond in a complex *N*-glycoside-linked sugar chain.

**23.** The cell according to any one of claims 20 to 22, wherein the antibody composition having a higher antibody-dependent cell-mediated cytotoxic activity is a an antibody composition having a ratio of a sugar chain in which fucose is not bound to *N*-acetylglucosamine in the reducing end through $\alpha$-bond of 20% or more of total complex *N*-glycoside-linked sugar chains bound to the Fc region in the antibody composition.

**24.** The cell according to any one of claims 20 to 23, wherein the antibody composition having a higher antibody-dependent cell-mediated cytotoxic activity is an antibody composition which has no sugar chain in which fucose is bound to *N*-acetylglucosamine in the reducing end in the sugar chain.

**25.** A transgenic non-human animal or plant or the progenies thereof, which is produced by using the cell according to any one of claims 1 to 9.

**26.** The transgenic non-human animal or plant or the progenies thereof according to claim 24, wherein the transgenic non-human animal is an animal selected from the group consisting of cattle, sheep, goat, pig, horse, mouse, rat, fowl, monkey and rabbit.

**27.** The transgenic non-human animal or plant or the progenies thereof according to claim 25 or 26, which is introduced with a gene encoding an antibody molecule.

**28.** The transgenic non-human animal or plant or the progenies thereof according to claim 27, wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

**29.** The transgenic non-human animal or plant or the progenies thereof according to claim 27 or 28, wherein the antibody molecule belongs to an IgG class.

**30.** A process for producing an antibody composition, which comprises rearing the transgenic non-human animal or plant according to any one of claims 27 to 29; isolating a tissue or body fluid comprising an antibody molecule introduced from the reared animal or plant; and recovering an antibody composition comprising a desired antibody molecule from the isolated tissue or body fluid.

**31.** A process for producing an antibody composition, which comprises isolating an antibody-producing cell from the transgenic non-human animal or plant or the progenies thereof according to any one of claims 26 to 29; culturing the isolated antibody-producing cell in a medium to form and accumulate an antibody composition in the culture; and recovering the antibody composition from the culture.

**32.** The process according to claim 30 or 31, wherein the antibody composition is an antibody composition having a higher antibody-dependent cell-mediated cytotoxic activity than an antibody composition produced by a transgenic non-human animal or plant or the progenies thereof in which genome is not modified.

**33.** The process according to claim 32, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by a transgenic non-human animal or plant or the progenies thereof in which genome is not modified.

**34.** The process according to claim 33, wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

**35.** The process according to any one of claims 32 to 34, wherein the antibody composition having a higher antibody-dependent cell-mediated cytotoxic activity is an antibody composition in which a ratio of a sugar chain in which fucose is not bound to *N*-acetylglucosamine in the reducing end in the sugar chain is 20% or more of total complex *N*-glycoside-linked sugar chains bound to the Fc region in the antibody composition.

**36.** The cell according to any one of claims 32 to 35, wherein the antibody composition having a higher antibody-dependent cell-mediated cytotoxic activity is an antibody composition which has no sugar chain in which fucose is bound to *N*-acetylglucosamine in the reducing end in the sugar chain.

**37.** An antibody composition comprising an antibody molecule having an *N*-glycoside-linked sugar chain in the Fc region, which has a sugar chain in which fucose is not bound to *N*-acetylglucosamine in the reducing end in the sugar chain among total complex *N*-glycoside-linked sugar chains bound to the Fc region in the antibody composition.

**38.** The antibody composition according to claim 37, wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

**39.** An antibody composition produced by the process of any one of claims 18 to 24.

**40.** An antibody composition produced by the process of any one of claims 18 to 24.

**41.** A medicament comprising as an active ingredient the antibody composition according to any one of claims 37 to 40.

**42.** The medicament according to claim 41, which is a diagnostic agent, an preventing agent or a treating agent for tumor-accompanied diseases, allergy-accompanied diseases, inflammatory-accompanied diseases, autoimmune diseases, cardiovascular diseases, viral infection-accompanied diseases or bacterial infection-accompanied diseases.

**43.** Use of the antibody composition according to any one of claims 37 to 40 in the manufacture of the medicament according to claim 41 or 42.

FIG. 1

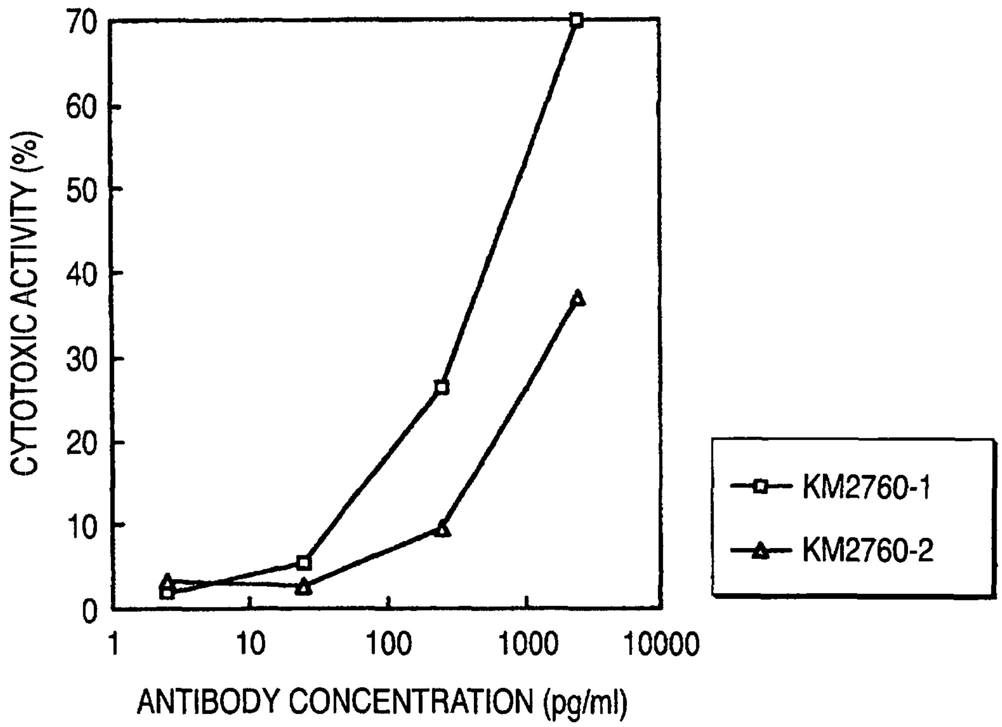
CYTOTOXIC ACTIVITY (%)
70
60
50
40
30
20
10
0
1
10
100
1000
10000
ANTIBODY CONCENTRATION (pg/ml)
KM2760-1
KM2760-2

FIG. 2

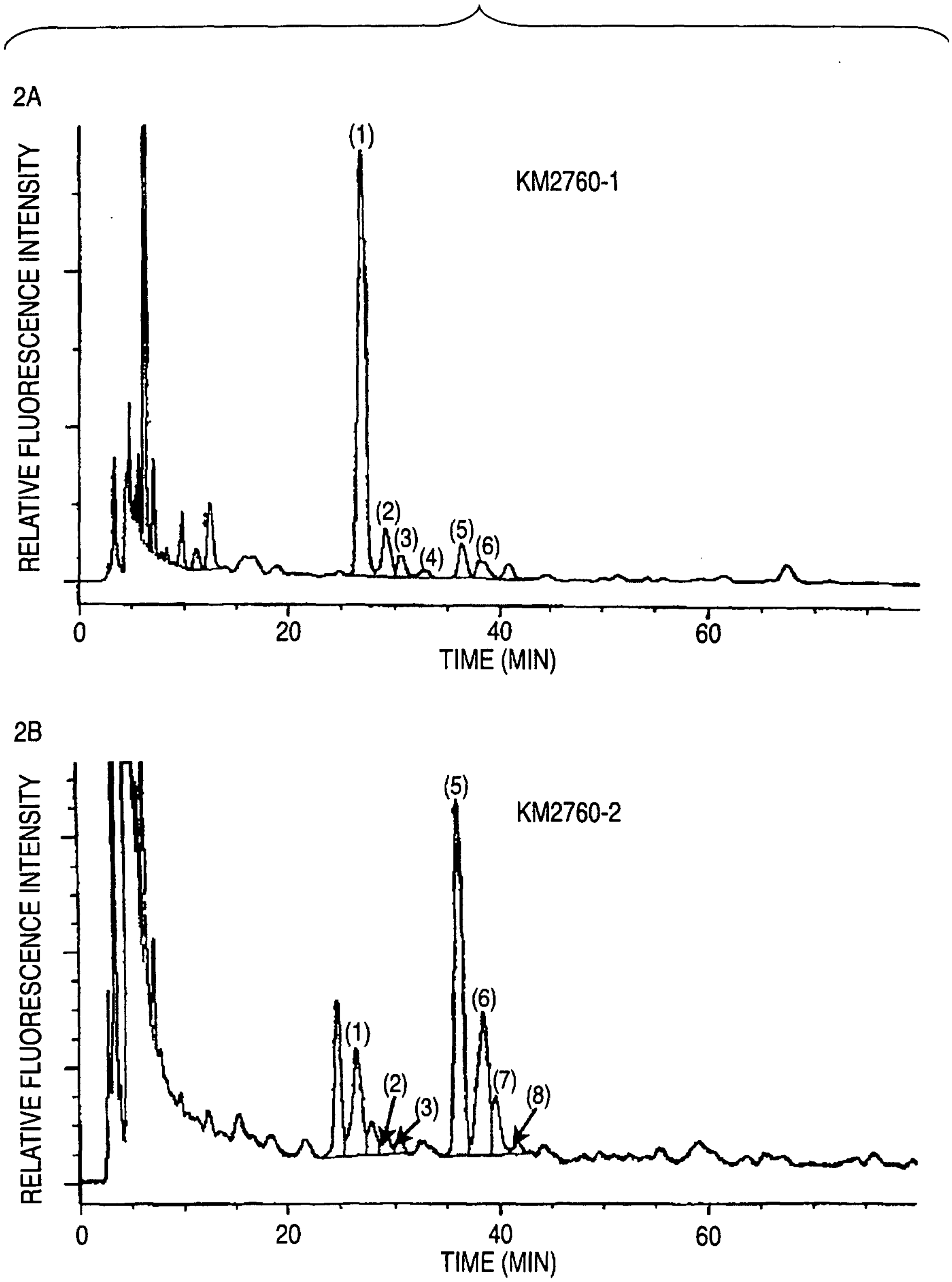
2A
RELATIVE FLUORESCENCE INTENSITY
KM2760-1
(1)
(2)
(3)
(4)
(5)
(6)
0
20
40
60
TIME (MIN)
2B
RELATIVE FLUORESCENCE INTENSITY
KM2760-2
(5)
(6)
(1)
(2)
(3)
(7)
(8)
0
20
40
60
TIME (MIN)

FIG. 3

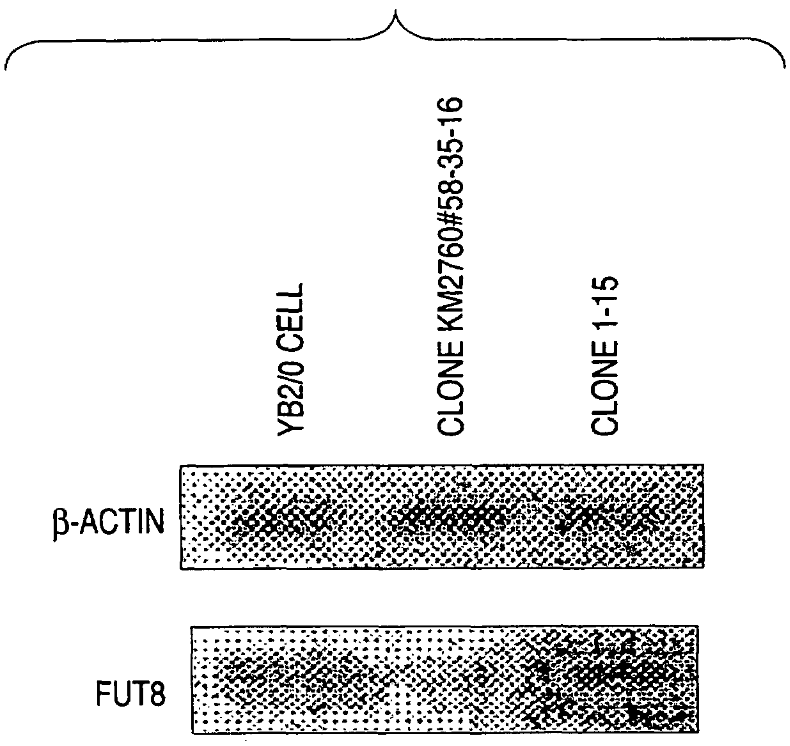

FIG. 4

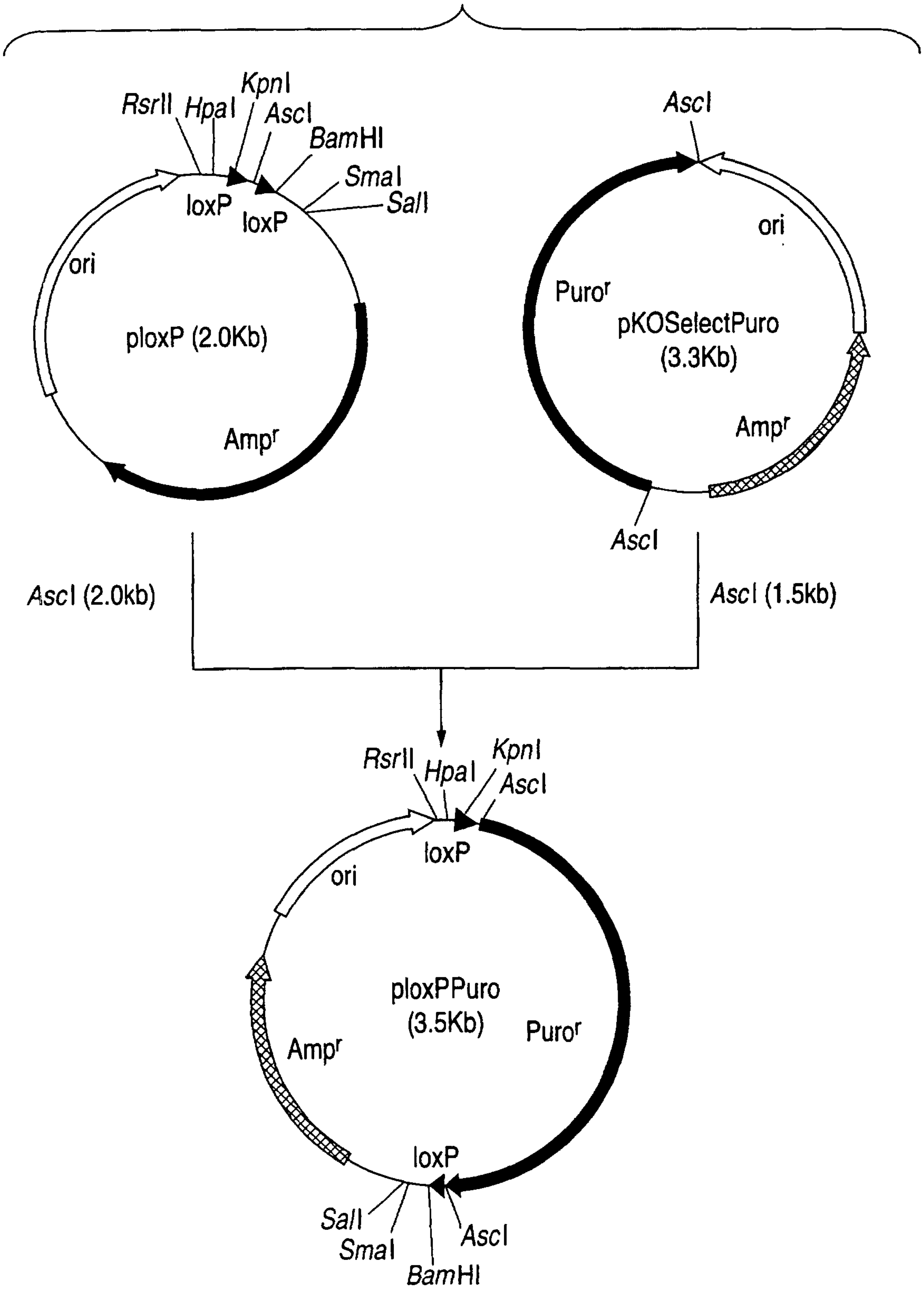
RsrII
HpaI
KpnI
AscI
BamHI
SmaI
SalI
loxP
loxP
ori
ploxP (2.0Kb)
Amp^r
AscI
ori
Puro^r
pKOSelectPuro
(3.3Kb)
Amp^r
AscI
AscI (2.0kb)
AscI (1.5kb)
RsrII
HpaI
KpnI
AscI
loxP
ori
ploxPPuro
(3.5Kb)
Amp^r
Puro^r
loxP
SalI
SmaI
BamHI
AscI

FIG. 5

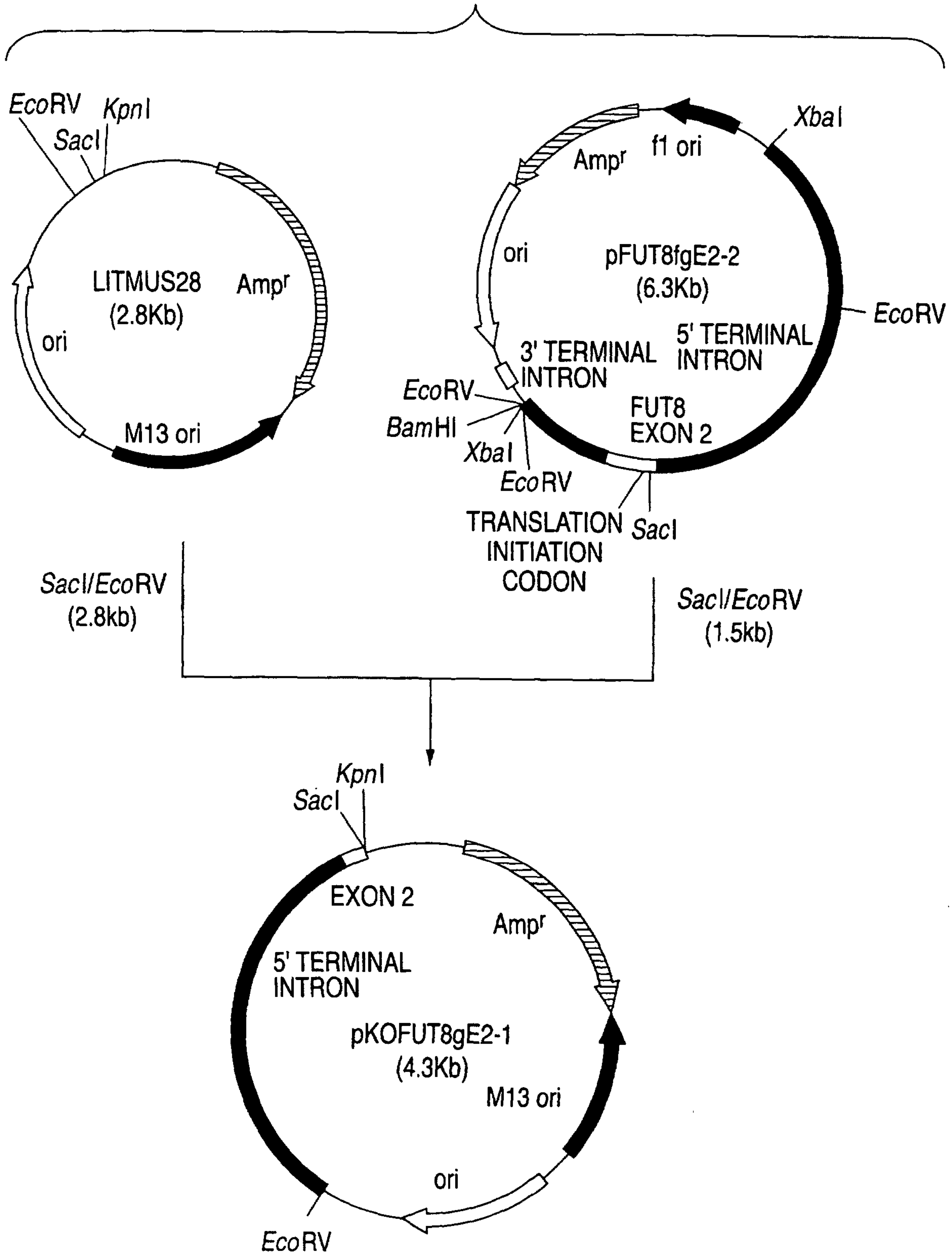
EcoRV
KpnI
SacI
LITMUS28
(2.8Kb)
Ampr
ori
M13 ori
XbaI
f1 ori
Ampr
ori
pFUT8fgE2-2
(6.3Kb)
EcoRV
5' TERMINAL
INTRON
3' TERMINAL
INTRON
EcoRV
BamHI
XbaI
EcoRV
FUT8
EXON 2
TRANSLATION
INITIATION
CODON
SacI
SacI/EcoRV
(2.8kb)
SacI/EcoRV
(1.5kb)
KpnI
SacI
EXON 2
Ampr
5' TERMINAL
INTRON
pKOFUT8gE2-1
(4.3Kb)
M13 ori
ori
EcoRV

FIG. 6

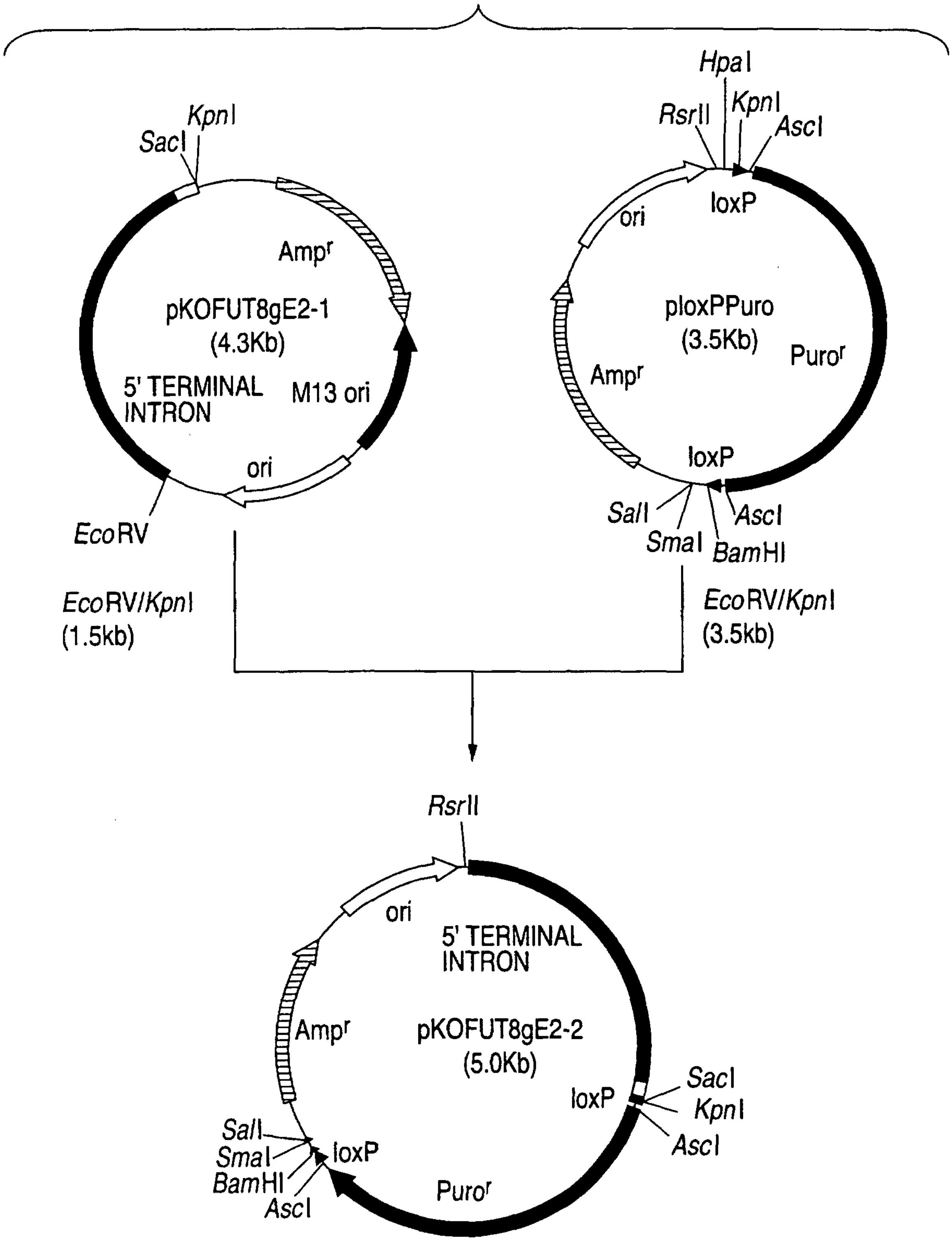
SacI
KpnI
Amp^r
pKOFUT8gE2-1
(4.3Kb)
5' TERMINAL INTRON
M13 ori
ori
EcoRV
HpaI
RsrII
KpnI
AscI
loxP
ori
ploxPPuro
(3.5Kb)
Puro^r
Amp^r
loxP
SalI
SmaI
BamHI
AscI
EcoRV/KpnI
(1.5kb)
EcoRV/KpnI
(3.5kb)
RsrII
ori
5' TERMINAL INTRON
Amp^r
pKOFUT8gE2-2
(5.0Kb)
loxP
SacI
KpnI
AscI
SalI
SmaI
BamHI
AscI
loxP
Puro^r

FIG. 7

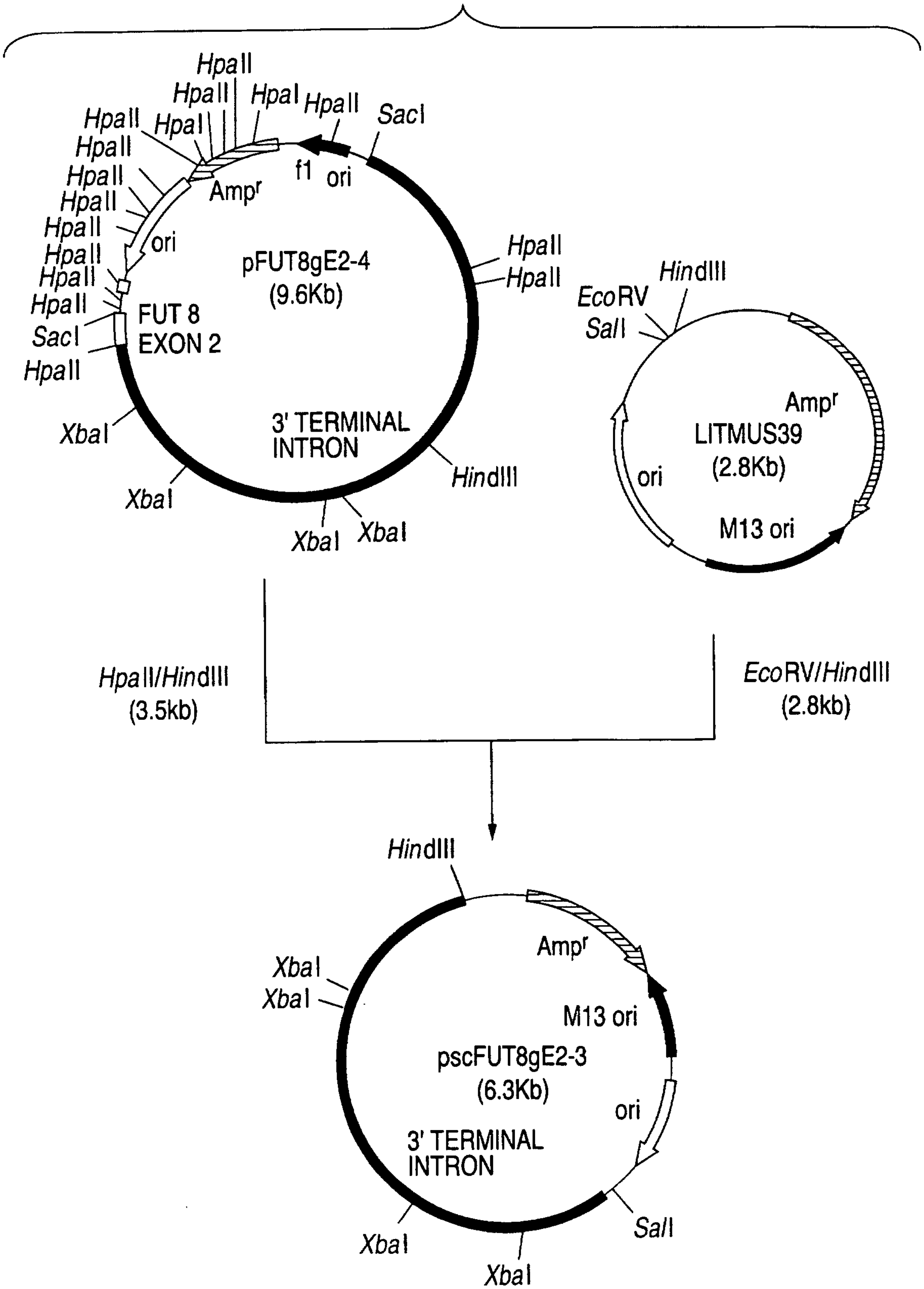
HpaII
HpaII
HpaI
HpaII
SacI
HpaII
HpaI
HpaII
HpaII
HpaII
HpaII
HpaII
HpaII
HpaII
SacI
HpaII
f1 ori
Ampʳ
ori
pFUT8gE2-4
(9.6Kb)
FUT 8
EXON 2
3' TERMINAL
INTRON
HpaII
HpaII
XbaI
XbaI
XbaI
XbaI
HindIII
HindIII
EcoRV
SalI
Ampʳ
LITMUS39
(2.8Kb)
ori
M13 ori
HpaII/HindIII
(3.5kb)
EcoRV/HindIII
(2.8kb)
HindIII
Ampʳ
XbaI
XbaI
M13 ori
pscFUT8gE2-3
(6.3Kb)
ori
3' TERMINAL
INTRON
XbaI
XbaI
SalI

FIG. 8

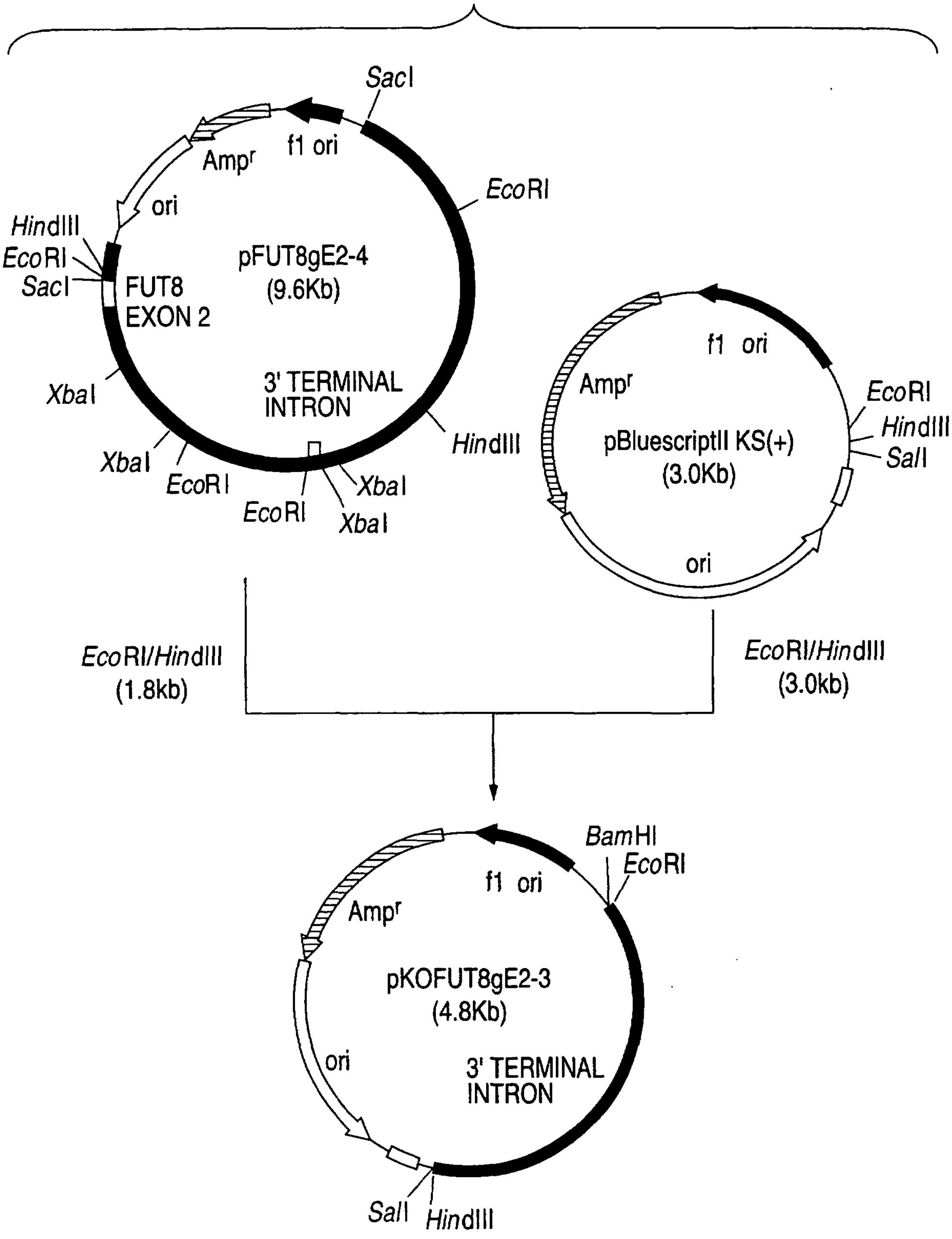
SacI
f1 ori
Amp^r
EcoRI
ori
HindIII
EcoRI
SacI
pFUT8gE2-4
(9.6Kb)
FUT8
EXON 2
XbaI
3' TERMINAL
INTRON
HindIII
XbaI
EcoRI
XbaI
EcoRI
XbaI
f1 ori
Amp^r
EcoRI
HindIII
SalI
pBluescriptII KS(+)
(3.0Kb)
ori
EcoRI/HindIII
(1.8kb)
EcoRI/HindIII
(3.0kb)
BamHI
EcoRI
f1 ori
Amp^r
pKOFUT8gE2-3
(4.8Kb)
ori
3' TERMINAL
INTRON
SalI
HindIII

FIG. 9

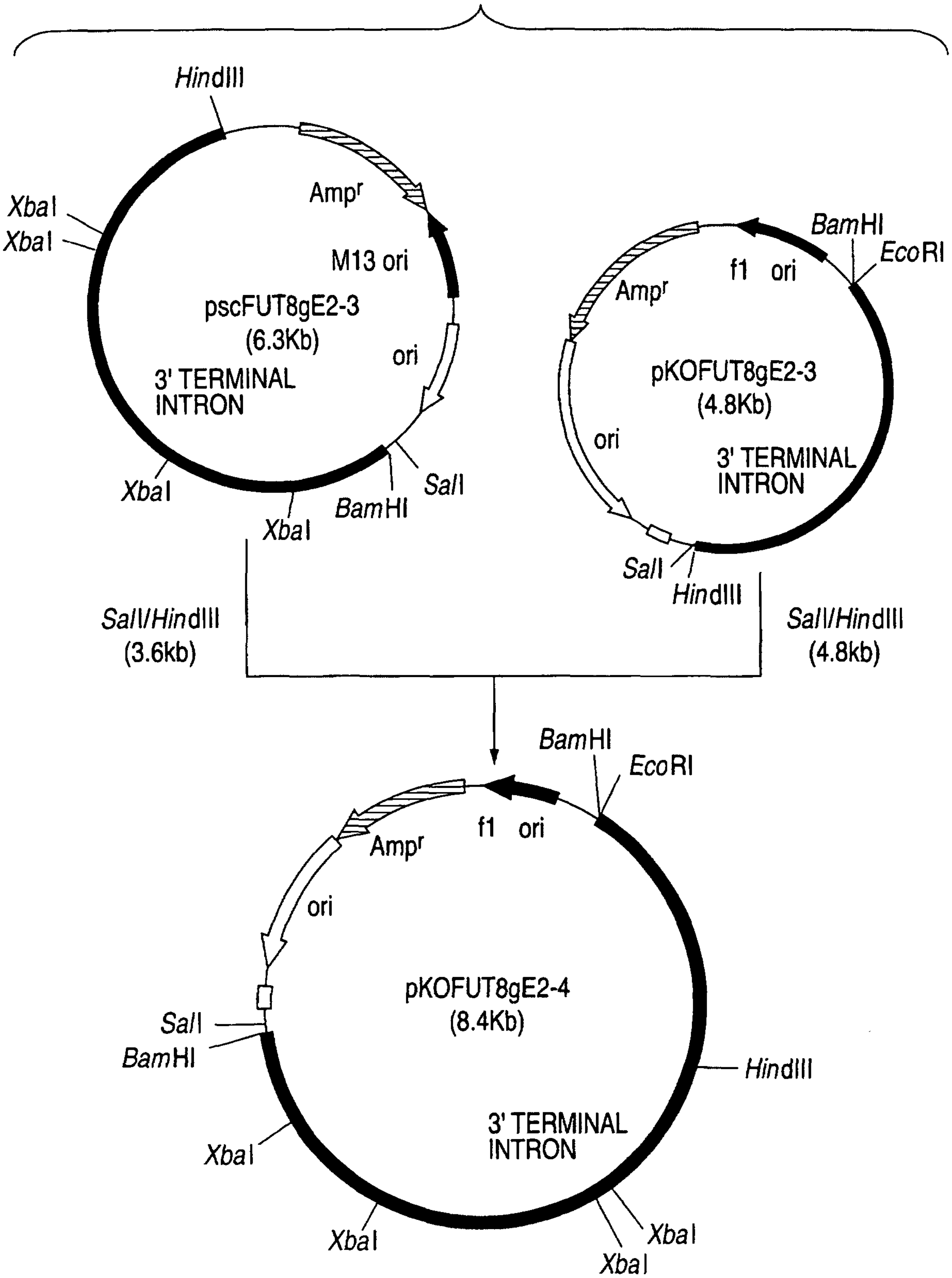
HindIII
XbaI
XbaI
Amp^r
M13 ori
pscFUT8gE2-3
(6.3Kb)
ori
3' TERMINAL INTRON
XbaI
XbaI
BamHI
SalI
BamHI
EcoRI
f1 ori
Amp^r
pKOFUT8gE2-3
(4.8Kb)
ori
3' TERMINAL INTRON
SalI
HindIII
SalI/HindIII
(3.6kb)
SalI/HindIII
(4.8kb)
BamHI
EcoRI
f1 ori
Amp^r
ori
pKOFUT8gE2-4
(8.4Kb)
SalI
BamHI
HindIII
3' TERMINAL INTRON
XbaI
XbaI
XbaI
XbaI

FIG. 10

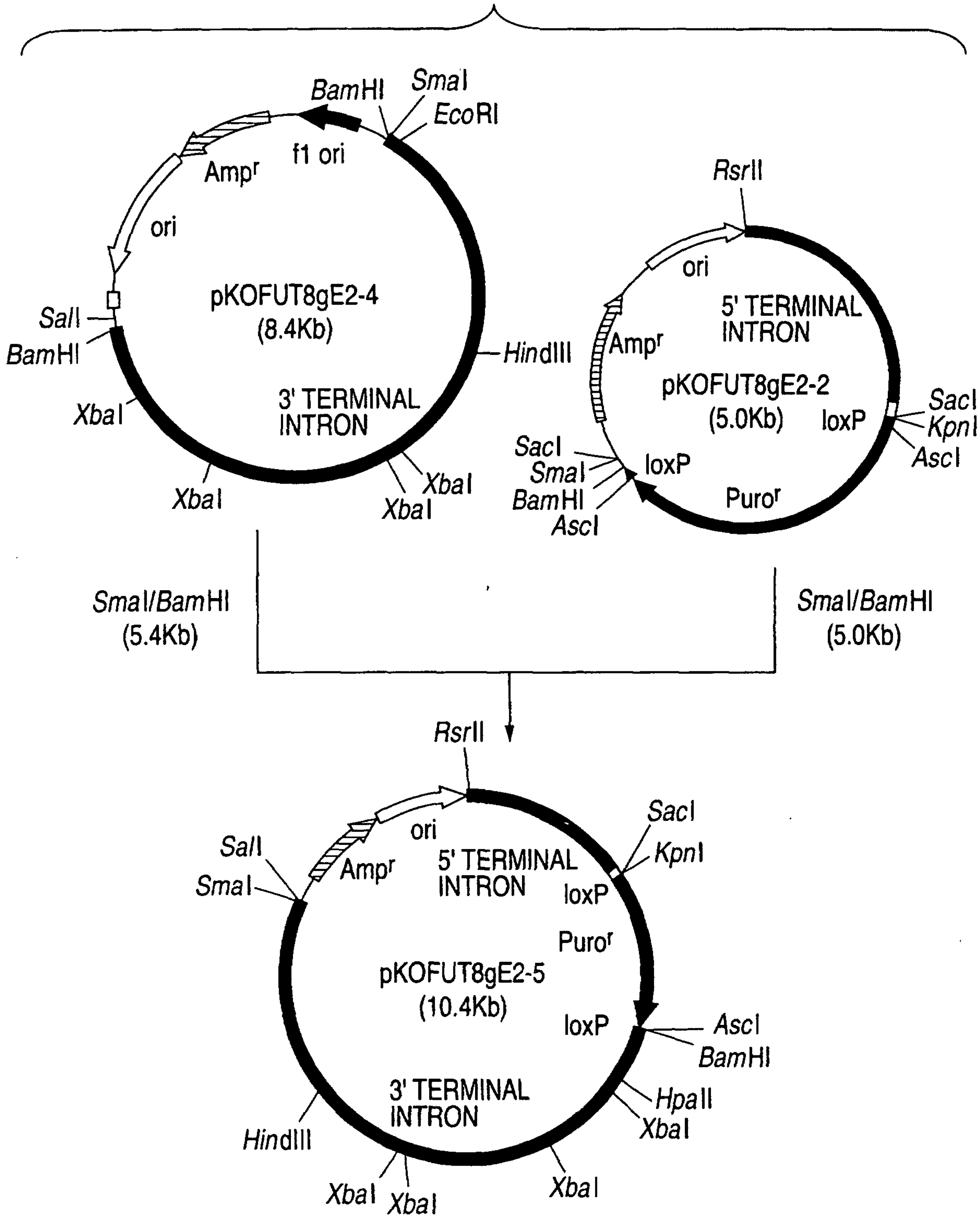
BamHI
SmaI
EcoRI
f1 ori
Amp^r
ori
pKOFUT8gE2-4
(8.4Kb)
SalI
BamHI
HindIII
3' TERMINAL INTRON
XbaI
XbaI
XbaI
XbaI
RsrII
ori
5' TERMINAL INTRON
Amp^r
pKOFUT8gE2-2
(5.0Kb)
loxP
SacI
KpnI
AscI
SacI
SmaI
BamHI
AscI
loxP
Puro^r
SmaI/BamHI
(5.4Kb)
SmaI/BamHI
(5.0Kb)
RsrII
SacI
KpnI
ori
SalI
SmaI
Amp^r
5' TERMINAL INTRON
loxP
Puro^r
pKOFUT8gE2-5
(10.4Kb)
loxP
AscI
BamHI
HpaII
XbaI
3' TERMINAL INTRON
HindIII
XbaI
XbaI
XbaI

FIG. 11

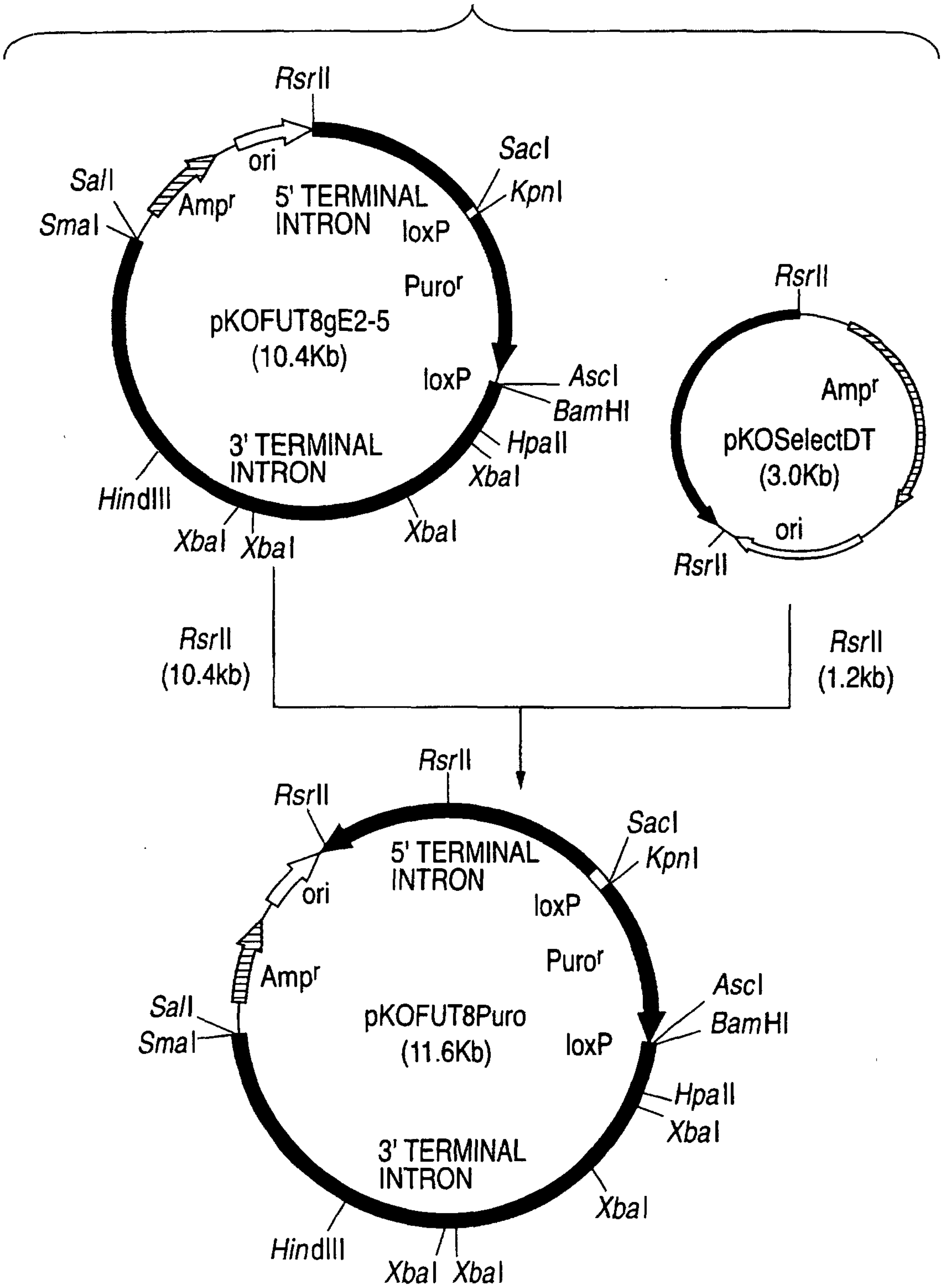
RsrII
SacI
KpnI
ori
SalI
SmaI
Amp^r
5' TERMINAL INTRON
loxP
Puro^r
pKOFUT8gE2-5 (10.4Kb)
loxP
AscI
BamHI
HpaII
XbaI
3' TERMINAL INTRON
HindIII
XbaI
XbaI
XbaI
RsrII
Amp^r
pKOSelectDT (3.0Kb)
ori
RsrII
RsrII (10.4kb)
RsrII (1.2kb)
RsrII
RsrII
SacI
KpnI
5' TERMINAL INTRON
ori
loxP
Puro^r
Amp^r
AscI
BamHI
SalI
SmaI
pKOFUT8Puro (11.6Kb)
loxP
HpaII
XbaI
3' TERMINAL INTRON
XbaI
HindIII
XbaI
XbaI

FIG. 12

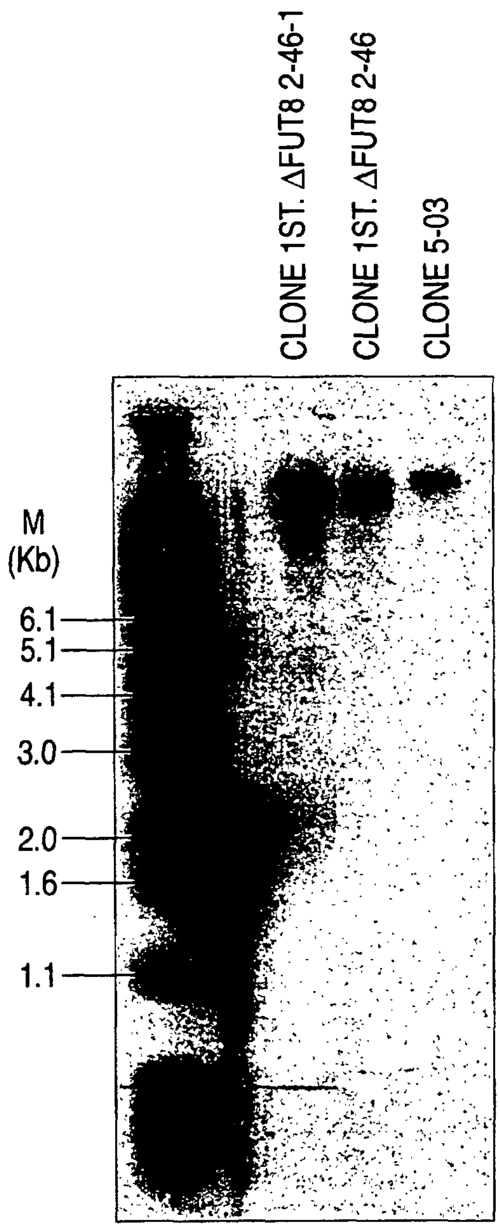
CLONE 1ST. ΔFUT8 2-46-1
CLONE 1ST. ΔFUT8 2-46
CLONE 5-03
M
(Kb)
6.1
5.1
4.1
3.0
2.0
1.6
1.1

FIG. 13

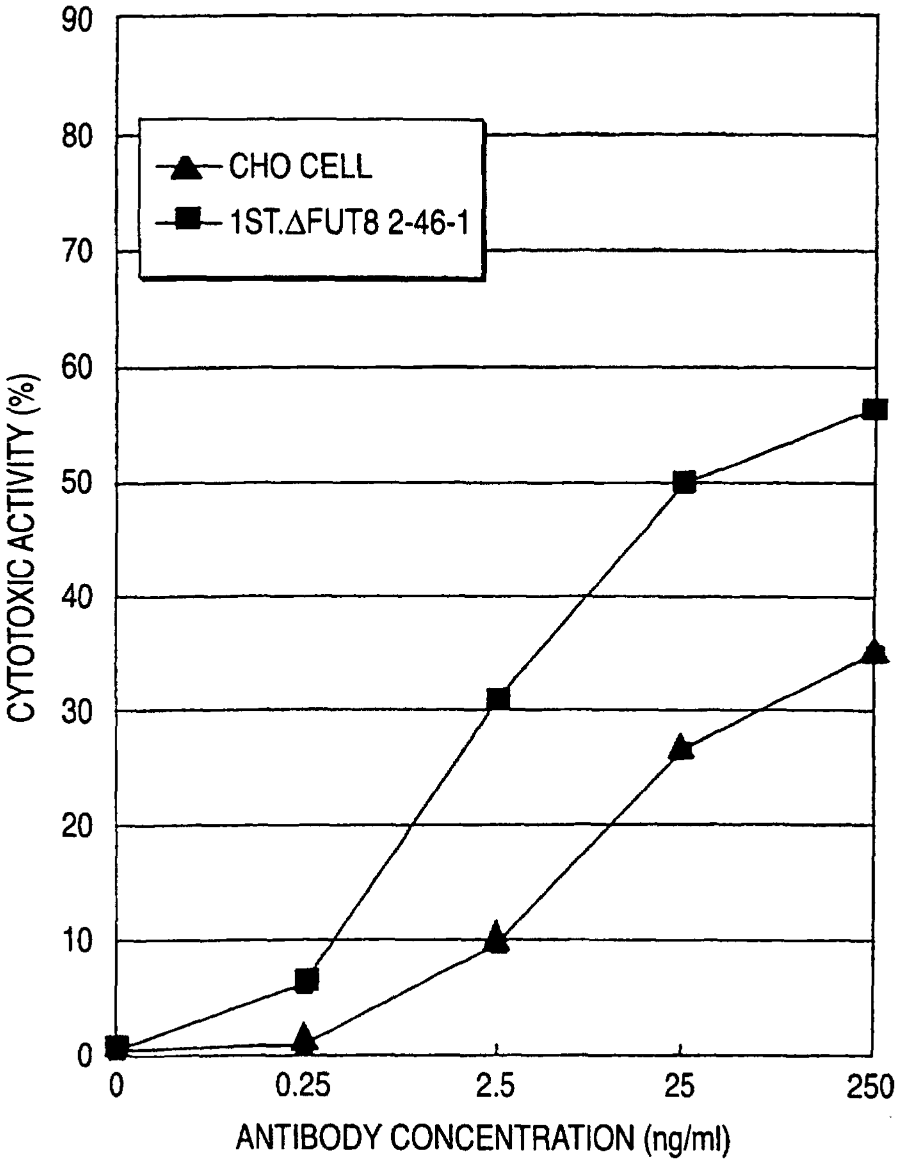
90
80
70
60
50
40
30
20
10
0
CYTOTOXIC ACTIVITY (%)
CHO CELL
1ST.ΔFUT8 2-46-1
0
0.25
2.5
25
250
ANTIBODY CONCENTRATION (ng/ml)

FIG. 14

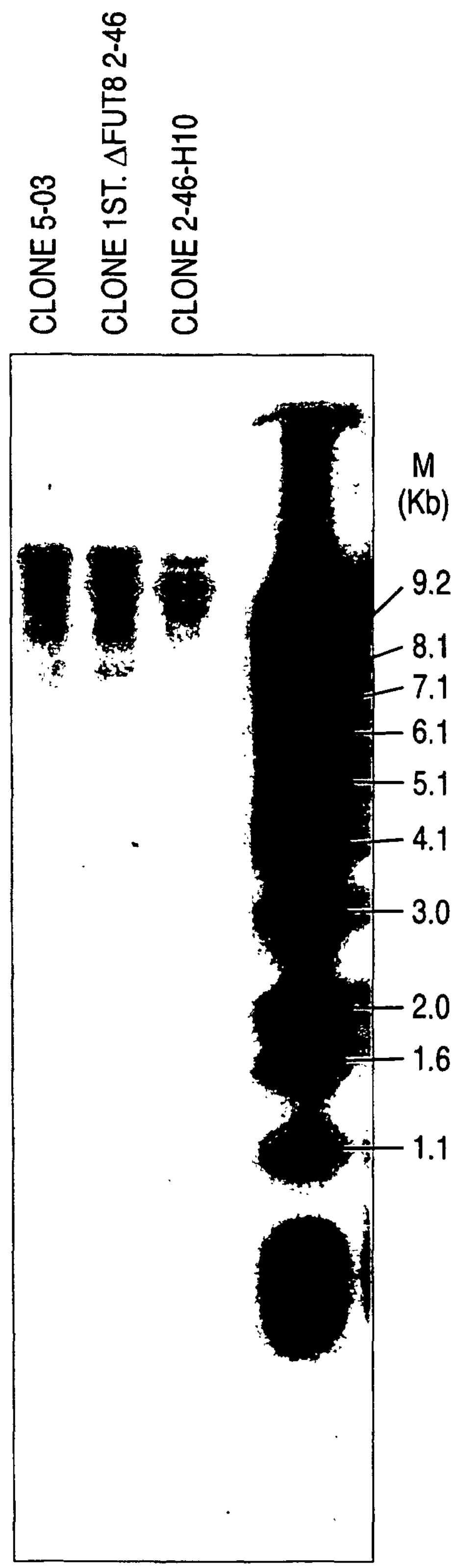
CLONE 5-03
CLONE 1ST. ΔFUT8 2-46
CLONE 2-46-H10
M
(Kb)
9.2
8.1
7.1
6.1
5.1
4.1
3.0
2.0
1.6
1.1

FIG. 15

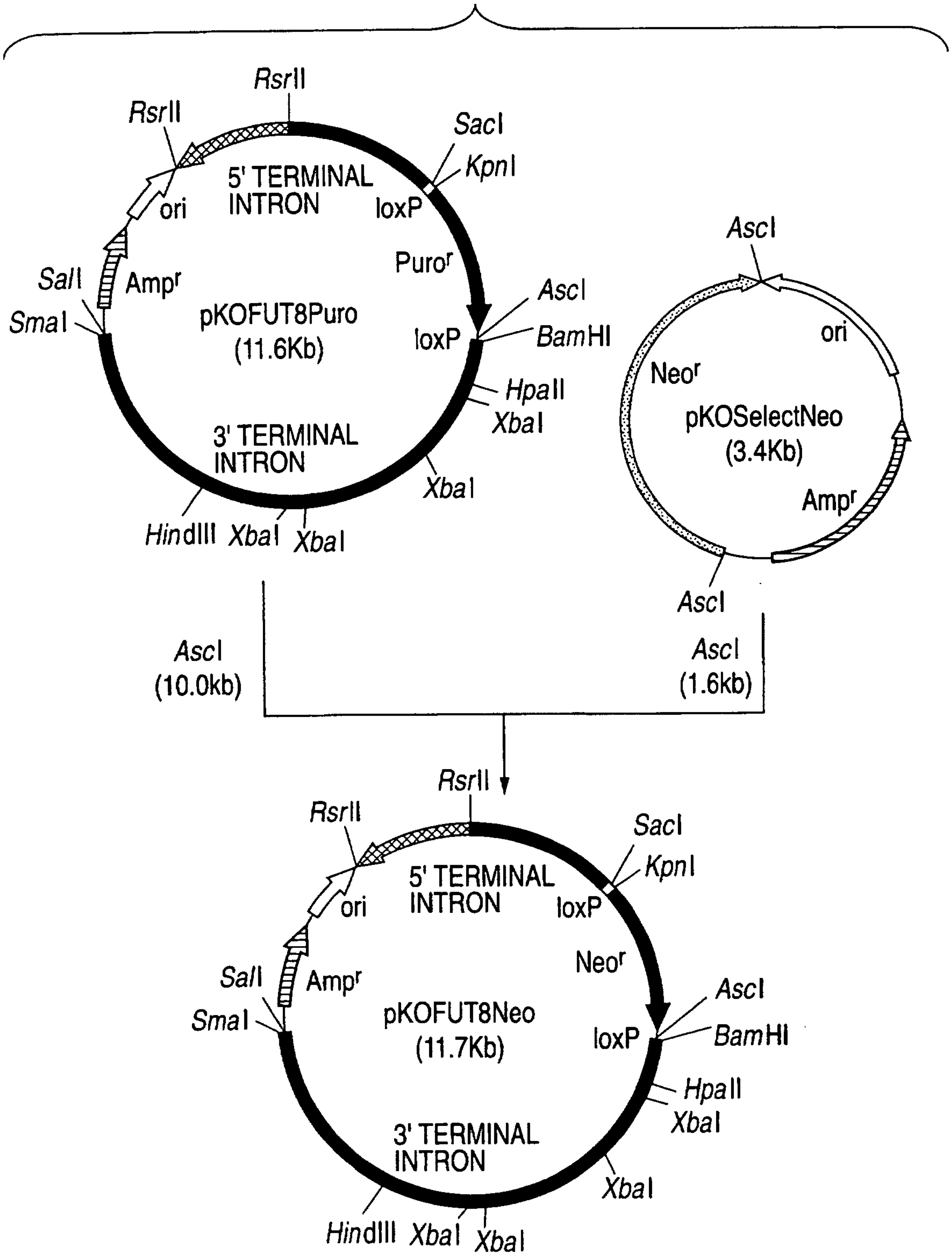
RsrII
RsrII
SacI
KpnI
5' TERMINAL INTRON
ori
loxP
Puro^r
Amp^r
SalI
SmaI
pKOFUT8Puro
(11.6Kb)
AscI
loxP
BamHI
HpaII
XbaI
3' TERMINAL INTRON
XbaI
HindIII
XbaI
XbaI
AscI
ori
Neo^r
pKOSelectNeo
(3.4Kb)
Amp^r
AscI
AscI
(10.0kb)
AscI
(1.6kb)
RsrII
RsrII
SacI
KpnI
5' TERMINAL INTRON
ori
loxP
Neo^r
Amp^r
SalI
SmaI
pKOFUT8Neo
(11.7Kb)
AscI
loxP
BamHI
HpaII
XbaI
3' TERMINAL INTRON
XbaI
HindIII
XbaI
XbaI

FIG. 16

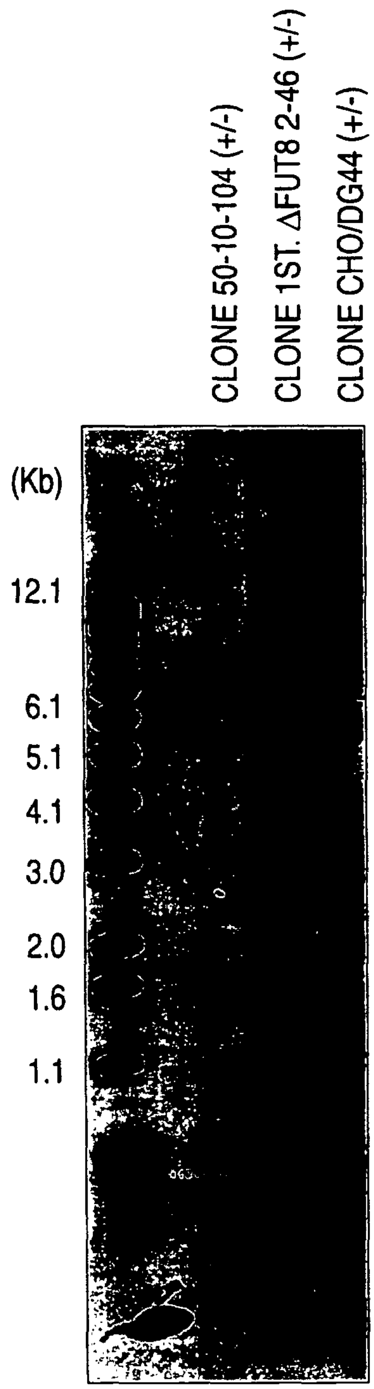
CLONE 50-10-104 (+/-)
CLONE 1ST. ΔFUT8 2-46 (+/-)
CLONE CHO/DG44 (+/-)
(Kb)
12.1
6.1
5.1
4.1
3.0
2.0
1.6
1.1

FIG. 17

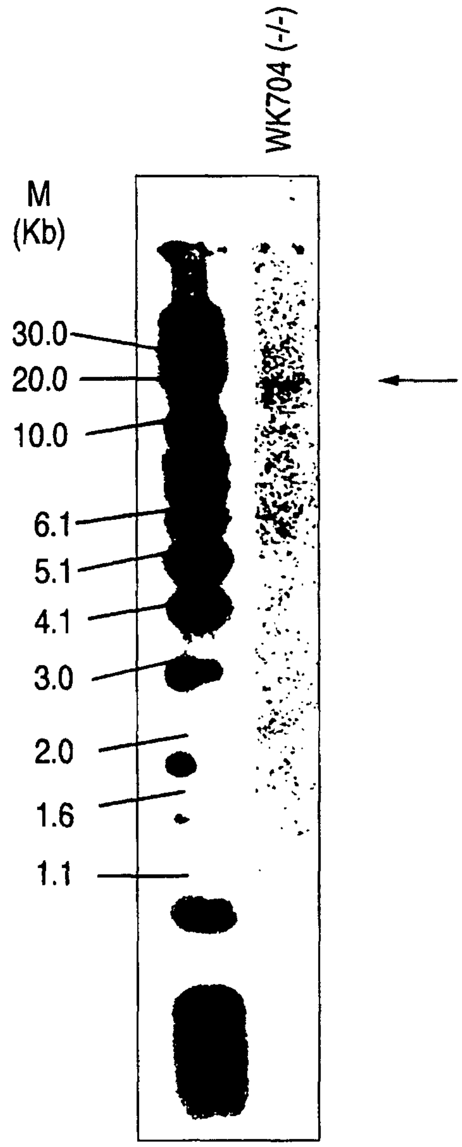

FIG. 18

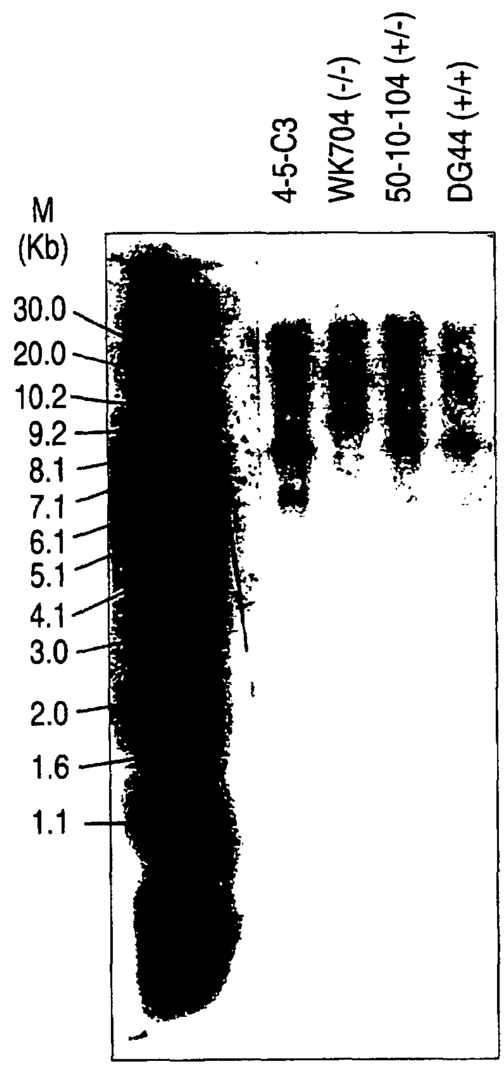
4-5-C3
WK704 (-/-)
50-10-104 (+/-)
DG44 (+/+)
M
(Kb)
30.0
20.0
10.2
9.2
8.1
7.1
6.1
5.1
4.1
3.0
2.0
1.6
1.1

FIG. 19

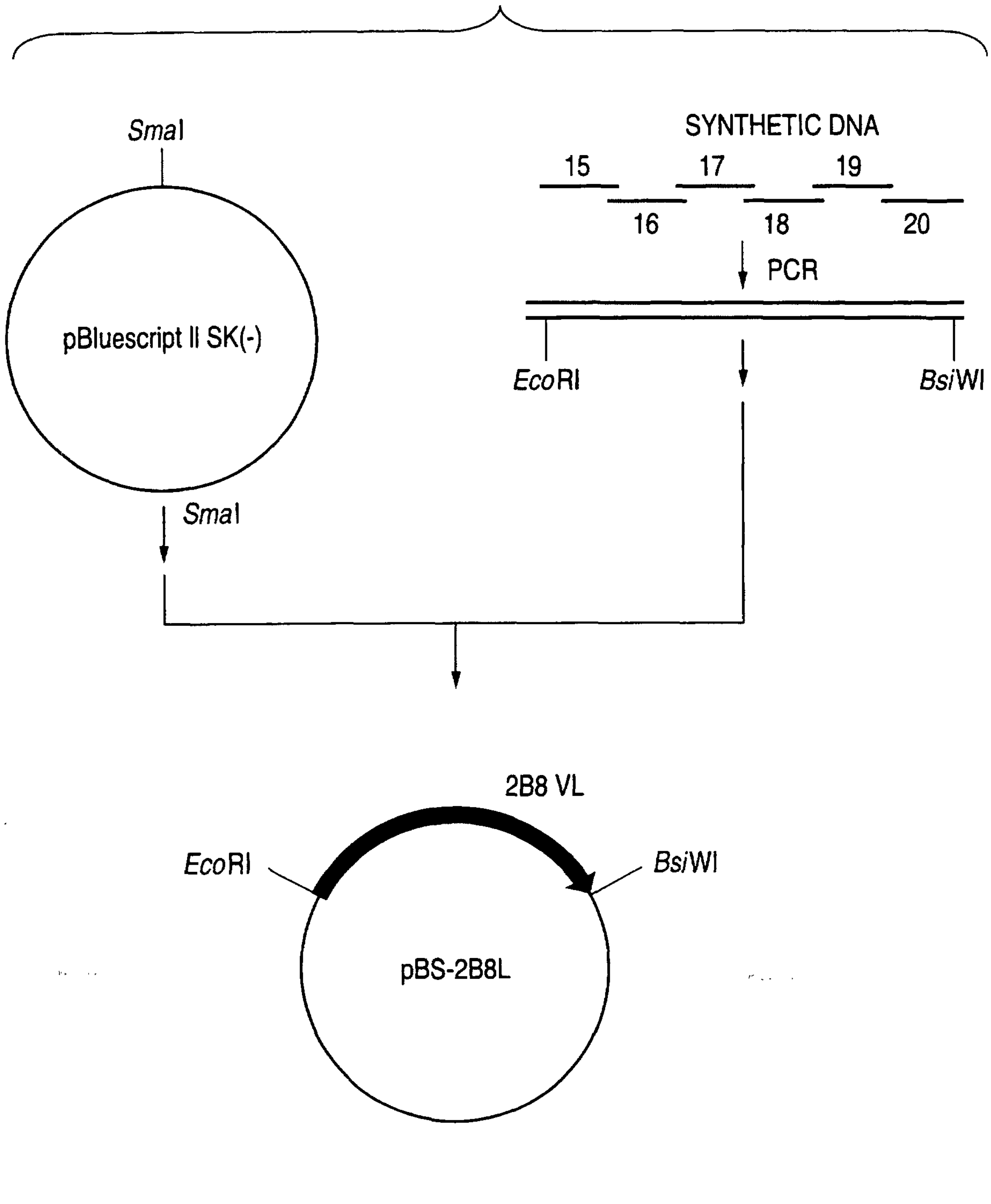
SmaI
pBluescript II SK(-)
SYNTHETIC DNA
15
17
19
16
18
20
PCR
EcoRI
BsiWI
SmaI
2B8 VL
EcoRI
BsiWI
pBS-2B8L

FIG. 20

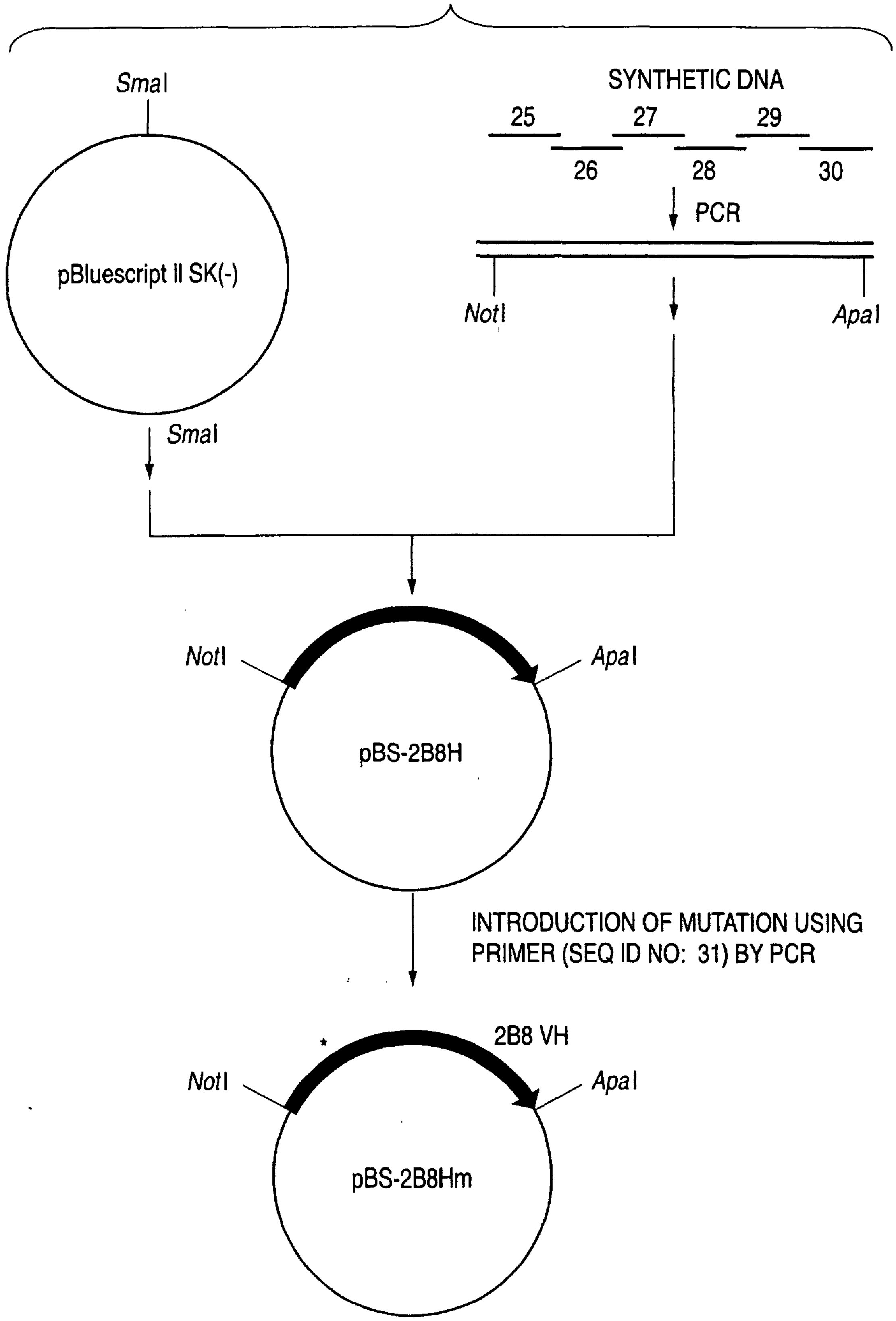
SmaI
pBluescript II SK(-)
SmaI
SYNTHETIC DNA
25
27
29
26
28
30
PCR
NotI
ApaI
NotI
ApaI
pBS-2B8H
INTRODUCTION OF MUTATION USING PRIMER (SEQ ID NO: 31) BY PCR
*
2B8 VH
NotI
ApaI
pBS-2B8Hm

FIG. 21

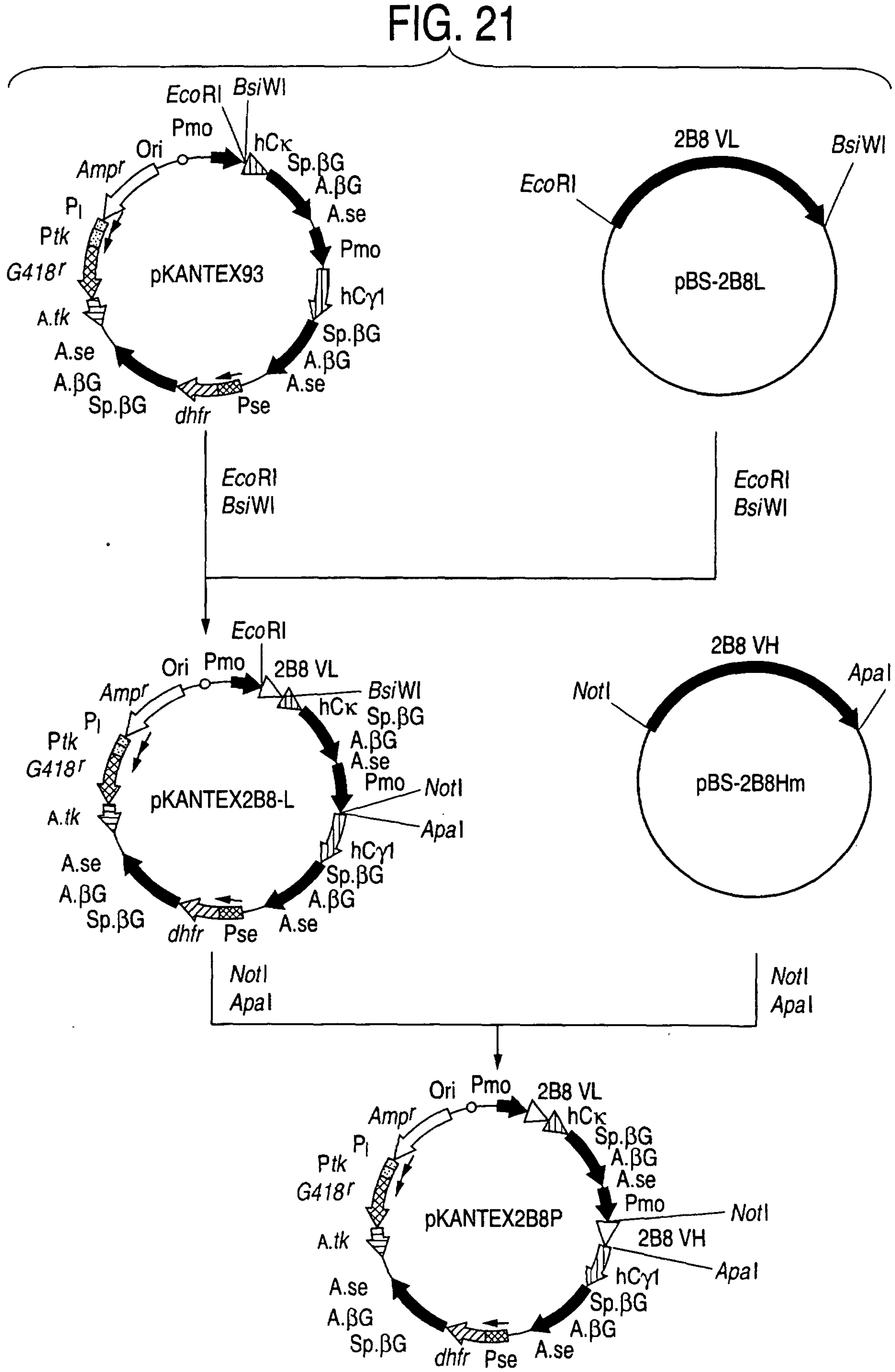
EcoRI
BsiWI
Pmo
Ori
hCκ
Sp.βG
A.βG
A.se
Ampr
Pl
Ptk
G418r
pKANTEX93
Pmo
hCγ1
A.tk
A.se
A.βG
Sp.βG
dhfr
Pse
2B8 VL
BsiWI
EcoRI
pBS-2B8L
EcoRI
BsiWI
EcoRI
BsiWI
EcoRI
Ori
Pmo
2B8 VL
BsiWI
pKANTEX2B8-L
NotI
ApaI
2B8 VH
ApaI
NotI
pBS-2B8Hm
NotI
ApaI
NotI
ApaI
pKANTEX2B8P
2B8 VH

FIG. 22

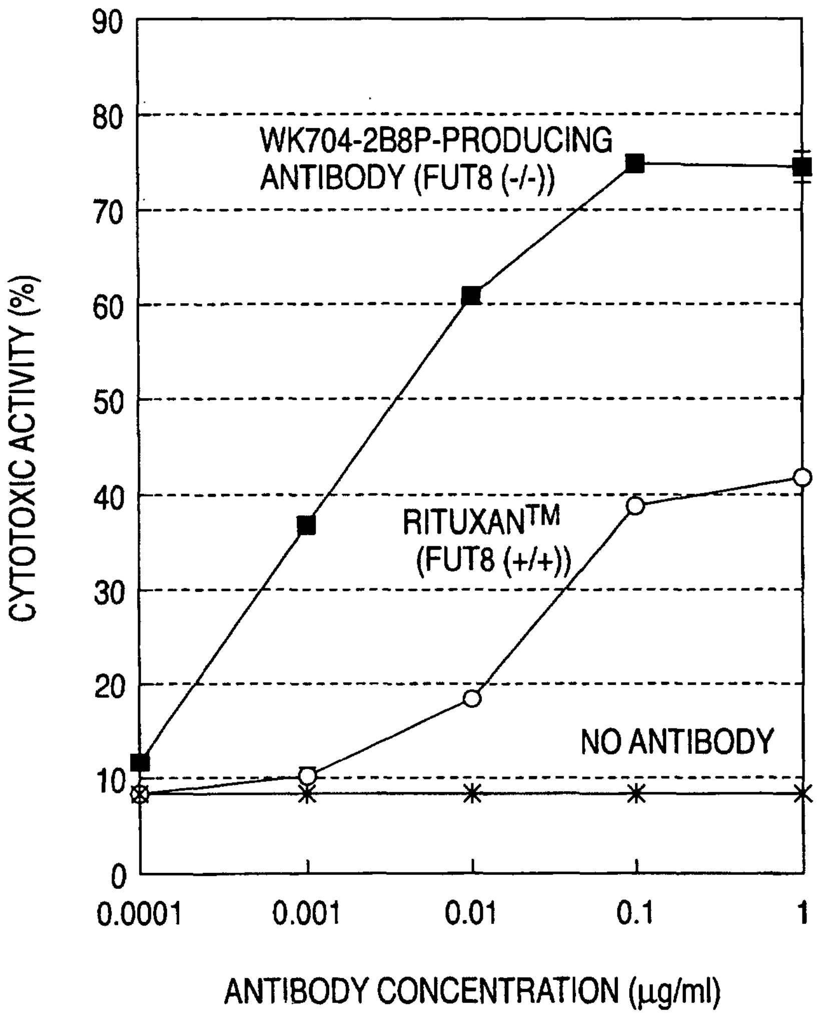
90
80
70
60
50
40
30
20
10
0
CYTOTOXIC ACTIVITY (%)
WK704-2B8P-PRODUCING
ANTIBODY (FUT8 (-/-))
RITUXAN™
(FUT8 (+/+))
NO ANTIBODY
0.0001
0.001
0.01
0.1
1
ANTIBODY CONCENTRATION (μg/ml)

FIG. 23

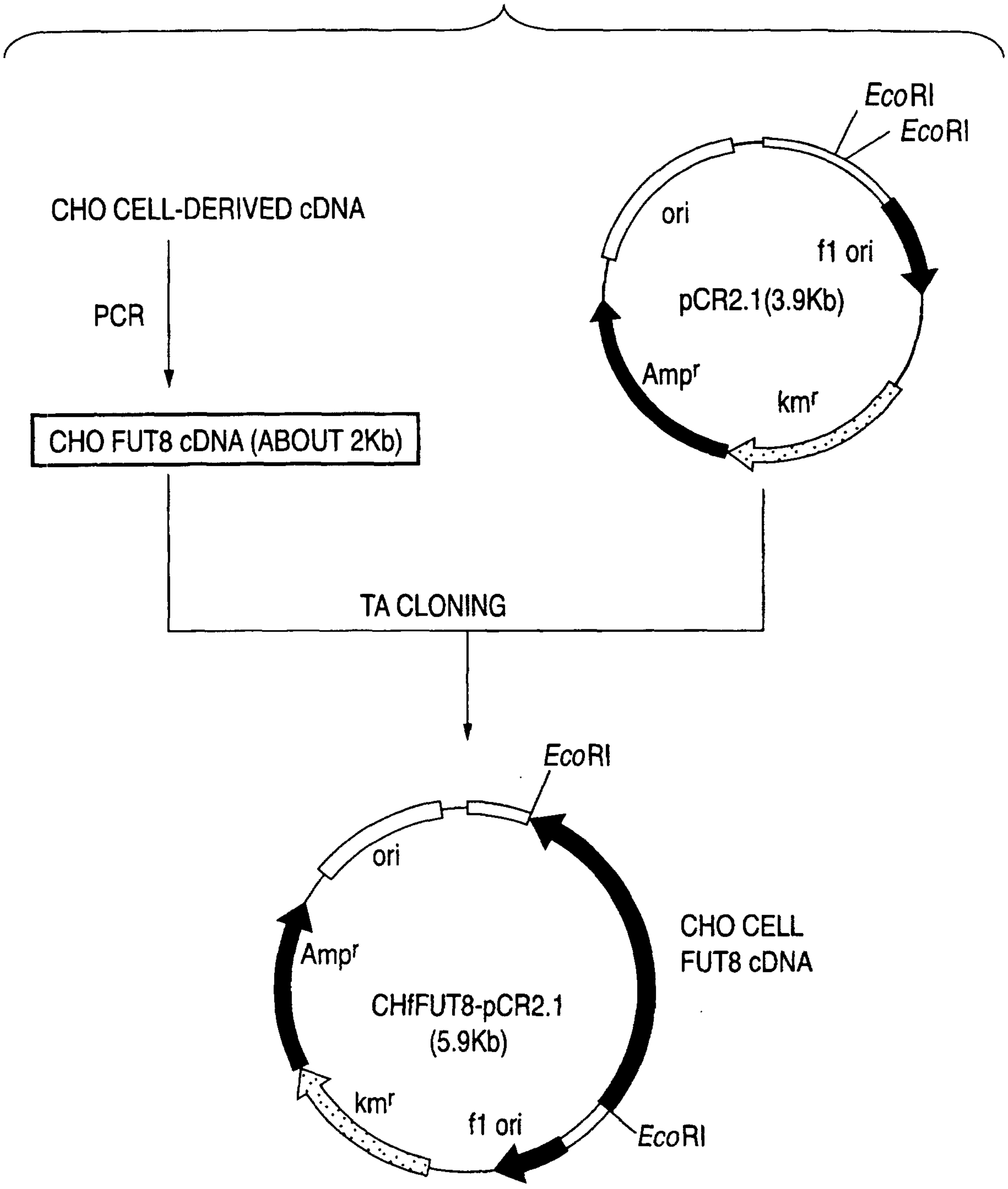
CHO CELL-DERIVED cDNA
PCR
CHO FUT8 cDNA (ABOUT 2Kb)
EcoRI
EcoRI
ori
f1 ori
pCR2.1(3.9Kb)
Amp^r
km^r
TA CLONING
EcoRI
ori
CHO CELL
FUT8 cDNA
Amp^r
CHfFUT8-pCR2.1
(5.9Kb)
km^r
f1 ori
EcoRI

## INTERNATIONAL SEARCH REPORT

International application No.
PCT/JP03/04507

A. CLASSIFICATION OF SUBJECT MATTER
Int.Cl[7] C12N15/09, C12N15/10, C12P21/08, A01H5/00, A01K67/027, C07K16/00, A61K39/395, A61P35/00, A61P37/00, A61P9/00, A61P31/12, A61P31/04

According to International Patent Classification (IPC) or to both national classification and IPC

B. FIELDS SEARCHED

Minimum documentation searched (classification system followed by classification symbols)
Int.Cl[7] C12N15/09, C12N5/10, C12P21/08, A01H5/00, A01K67/027, C07K16/00

Documentation searched other than minimum documentation to the extent that such documents are included in the fields searched

Electronic data base consulted during the international search (name of data base and, where practicable, search terms used)
BIOSIS(DIALOG), WPI(DIALOG), SwissProt/PIR/GeneSeq, GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y | EP 1176195 A1 (KYOWA HAKKO KOGYO CO., LTD.), 30 January, 2002 (30.01.02), & WO 00/61739 A1 & AU 200036728 A & JP 2000-611663 A | 1-4,7-43<br>5-6 |
| Y | HAYASHI, H. et al., Molecular cloning of mouse alpha-1,6-fucosyltransferase and expression of its mRNA in the developing cerebrum, DNA Seq., 2000, Vol.11, No.1-2, pages 91 to 96 | 5-6 |
| A | DOMINO, S.E. et al., Deficiency of reproductive tract alpha(1,2)fucosylated glycans and normal fertility in mice with targeted deletions of the FUT1 or FUT2 alpha(1,2)fucosyltransferase locus, Mol.Cell Biol., 2001, Vol.21, No.24, pages 8336 to 8345 | 1-43 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 May, 2003 (06.05.03) | 20 May, 2003 (20.05.03) |
| Name and mailing address of the ISA/<br>Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP03/04507

C (Continuation). DOCUMENTS CONSIDERED TO BE RELEVANT

| Category* | Citation of document, with indication, where appropriate, of the relevant passages | Relevant to claim No. |
|---|---|---|
| P,X | SHINKAWA, T. et al., The absence of fucose but not the presence of galactose or bisecting N-acetylglucosamine of human IgG1 complex-type oligosaccharides shows the critical role of enhancing antibody-dependent cellular cytotoxicity, J.Biol.Chem., 2003 Jan., Vol.278, No.5, pages 3466 to 3473 | 1-43 |

Form PCT/ISA/210 (continuation of second sheet) (July 1998)